# EUROPEAN PATENT APPLICATION

(11) **EP 3 263 567 A1**
(43) Date of publication of application: **03.01.2018**
(21) Application number: 16177663.8
(22) Date of filing: 01.07.2016
(51) Int. Cl.: C07D 401/14, A61K 31/444, A61K 31/519, C07D 471/04, C07D 487/04, A61P 9/00

(54) **CARBOXAMIDE-SUBSTITUTED PYRAZOLES AND TRI(HETERO)ARYL-PYRAZOLES FOR USE IN METHODS OF TREATING AND / OR PREVENTING CARDIOVASCULAR DISEASES AND / OR COMORBIDITIES THEREOF**

(71) Applicant: AiCuris Anti-infective Cures GmbH, 42117 Wuppertal (DE)
(72) Inventor: The designation of the inventor has not yet been filed
(74) Representative: Kilger, Ute

(57) **Abstract**

The present invention relates to carboxamide-substituted pyrazoles and tri(hetero)aryl pyrazoles, pharmaceutical formulations containing such compounds, a process for manufacture and the use of such compounds in methods of reducing the heart rate and accordingly for its use in methods of treating and/or preventing cardiovascular diseases and/or cardiovascular comorbidities, which are exacerbated by an increased heart rate of ≥ 90 beats/min. (bpm), such as cardiac arrhythmia, hypertensive heart disease, tachycardia, inappropriate sinus tachycardia, angina pectoris and stable angina pectoris, myocardial ischemia, coronary artery disease, heart failure, and stroke, as well as the cardiovascular comorbidities diabetes, metabolic syndrome, obesity, and chronic kidney disease in mammals, including humans.

## Description

The present invention relates to carboxamide-substituted pyrazoles and tri(hetero)aryl pyrazoles, pharmaceutical formulations containing such compounds, processes for manufacture and the use of such compounds in methods of reducing the heart rate and, accordingly, for its use in methods of treating and/or preventing cardiovascular diseases and/or cardiovascular comorbidities, which are exacerbated by an increased average 24-h heart rate of ≥ 90 beats/min. (bpm), such as cardiovascular diseases selected from the group comprising cardiac arrhythmia (hereinafter CA), hypertensive heart disease (hereinafter HHD), tachycardia, inappropriate sinus tachycardia (hereinafter IST), angina pectoris (hereinafter AP) and stable angina pectoris (hereinafter SAP), myocardial ischemia (hereinafter MI), coronary artery disease (hereinafter CAD), heart failure (hereinafter HF), and stroke, as well as cardiovascular comorbidities selected from the group comprising diabetes, metabolic syndrome, obesity, and chronic kidney disease (hereinafter CKD) in humans and/or animals.

Cardiovascular diseases (hereinafter abbreviated CVD(s)) are a class of diseases that involve the heart and/or blood vessels. CVDs generally include coronary artery diseases (CAD(s)) such as AP and myocardial infarction (also known as heart attack). Other CVDs are *inter alia* CA, HHD, IST, AP and SAP, MI, HF, and stroke.

The mechanisms underlying CVDs may vary depending on the disease concerned. For instance, CAD and stroke involve atherosclerosis. This may be caused by high blood pressure, smoking, diabetes, lack of exercise, obesity, high blood cholesterol, poor diet, and excessive alcohol consumption, among others. Of note, high blood pressure results in 13 % of CVD deaths.

CVDs involving the blood vessels are known as vascular diseases and are reflected by *inter alia* CAD, peripheral arterial disease (disease of blood vessels that supply blood to the arms and legs), cerebrovascular disease (disease of blood vessels that supply blood to the brain, including stroke), renal artery stenosis, and aortic aneurysm.

CVDs that involve the heart are reflected by cardiomyopathy (diseases of cardiac muscle), HHD (diseases of the heart secondary to high blood pressure or hypertension), HF, pulmonary heart disease (a failure at the right side of the heart with respiratory system involvement), cardiac dysrhythmias (abnormalities of heart rhythm).

CVDs are still leading cause of morbidity and mortality worldwide.

There are approx. 300 million cases of dyslipidemia in the US, Japan, and Western Europe. Dyslipidemia is associated with more than half of all cases of ischemic heart disease globally. About 17 million deaths occur worldwide each year from CVD. About 26 million people worldwide suffer from HF, accounting for 1 % - 2 % of healthcare resources in industrialized countries. However, full treatment goals are not met with current therapeutics, such as beta blockers, angiotensinconverting-enzyme (ACE) inhibitors or angiotensin receptor blockers (ARBs), and diuretics in all HF patients.

In consequence, there is a remaining need for new therapeutic options to address the aforementioned CVDs in the general population, and therefore new therapeutic options are of exceptional medical importance.

Heart rate is an important contributor in the pathophysiology of CVDs, as e.g. in case of CAD and HF, and is being increasingly recognised as a modifiable risk factor in respective patients.

There are many mechanisms through which an elevated heart rate might directly affect cardiovascular risk, including increased myocardial oxygen demand, energy depletion, accelerated atherosclerosis, and increased risk of plaque rupture. This makes reduction of heart rate an important therapeutic target in patients with e.g. CAD and/or HF.

Heart rate is determined by spontaneous electrical pacemaker activity in the sinoatrial node (hereinafter SAN). Cardiac pacemaker cells generate the spontaneous slow diastolic depolarisation that drives the membrane voltage away from a hyperpolarised level towards the threshold level for initiating a subsequent action potential, generating rhythmic action potentials that propagate through the heart and trigger myocardial contraction. The rate of spontaneous diastolic depolarization is significantly influenced by l*_{f}*, a mixed sodium-potassium current involving ion movement across the so-called f-channels, which are activated by hyperpolarization, and the opening of which is dependent on the intracellular availability of cyclic adenosine monophosphate.

Background information on heart rate reduction via funny channel blockers can be *inter alia* derived from Curr Opin Pharmacol. 2007 Apr;7(2):208-13. Heart rate reduction via selective 'funny' channel blockers. Bucchi A1, Barbuti A, Baruscotti M, DiFrancesco D.; Curr Med Res Opin. 2005 Jul;21 (7):1115-22. Cardiac pacemaker l(f) current and its inhibition by heart rate-reducing agents. DiFrancesco D.; Circ Res. 2010 Feb 19;106(3):434-46. The role of the funny current in pacemaker activity. DiFrancesco D.; Drugs. 2013 Sep;73(14):1569-86. Selective and specific inhibition of lf with ivabradine for the treatment of coronary artery disease or heart failure. Deedwania P.; Molecules. 2012 Apr 5;17(4):4225-35. *HCN channels and heart rate.* Scicchitano P1, Carbonara S, Ricci G, Mandurino C, Locorotondo M, Bulzis G, Gesualdo M, Zito A, Carbonara R, Dentamaro I, Riccioni G, Ciccone MM.

The l*_{f}* current is thus an ionic current that determines the slope of the diastolic depolarisation, which in turn controls the heart rate.

The cardiac impulse originates in the SAN, located at the right atrial endocardium, between the upper and lower cava vein, and formed by highly specialized cells that are able to generate action potentials, which start the sinus rhythm.

These action potentials are fundamental in order to allow cardiac muscle cells to contract and make the heart to play its work. Within cardiac cycle length, this means that phase 4 is characterized by a slow increase in membrane potential to the threshold for triggering a new action potential. There are many currents (both inward and outward) involved in the functioning of the SAN, able to determine the profile of the final current. In particular, the three main ones are:
- an inward current, i.e. *l_{f}*, induced by hyperpolarization (also known as the "funny current");
- an inward current of calcium, i.e. *l_{ca}*;
- an outward current, i.e. *l_{K}.*

The funny current, i.e. *l_{f}*, is highly expressed in spontaneously active cardiac regions, such as the SAN, which is the natural pacemaker region, the atrioventricular node and the Purkinje fibres of conduction tissue. The funny current is a mixed sodium-potassium current that activates upon hyperpolarization at voltages in the diastolic range (normally from - 60/- 70 mV to - 40 mV).

When at the end of a sinoatrial action potential the membrane repolarizes below the *l_{f}* threshold (about - 40/ - 50 mV), the funny current is activated and supplies inward current, which is responsible for starting the diastolic depolarization phase. By this mechanism, the funny current controls the rate of spontaneous activity of sinoatrial myocytes, and hence the cardiac rate.

The molecular determinants of the pacemaker current (i.e. the *l_{f}* current) belong to the hyperpolarization-activated cyclic nucleotide-gated channels family (HCN, or HCN channel) of which four isoforms (HCN1 - 4) are known. Based on their sequence, HCN channels are classified as members of the superfamily of voltage-gated K⁺ (Kv) and CNG channels.

HCN channels are a family of six transmembrane domain, single pore-loop, hyperpolarization activated, non-selective cation channels. Among the four members HCN1 - 4, HCN4 is considered the main isoform that is able to control the heart rate.

Hence, also genetic alterations of HCN4 channels may be coupled to rhythm disturbances, which have been already reported in humans. For example, an inherited mutation of a highly conserved residue in the CNBD of the HCN4 protein (S672R) is associated with inherited sinus bradycardia.

*In vitro* studies indicate that the S672R mutation causes a hyperpolarizing shift of the HCN4 channel open probability curve of about 5 mV in heterozygosis, an effect similar to the hyperpolarizing shift caused by parasympathetic stimulation and able to explain a reduction of inward current during diastole and the resulting slower spontaneous rate.

Due to HCN channels' main function in the generation of the sinus rhythm and, therefore, heart rate control, it is to be considered that dysfunction in funny channels would cause arrhythmic behavior.

Molecular approaches have tried to clarify the relationships between mutations of the HCN channel genes and alterations in the cardiac function, above all according to a simple arrhythmic level.

A missense mutation (S672R) on the exon 7 in hHCN4 gene induces the production of an isoform of the pacemaker channel changed at cAMP binding site level, which was responsible for a familial form of an asymptomatic bradycardia.

*l_{f}*, generates spontaneous activity and modulates cardiac rate. *l_{f}*, activation is the key process in the modulation of rate by autonomic transmitters. The mammalian pacemaker region (SAN) is densely innervated by the autonomic nervous system: sympathetic ß-adrenergic stimulation accelerates, and parasympathetic muscarinic stimulation slows cardiac rate.

Hence, the discovery of *l_{f}*, and the detailed investigation of its properties were relevant not only in relation to the understanding of the basic principles underlying pacemaker generation and modulation, but also in that it provided the possibility to implement an approach to pharmacological control of heart rate based on the concept of *l_{f}* and its function.

Due to the direct correlation between *l_{f}* and pacemaker activity, *l_{f}* inhibition may lead to slowing down cardiac rate.

Following this, pharmacological reduction of heart rate is a crucial target in the treatment of several pathological conditions characterized by a deficiency of oxygen supply to the working myocardium, such as AP, a common manifestation of CAD, MI and HF.

Several recently developed drugs able to specifically slow down heart rate without substantial side effects (the so-called "heart rate-inhibiting" substances) have indeed been shown to act by selective blockade of funny channels (HCN4). These agents may have a significant impact on specific cardiac therapies, particularly when slowing down heart rate is beneficial, such as for cardiovascular diseases selected from the group comprising CA, HHD, tachycardia, IST, AP and SAP, MI, CAD, HF, and stroke, as well as to cardiovascular comorbidities selected from the group comprising diabetes, metabolic syndrome, obesity, and CKD in humans and/or animals.

Ideally, pharmacological interventions aimed at reducing heart rate selectively should be free of consequences on other cardiovascular parameters. Unfortunately, however, conventional drug therapies based on beta blockers and Ca²⁺ antagonists are often associated with mild to severe decreases in ventricular contractility and modification of vascular tone.

Although, in the past several years, various funny channel inhibitors/blockers such as alinidine (ST567), falipamil (AQ-A39), ZD7288 and zatebradine (UL-FS 49) have been developed and shown to reduce heart rate, but, because of lack of selectivity and/or presence of undesired side effects, further development aimed at their clinical use has not been pursued.

More recently, ivabradine, a new molecule selected for higher funny channel selectivity and reduced side effects, has been developed and is now being marketed for pharmacological treatment of chronic stable angina. Ivabradine is currently already used as a treatment option for SAP and HF in patients.

Ivabradine is a pure heart rate lowering agent acting by inhibiting the funny current (l*_{f}*) in the SAN.

Ivabradine is thus selective for the *l_{f}* current and exerts significant inhibition thereof and heart rate reduction at concentrations that do not affect other cardiac ionic currents. Ivabradine lowers heart rate without any negative inotropic or lusitropic effect, thus preserving ventricular contractility. Ivabradine does not affect blood pressure, myocardial contractility, intra-cardiac conduction, or ventricular repolarisation.

Ivabradine blocks specifically HCN4 channels in the low micromolar range (IC₅₀ mean approx. = 2.15 µM), whereby the steady-state dose-responses for HCN-1 to 4 showed no subtype-specificity (Stieber et al. (2006) Mol Pharmacol 69, 1328-1337).

Efficacies of ivabradine, specifically in IST treatment, have been *inter alia* described in Cardiol J. 2010;17(2):166-71. Efficacy of ivabradine in four patients with inappropriate sinus tachycardia: a three month-long experience based on electrocardiographic, Holter monitoring, exercise tolerance and quality of life assessments. Kaplinsky E1, Comes FP, Urondo LS, Ayma FP; Heart Rhythm. 2010 Sep;7(9):1318-23. Efficacy of ivabradine administration in patients affected by inappropriate sinus tachycardia. Calò L1, Rebecchi M, Sette A, Martino A, de Ruvo E, Sciarra L, De Luca L, Zuccaro LM, Giunta G, Ciccaglioni A, Lioy E, Fedele F.; Naunyn Schmiedebergs Arch Pharmacol. 2010 Dec;382(5-6):483-6. Ivabradine in patients with inappropriate sinus tachycardia. Zellerhoff S1, Hinterseer M, Felix Krull B, Schulze-Bahr E, Fabritz L, Breithardt G, Kirchhof P, Kääb S.; Europace. 2013 Jan;15(1):116-21. Metoprolol succinate vs. ivabradine in the treatment of inappropriate sinus tachycardia in patients unresponsive to previous pharmacological therapy. Ptaszynski P, Kaczmarek K, Ruta J, Klingenheben T, Wranicz JK.; Drugs. 2013 Sep;73(14):1569-86. *Selective and specific inhibition of If with ivabradine for the treatment of coronary artery disease or heart failure.* Deedwania P.

Background information on IST can be *inter alia* derived from J Am Coll Cardiol. 2013 Feb 26:61(8):793-801. *Inappropriate sinus tachycardia.* Olshansky B, Sullivan RM.

Although, ivabradine is generally well tolerated, patients may still complain of side effects, such as visual symptoms and bradycardia.

For instance, ivabradine is contraindicated in severe bradycardia, II or III degree atrio-ventricular block, ejection fraction < 35%, and severe hypotension. In addition, it should not be used in patients who are already taking CYP 3A4 inhibitors, such as ketoconazole, macrolides (such as erythromycin), and nefazodone (used in the treatment of human immunodeficiency virus).

The most common side effects yet observed in clinical trial participants were too much slowing of the heart rate (bradycardia) and temporary vision disturbances (such as flashes of light).

Thus, it is desirous to have more potent agents that specifically lower the heart rate, but do not involve modifications of any other cardiovascular parameters, and thus, would lack adverse side effects. Because HCN channel isoforms are unevenly distributed among excitable tissues, the identification of new isoform-specific compounds that serves the above-mentioned purpose remain a clinical need.

For now, to achieve heart rate reduction, beta blocking medication or calcium antagonists are still routinely administered prior to computer tomography coronary angiography (CTCA) examinations. Ivabradine appears to be an alternative, but still provides for the above-mentioned side effects. Hence, the use of beta blockers, calcium antagonists, or ivabradine is limited, however, due to various contraindications. In recent studies, for instance, a substantial portion of patients requiring heart rate reduction has contraindications to beta blockers.

Thus, there exists a need for improved heart-rate-lowering medication.

The inventors, surprisingly and unexpectedly, found that the herein disclosed compounds of the classes carboxamide-substituted pyrazoles and tri(hetero)arylpyrazoles according to the general formula (I) are very potent and effective HCN4 blockers/inhibitors, and are thus useful in methods of treating and/or preventing cardiovascular diseases and / or comorbidities thereof, in particular cardiovascular diseases selected from the group comprising CA, HHD, tachycardia, IST, AP and SAP, MI, CAD, HF, and stroke, as well as to cardiovascular comorbidities selected from the group comprising diabetes, metabolic syndrome, obesity, and CKD in humans and/or animals.

With the above context, the present invention is also directed to compounds of the general formula (I) for use in a method of treatment and/or prophylaxis of metabolic syndrome, diabetic complications, atherosclerosis, vascular dimensia, coronary heart disease, or congestive heart failure in a human and/or animal by administering to a human and/or animal in need of such treatment a therapeutically effective amount of a compound of the general formula (I) or a pharmaceutically acceptable salt of said compound.

Suprisingly, the inventors found the herein disclosed carboxamide-substituted pyrazoles and tri(hetero)aryl-pyrazoles showed a more potent inhibition of HCN4 by a factor of 7.7 to 149 when directly compared with ivabradine (IC₅₀ = 4.46 µM).

Thus, in one aspect of the invention, the herein disclosed compounds of the general formula (I) are intended for the use in the aforementioned cardiovascular diseases and / or comorbidities thereof. Thereby, the inventors found that the compounds of the general formula (I) unexpectedly do not interact with other ion channels of Nav, Kav, and Cav substantially (as exemplarily shown for 4-[5-[3-Chloro-5-(trifluoromethoxy) phenyl]-1-(pyridin-3-yl)-1H-pyrazol-3-yl]-5,6- dihydro-7H-pyrrolo[3,4-d] pyrimidin-7-one, i.e. compound A in accordance with the invention; see further below).

Due to the above described isolated effect in blocking/inhibiting the HCN4 receptor, the herein disclosed carboxamide-substituted pyrazoles and tri(hetero)arylpyrazoles may be effective in lowering the heart rate in humans and animals, and may thus be exploited for its use in methods of treating and/or preventing the aforementioned cardiovascular diseases and / or comorbidities thereof.

Structurally, the substance classes of carboxamide-substituted pyrazoles and tri(hetero)aryl-pyrazoles differ greatly from known HCN inhibitors (i.e. ivabradine, alinidine, zatebradine, cilobradine), and thus the compounds of general formula (I) in accordance with the invention may react in the methods of treatment and/or prevention as recited herein with different binding pockets and mechanisms than the compounds known in the art.

Tri(hetero)aryl-pyrazoles and analogues thereof as well as derivatives thereof are known in the art, and are described *inter alia* in DE 10 2012 016 908; WO 2014/027112; WO 2015/121413; WO 2013/079586; DE 10 2011 055 815; DE 10 2009 036 604; WO 2011/012630; DE 10 2008 015 032; DE 10 2008 015 033; WO 2009/115213; WO 2010/075962; DE 10 2008 062863; DE 10 2008 062 878; WO 2010/075957; DE 10 2009 038 123; and WO 2011/020822.

The chemical substance class of pyrazoles has already been described for various medical indications: US 5,624,941 and EP 576357 describe pyrazoles as cannabinoid receptor antagonists; EP 418845, EP 554829 and WO 04/050632 among other things for the treatment of inflammatory and thrombotic diseases; WO 03/037274 as sodium ion channel inhibitors for the treatment of pain; WO 06/015860 as adenosine receptor ligands for the treatment of inflammatory and obstructive respiratory diseases; EP 1762568 as inhibitors of platelet aggregation; WO 07/002559 as modulators of the activity of nuclear receptors; WO 07/020388 and WO 05/080343 as cannabinoid receptor modulators, among other things for the treatment of obesity and psychiatric and neurological disorders; WO 07/009701 and EP 1743637 for the treatment of cardiovascular risk factors; and DE 10 2004 054 666 to fight weeds or to regulate plant growth.

WO 2011/058149 describes tricyclic pyrazole derivatives as PI3k inhibitors for the treatment of autoimmune diseases. WO 2008/074982 describes pyrazole derivatives as CB1 receptor modulators in the treatment of overweight. Pyrazole derivatives as agents against blood platelet aggregation for the treatment of ischemic diseases are described in WO 2004/069824 and WO 2006/004027. Pyrazole derivatives as COX-1 inhibitors are described in WO 2004/050632 and US 2004/0116475. WO 2008/017932 describes various aryl sulfonamides, among them a pyrazole-containing example, as carbonic anhydrase inhibitors.

DE 10 2008 015 033 and DE 10 2008 015 032 describe phenyl-substituted pyrazoles and their use for the treatment and prevention of infections with retroviruses.

With the above context, subject matter of the present invention are the compounds of the general formula (I) 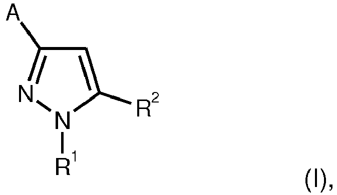 wherein
- A: stands for a group of formula (i) or (ii) 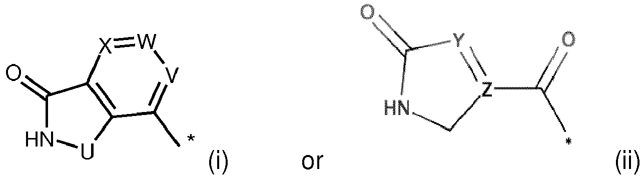
wherein in formula (i)
- U: stands for carbon,
whereby carbon can be substituted with 1 to 2 alkyl substituents, which are selected independently of one another, or with an oxo substituent,
- V: stands for nitrogen,
- W: stands for carbon,
whereby carbon can be substituted with a substituent selected from the group consisting of halogen, amino, hydroxy, methoxy, and methyl,
- X: stands for nitrogen or carbon,
whereby carbon can be substituted with a substituent selected from the group consisting of halogen, amino, hydroxy, methoxy, and methyl, and
- *: is the point of attachment to the carbon atom;
wherein in formula (ii)
- Y: stands for carbon,
whereby carbon can be substituted with halogen, amino, hydroxy, methoxy, and methyl,
- Z: stands for carbon or nitrogen, and
- *: is the point of attachment to the carbon atom;

- R¹: stands for phenyl or pyridyl,
whereby phenyl is substituted with 1 to 2 substituents, the substituents being selected independently of one another from the group consisting of halogen, hydroxy, amino, cyano, and nitro;
- R²: stands for phenyl,
whereby phenyl is substituted with 1 to 2 substituents, the substituents being selected independently of one another from the group consisting of halogen, hydroxy, amino, nitro, (C1-C4)-alkyl, (C1-C4)-alkylamino and (C1-C4)-alkoxy,
wherein
alkyl, alkylamino and alkoxy in turn may be substituted one to three times identically or differently with radicals selected from the group consisting of halogen, hydroxy, amino,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

In another aspect, further subject matter of the present invention are the compounds of the above represented formula (I),

in which A stands for a group of said formula (i) or (ii) as represented above,
wherein in formula (i)
- U: stands for carbon,
- W: stands for carbon,
whereby carbon can be substituted with a methyl,
- X: stands for nitrogen or carbon,
whereby carbon can be substituted with a methyl;
- X: preferably stands for nitrogen or carbon, and
- *: is the point of attachment to the carbon atom,
and wherein
- R¹: stands for phenyl or pyridyl,
whereby phenyl is substituted with 1 to 2 substituents, the substituents being selected independently of one another from the group consisting of halogen, cyano, and nitro,
whereby said 1 to 2 substituents are more preferably selected independently of one another from the group consisting of halogen and cyano, and said halogen is more preferably selected independently of one another from Cl and F,
whereby
- R¹: preferably stands for pyridyl,
wherein
- R¹: more preferred stands for 3-pyridyl or 4-pyridyl,
wherein
- R¹: most preferred stands for 3-pyridyl,
and wherein
- R²: stands for phenyl,
whereby phenyl is substituted with 1 to 2 substituents, the substituents being selected independently of one another from the group consisting of halogen, and (C1-C4)-alkoxy,
wherein
alkoxy in turn may be substituted one to three times identically or differently with radicals selected from the group consisting of halogen,
whereby said 1 to 2 substituents are more preferably selected independently of one another from the group consisting of halogen and (C1-C4)-alkoxy, and said halogen is more preferably selected independently of one another from Cl and F, and the alkoxy on their part can be substituted one to three times with fluorine atoms, more preferred with two fluorine atoms, most preferred with three fluorine atoms,
- R²: preferably stands for phenyl,
whereby phenyl is substituted with two substituents, said two substituents being more preferably selected independently of one another from the group consisting of halogen and (C1-C3)-alkoxy, and the halogen more preferably being selected independently of one another from Cl and F, and the alkoxy on their part can be substituted with three fluorine atoms,
- R²: most preferred stands for 3-Cl-5-CF₃O-phenyl;
wherein in formula (ii)
- Y: stands for carbon,
whereby carbon can be substituted with amino,
- Z: stands for carbon or nitrogen, and
- *: is the point of attachment to the carbon atom,
whereby
- R¹: stands for phenyl or pyridyl,
whereby phenyl is substituted with two substituents, the substituents being selected independently of one another from the group consisting of halogen and cyano,
- R¹: stands preferably for phenyl or 3-pyridyl,
whereby phenyl is substituted with two substituents, the substituents being selected independently of one another from the group consisting of halogen and cyano, whereby said halogen is more preferably selected independently of one another from Cl and F, most preferred said halogen is fluorine,
and wherein
- R²: stands for phenyl,
whereby phenyl is substituted with 1 to 2 substituents, the substituents being selected independently of one another from the group consisting of halogen, and (C1-C4)-alkoxy,
wherein
alkoxy in turn may be substituted one to three times identically or differently with radicals selected from the group consisting of halogen,
whereby said 1 to 2 substituents are more preferably selected independently of one another from the group consisting of halogen and (C1-C4)-alkoxy, and said halogen is more preferably selected independently of one another from Cl and F, and the alkoxy on their part can be substituted one to three times with fluorine atoms, more preferred with two fluorine atoms, most preferred with three fluorine atoms,
- R²: preferably stands for phenyl,
whereby phenyl is substituted with two substituents, said two substituents being more preferably selected independently of one another from the group consisting of halogen and (C1-C3)-alkoxy, and the halogen more preferably being selected independently of one another from Cl and F, and the alkoxy on their part can be substituted with three fluorine atoms,
- R²: most preferred stands for 3-Cl-5-CF₃O-phenyl,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

In another aspect, further subject matter of the present invention are the compounds of the above represented formula (I),
wherein
A has the aforementioned meaning, namely stands for a group of the formula (i) or (ii) 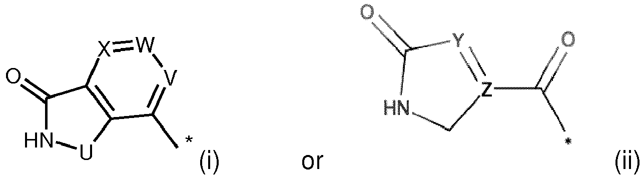 wherein in formula (i)
- U: stands for CH2,
- V: stands for N,
- W: stands for CH or CMe, wherein CMe stands for C-CH3,
- X: stands for N or CH, and
- *: is the point of attachment to the carbon atom,
and wherein
- R¹: stands for phenyl or 3-pyridyl or 4-pyridyl,
- R¹: preferably stands for 3-pyridyl, and
whereby phenyl is substituted with two substituents, whereby the substituents are selected independently of one another from the group consisting of halogen and cyano,
- R²: stands for phenyl,
whereby phenyl is substituted with two substituents, the substituents being selected independently of one another from the group consisting of halogen and (C1-C3)-alkoxy,
wherein
halogen is preferably chlorine, and
alkoxy on their part can be substituted with three fluorine atoms;
wherein in formula (ii)
- Y: stands for CH2 or C-NH2,
- Z: stands for CH or N, and
- *: is the point of attachment to the carbon atom,
and wherein
- R¹: stands for phenyl or 3-pyridyl or 4-pyridyl,
preferably for 3-pyridyl, and
whereby phenyl is substituted with two substituents, where the substituents are selected independently of one another from the group consisting of halogen and cyano,
- R²: stands for phenyl,
whereby phenyl is substituted with two substituents, the substituents being selected independently of one another from the group consisting of halogen and (C1-C3)-alkoxy,
wherein
halogen is preferably chlorine, and
alkoxy on their part can be substituted with three fluorine atoms,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

In another aspect, further subject matter of the present invention are the compounds of the represented formula (I) above,
wherein
A has the aforementioned meaning, namely stands for a group of the formula (i) or (ii) 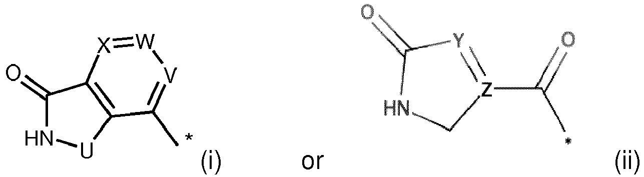 wherein in formula (i)
- U: stands for CH2,
- V: stands for N,
- W: stands for CH or CMe, wherein CMe stands for C-CH3,
- X: stands for N or CH, and
- *: is the point of attachment to the carbon atom,
and wherein
- R¹: stands for 3-pyridyl,
- R²: stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part can be substituted with three fluorine atoms;
wherein in formula (ii)
- Y: stands for CH2 or C-NH2,
- Z: stands for CH or N, and
- *: is the point of attachment to the carbon atom,
and wherein
- R¹: stands for 3-pyridyl,
- R²: stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part can be substituted with three fluorine atoms,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

In another aspect, further subject matter of the present invention are the compounds of the represented formula (I),
wherein
A has the aforementioned meaning, namely stands for a group of the formula (i) or (ii) 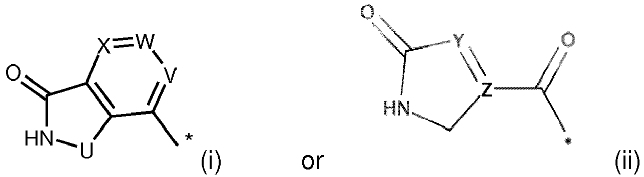 wherein in formula (i)
- U: stands for CH2,
- V: stands for N,
- W: stands for CH,
- X: stands for N, and
- *: is the point of attachment to the carbon atom,
and wherein
- R¹: stands for 3-pyridyl, and
- R²: stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part is substituted with three fluorine atoms;
wherein in formula (ii)
- Y: stands for CH2 or C-NH2,
- Z: stands for CH or N, and
- *: is the point of attachment to the carbon atom,
and wherein
- R¹: stands for 3-pyridyl,
- R²: stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part can be substituted with three fluorine atoms,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the present invention are the compounds of formula (I) 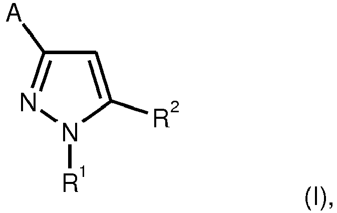 wherein
- A: stands for a group of formula (i) or (ii) as represented above,
wherein formula (i) is selected from a group consisting of 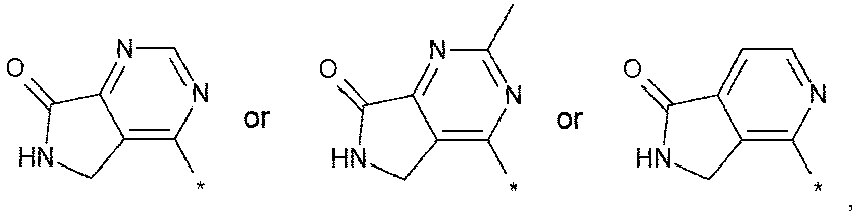 and wherein
- R¹: is selected from a group consisting of 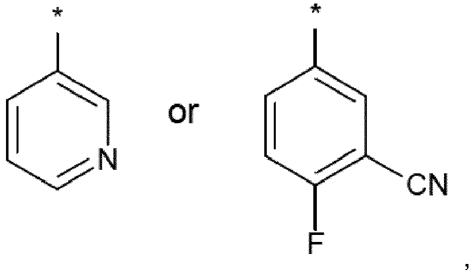
and wherein
- R²: is 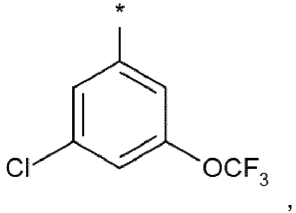
and wherein
- *: is the point of attachment to the carbon atom;
wherein formula (ii) is selected from a group consisting of 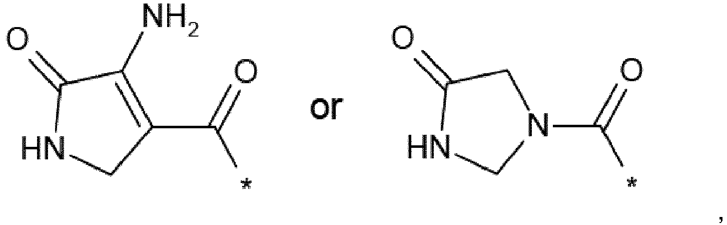 and wherein
- R¹: is selected from a group consisting of 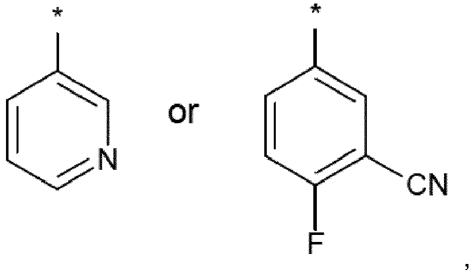
and wherein
- R²: is 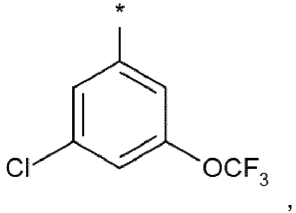
and wherein
- *: is the point of attachment to the carbon atom
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the present invention are the compounds of the represented formula (I),
wherein said compound is 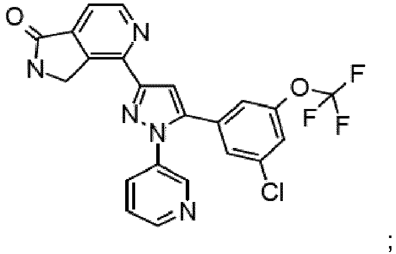 i.e. 4-[5-[3-Chloro-5-(trifluoromethoxy) phenyl]-1-(pyridin-3-yl)-1H-pyrazol-3-yl]-2,3-dihydro-1H-pyrrolo[3,4-c]pyridin-1-one,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the invention are the compounds of the represented formula (I),
wherein said compound is 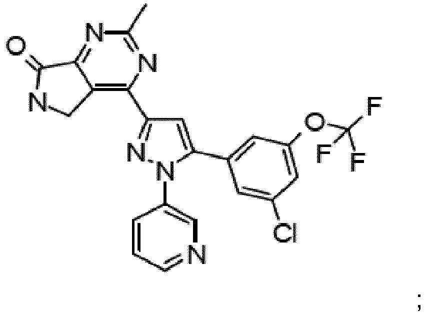 i.e. 4-[5-[3-Chloro-5-(trifluoromethoxy) phenyl]-1-(pyridin-3-yl)-1H-pyrazol-3-yl]-5,6-dihydro-2-methyl-7H-pyrrolo[3,4-d] pyrimidin-7-one,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the invention are the compounds of the represented formula (I),
wherein said compound is 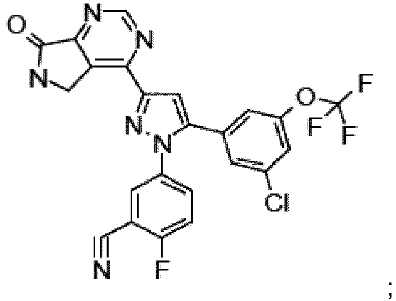 i.e. 4-[5-[3-Chloro-5-(trifluoromethoxy) phenyl]-1-(3-cyano-4-fluoro-phenyl)-1 H-pyrazol-3-yl]-5,6-dihydro-7H-pyrrolo[3,4-d] pyrimidin-7-one,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the invention are the compounds of the represented formula (I),
wherein said compound is 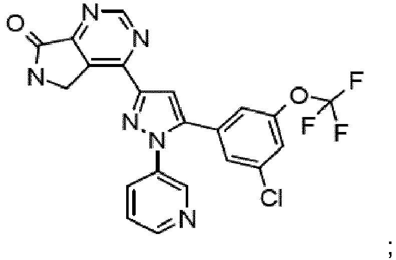 i.e. 4-[5-[3-Chloro-5-(trifluoromethoxy) phenyl]-1-(pyridin-3-yl)-1H-pyrazol-3-yl]-5,6-dihydro-7H-pyrrolo[3,4-d]pyrimidin-7-one,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the invention are the compounds of the represented formula (I),
wherein said compound is 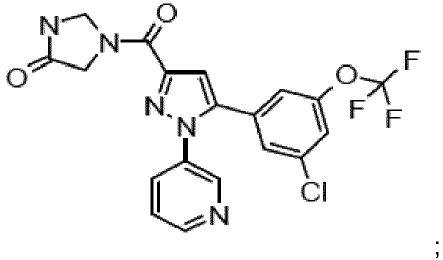 i.e. 1-[[5-[3-Chloro-5-(trifluoromethoxy) phenyl]-1-(pyridin-3-yl)-1H-pyrazol-3-yl]carbonyl]-imidazolidin-4-one,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

With the above context, in another aspect, subject matter of the invention are the compounds of the represented formula (I),
wherein said compound is 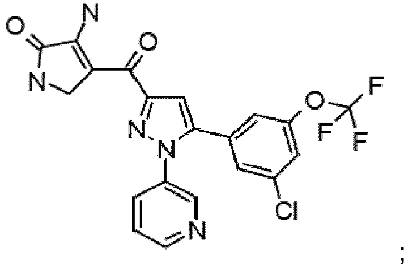 i.e. 3-Amino-4-[5-[3-chloro-5-(trifluoromethoxy) phenyl]-1-(pyridin-3-yl)-1H-pyrazol-3-yl-carbonyl]-1,5-dihydro-2H-pyrrol-2-one,
and their co-crystals or the polymorphs of their co-crystals, and their salts, their solvates or the solvates of their salts
for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

Provided that the compounds in accordance with the invention can occur in tautomeric forms, the present invention comprises any tautomeric form for its use in a method of treating and/preventing the aforementioned cardiovascular diseases and / or cardiovascular comorbidities thereof.

Isomeres are to be understood as tautomeres or tautomeric forms within the scope of the present invention, which are characterized in that said isomeres have the same chemical formula as their original compound in accordance with the respective formula (I) or their co-crystals or the polymorphs of their co-crystals, or their salts, their solvates or the solvates of their salts; isolated atoms, however, can be positionally arranged differently.

Given the context of the present invention, these isomers are therefore described as tautomeres or tautomeric forms because they can quickly interconvert into each other through the migration of isolated atoms or atom groups, and, in so doing, the respective isomers can be in a rapidly changing chemical equilibrium with one another.

Given the context of the present invention, the tautomers or tautomeric forms thus only differ in the position of a chemical group and in the position of a double bond and can be understood as the same chemical compound in accordance with the formula (I) or their co-crystals or the polymorphs of their co-crystals, or their salts, their solvates or the solvates of their salts.

As salts, within the scope of the present invention, physiologically acceptable salts of the compounds pursuant to the invention are preferred. Comprised, however, are also salts, which are not suitable for pharmaceutical administration itself, but can, for example, be used for the isolation or cleaning of the compounds in accordance with the invention.

Physiologically acceptable salts of the compounds of the invention include acid addition salts of mineral acids, carboxylic acids, and sulfonic acids, e.g. salts of hydrochloric acid, hydrobromic acid, sulfuric acid, phosphoric acid, methane sulfonic acid, ethane sulfonic acid, p-toluene sulfonic acid, benzene sulfonic acid, naphthalene disulfonic acid, acetic acid, trifluoroacetic acid, propionic acid, lactic acid, tartaric acid, malic acid, citric acid, fumaric acid, maleic acid and benzoic acid.

Physiologically acceptable salts of the compounds in accordance with the inventions also comprise salts of customary bases, such as exemplary and preferably alkali metal salts (e.g., sodium and potassium salts), alkaline earth metal salts (e.g., calcium and magnesium salts) and ammonium salts, derived from ammonia or organic amines with 1 to 16 C atoms, such as exemplarily and preferably ethylamine, diethyl amine, triethyl amine, ethyl diisopropylamine, monoethanolamine, diethanolamine, triethanolamine, dicyclohexylamine, dimethylaminoethanol, procaine, dibenzylamine, *N*-methylmorpholine, arginine, lysine, ethylendiamine and *N*-methylpiperidine.

Solvates within the scope of the invention are those forms of the compounds in accordance with the invention that in a solid or liquid state forms a complex through coordination with solvent molecules. Hydrates are a special form of the solvate in which the coordination takes place with water.

Accordingly, within the scope of the present invention the term "solvates" also always includes their hydrates with the above-cited definition.

Co-crystals within the scope of the invention are those solid forms of the compounds in accordance with the invention that are crystalline single phase materials composed of two or more different molecular and/or ionic compounds generally in a stoichiometric ratio which are neither solvates nor simple salts as defined above.

With this context, in accordance with the invention, the difference between a crystalline salt and a co-crystal lies in the transfer of a proton. The transfer of protons from one component to another in a crystal is dependent on the environment. For this reason, crystalline salts and co-crystals may be thought of in accordance with the invention as two ends of a proton transfer spectrum, where the salt has completed the proton transfer at one end and an absence of proton transfer exists for co-crystals at the other end.

Polymorphs of co-crystals within the scope of the invention are those solid forms of the above-defined co-crystals, which, however, exist in more than one form or co-crystal structure. Said polymorphs of co-crystals may vary in certain morphologies of those solid forms, such as the variables of crystal habit, amorphous fraction or crystallographic defects.

Within the scope of the present invention the substituents for the general compounds of formula (I), unless otherwise specified, have the following meaning:

Alkyl as well as the alkyl parts in alkoxy stand for branched or unbranched alkyl and, unless otherwise stated, comprise (C1-C6)-alkyl, in particular (C1-C4)-alkyl, such as, e.g., methyl, ethyl, propyl, isopropyl, butyl, isobutyl, tert. butyl.

Alkoxy within the scope of the invention preferably stands for a branched or unbranched alkoxy residue, in particular with 1 to 6, 1 to 4 or 1 to 3 carbon atoms. Preferred is an unbranched or branched alkoxy residue with 1 to 3 carbon atoms. Exemplarily and preferably, reference is made to: methoxy, ethoxy, n-propoxy, isopropoxy, t-butoxy, n-pentoxy and n-hexoxy.

Halogen stands for fluorine, chlorine, bromine or iodine, with fluorine and chlorine being preferred, unless otherwise specified.

In the general formula (I) of the group that can stand for residue "A", the end point of the line, next to which is an "*", does not stand for a carbon atom or a CH2-group but is a component of the bond to the atom, to which said residue "A" is bound.

The above stated general definitions of residues or those regarding preferred ranges apply both to the end products of the general formula (I) and analogously also to their co-crystals or polymorphs of said co-crystals, their salts, solvates and solvates of their salts, and analogously also to the respective starting material or intermediate products thereof required for manufacturing (see further below).

Regarding the description of the residues in accordance with the invention of the general formula (I), the following applies:

The respective combinations or preferred combinations of residues specifically stated in the definitions of residues are also optionally replaced by residue definitions of other combinations independently of the respectively stated combinations of residues.

Also disclosed herein are methods for the general synthesis of the compounds of formula (I) as defined above, wherein A stands for a group of formula (i) 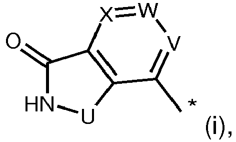 whereby a compound of formula (II) 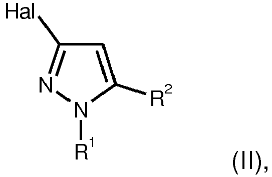 wherein
Hal stands for chlorine, bromine or iodine and
R¹ and R² have the meaning as specified above,
is reacted with a compound of formula (III) 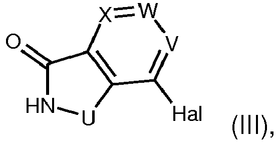 wherein
Hal stands for chlorine, bromine or iodine and
U, V, W and X have the meaning as specified above.

Methods for the synthesis of the compounds of general formula (I), wherein A stands for a group of formula (i) in accordance with the present invention are described in more detail in WO 2014/027112, which is hereby incorporated by reference.

Said methods for the synthesis of the compounds of general formula (I), wherein A stands for a group of formula (i) in accordance with the present invention, can be summarized in the following general synthesis scheme:

### General synthesis scheme:

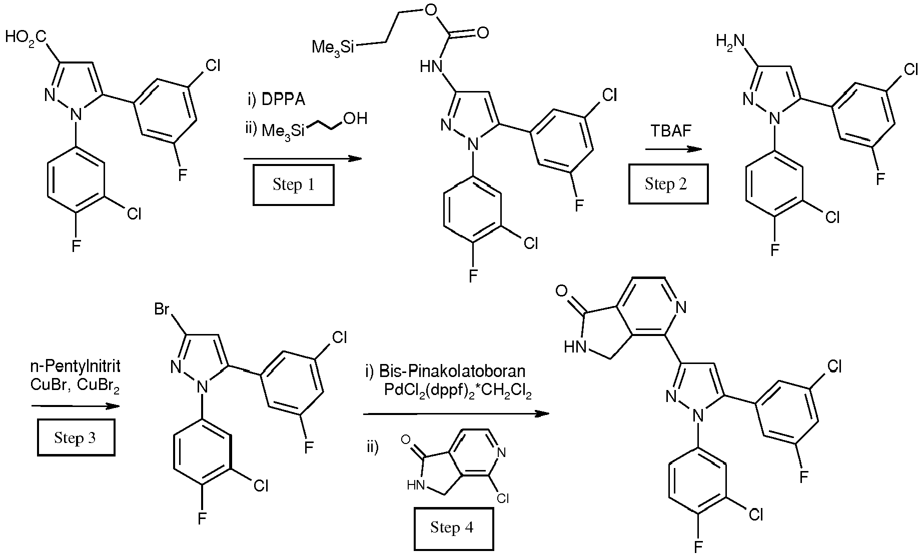

The conversion generally takes place in two stages in inert solvents, first forming an organo-metallic component, followed by the conversion in the presence of a catalyst complex and a base, preferably in a temperature range from 50 °C to 150 °C under high pressure and the exclusion of oxygen.

The compounds of general formula (I), wherein A stands for a group of formula (i) in accordance with the present invention, produced in accordance with the method specified above may have protective groups that can be cleaved, in accordance with the conditions known to one skilled in the art, in order to obtain additional compounds of formula (I).

The compounds of general formula (I), wherein A stands for a group of formula (i) in accordance with the present invention, produced in accordance with the method specified above can be converted into additional compounds of formula (I) by selective oxidation using oxidizing agents known to one skilled in the art.

Also disclosed herein are methods for the general synthesis of the compounds of formula (I), wherein A stands for a group of formula (ii) 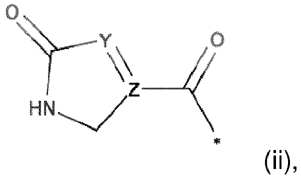 whereby a compound of Formula (IV) 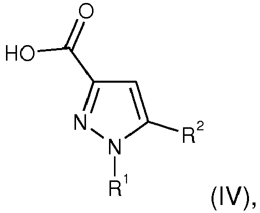 in which
R¹ and R² have the above-indicated meaning,
is reacted with a 5- to 6-membered heterocyclic compound or a salt thereof.

In general, the reaction takes place in inert solvents, in the presence of a dehydrating reagent, optionally in the presence of a base, preferably in a temperature range of -30 °C to 50 °C at normal pressure.

The manufacture of the compounds of general formula (I), wherein the group A stands for formula (ii) in accordance with the invention, can be illustrated by the following synthesis diagram.

### Synthesis Diagram:

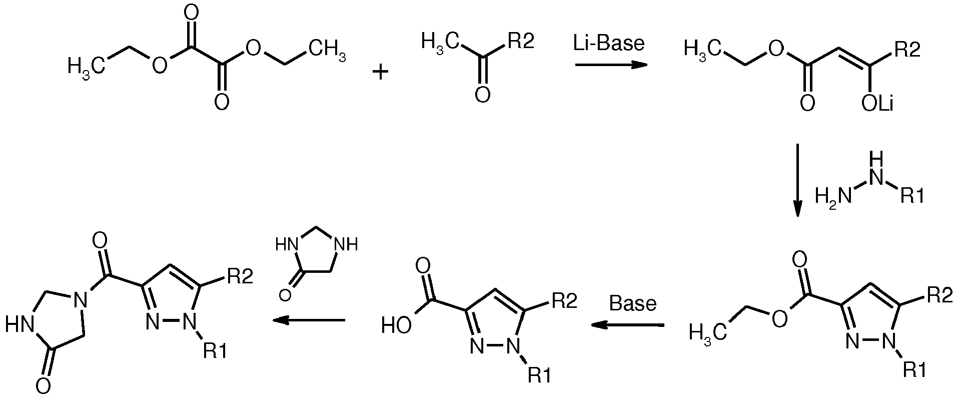

Methods for the synthesis of the compounds of general formula (I), wherein A stands for a group of formula (ii) in accordance with the present invention, are described in more detail in WO 2013/079586, which is hereby incorporated by reference.

Given the general synthesis for the compounds of formula (I), wherein A stands for a group of formula (i) in accordance with the present invention, the inventors also found novel processes for the synthesis of the particular compound 4-[5-[3-Chloro-5-(trifluoromethoxy) phenyl]-1-(pyridin-3-yl)-1H-pyrazol-3-yl]-5,6- dihydro-7H-pyrrolo[3,4-d] pyrimidin-7-one; i.e. compound A with the context of the present invention.

The below schemes depict said new processes from the starting materials through intermediate compounds towards the finally synthesized compound A:

### Sythesis Phase I - starting materials / intermediate of Compound 5

### Synthesis of an acetyl-pyrazyl intermediate for the synthesis of 4-[5-[3-Chloro-5-(trifluoromethoxy) phenyl]-1-(pyridin-3-yl)-1H-pyrazol-3-yl]-5,6-dihydro-7H-pyrrolo[3,4-d]pyrimidin-7-one (i.e. compound A)

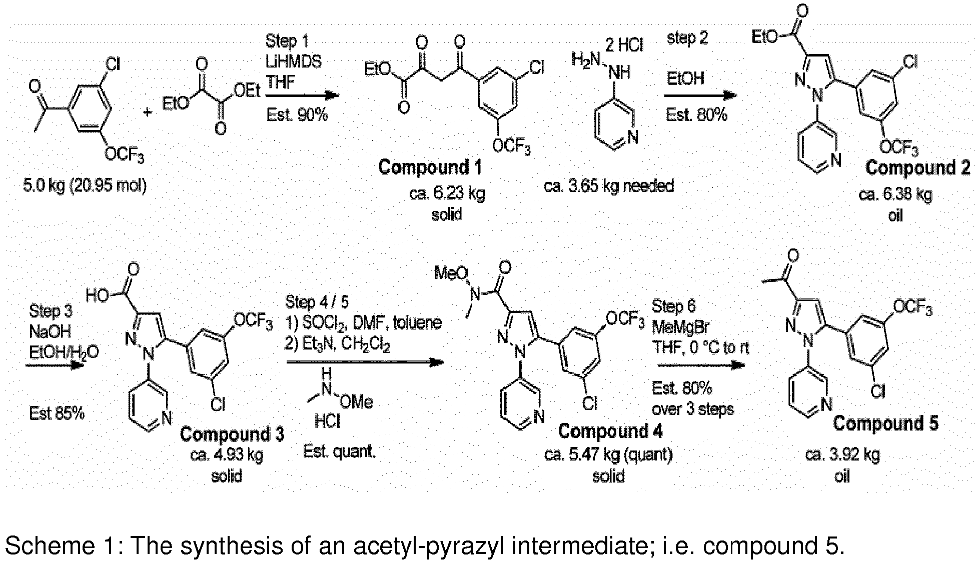

Thus, in another aspect, subject matter of the present invention is a process for the synthesis of 4-[5-[3-Chloro-5-(trifluoromethoxy) phenyl]-1-(pyridin-3-yl)-1 H-pyrazol-3-yl]-5,6- dihydro-7H-pyrrolo[3,4-d] pyrimidin-7-one (compound A), wherein said process comprises the following starting materials and the synthesis of an acetyl-pyrazyl intermediate; i.e. compound 5. While referring to scheme 1 above, said pre-synthesis comprises the following steps:
1) The synthesis of compound 5 may be carried out with LiHMDS as base as shown in the above scheme 1. Preferably, in accordance with the invention, sodium or potassium ethoxide as alternative base can be used for the addition of acetophenones to diethyl oxalate; see scheme 2 below: 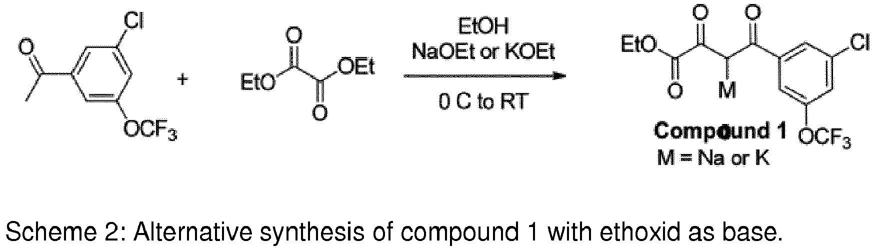
1a) Optionally, the solvent volume in step 1) may be adjusted and the use of MTBE may be avoided to perform the reaction at reflux temperature to influence the particle size of the formed suspension and thereby to shorten filtration times of the obtained potassium salt.
1b) Preferably, with the context of the present invention for the synthesis of compound 1 of scheme 1, in e.g. a 20 L reactor with mechanical stirring under nitrogen atmosphere, to a mixture of 1-(3-chloro-5-(trifluoromethoxy)phenyl)ethanone (exemplarily 500 g, 2096 mmol) and diethyl oxalate (exemplarily 337 g, 2305 mmol, 0.293 L) in ethanol (exemplarily 12 L), a solution of potassium ethoxide (exemplarily 213 g, 2410 mmol, 95 %) in ethanol (exemplarily 2 L) at 80 °C (total addition time of the base ca. 1 hour) is added dropwise. Solid material is formed and the mixture may become very thick, however, it remains stirrable at preferably 500 rpm. After the addition of base is completed, the mixture is stirred for one additional hour. The reaction mixture is then cooled to approximately 10 °C, stirred for approximately 30 minutes and then filtered off on, e.g. a Büchner funnel with a paper filter. The reactor walls are rinsed with ethanol (exemplarily 0.5 L). Most of the ethanol should be removed by filtration. The solid residue is washed with exemplarily 3 L MTBE and the solid is dried in a vacuum stove. Crude yield: approximately 695 g yellow solid (88 %).
2) Synthesis of compound 2 follows from 3-hydrazinylpyridine dihydrochloride and compound 1 as depicted by the scheme 3 below: 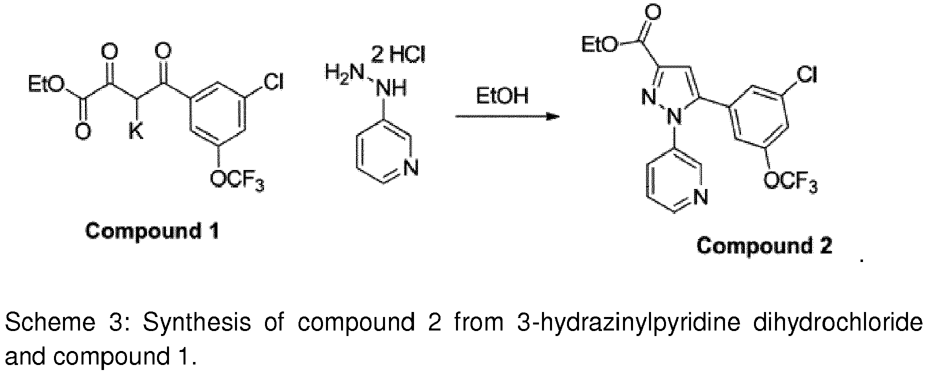
2a) Preferably, with the context of the present invention for the synthesis of compound 2, the e.g. 20 L reactor with mechanical stirring under nitrogen atmosphere, is charged with ethanol (Abs) (exemplarily 15 L) and 3-hydrazinylpyridine dihydrochloride (exemplarily 1040 g, 5713 mmol). To said stirred suspension at room temperature (1-(3-chloro-5-(trifluoromethoxy)phenyl)-4-ethoxy-1,3,4-trioxobutan-2-yl)potassium (exemplarily 2050 g, 5441 mmol) is added portion wise. The formed suspension is heated to reflux (approximately 78 ºC) for about 2 hours. The mixture is cooled to room temperature (rt) and is filtered using a glass filter to remove the formed potassium chloride. The clear yellow/orange filtrate is concentrated *in vacuo* to yield a sticky (probably not completely dry) solid, which is further triturated in MTBE (exemplarily 2.5 L). The solids are filtered off to yield approx. 2.005 kg (89 %) of a pale yellow solid.
3) Synthesis of compound 3 from compound 2 follows using preferably the potassium ethoxide method as depicted in the scheme 4 below: 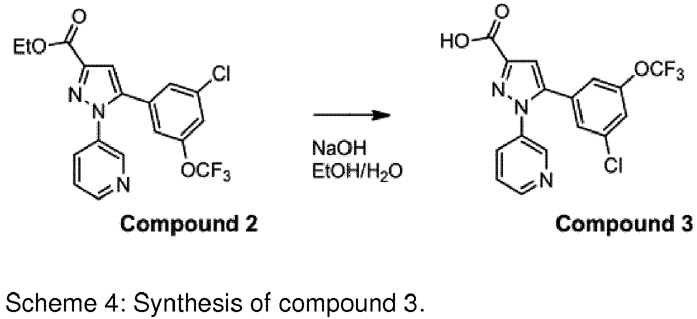
3a) Extra water may be added to redissolve the solids and to remove most of the ethanol after acidification and concentration of the reaction mixture. The product of compound 3 is filtered off.
3b) Preferably, a 1:1 mixture of water and ethanol shall be used instead of pure ethanol as solvent and gentle heating to 40 °C being applied, followed by saponification, acidifcation with HCl, precipitation of compound 3 and collection thereof by filtration. A drying procedure using Dean-Stark conditions may follow to remove residual water.
3c) More preferred for synthesis of compound 3, to a cooled (approximately 0 °C) suspension of ethyl 5-(3-chloro-5-(trifluoromethoxy)phenyl)-1-(pyridin-3-yl)-1 H-pyrazole-3-carboxylate (exemplarily 1128 g, 2739 mmol) in ethanol (exemplarily 5 L) and water (exemplarily 5 L) is added a solution of sodium hydroxide (exemplarily 548 g, 1.37 mol) in water (exemplarily 5 L) dropwise while keeping the mixture below approx. 5 °C. Stirring follows for approx. 30 minutes while slowly allowing the mixture to warm to approx. 20 °C. During the addition of the NaOH and warming up, a solid precipitates. The mixture is warmed to approx. 40 °C. A clear solution forms. Slow addition of hydrochloric acid (exemplarily 1350 g, 1.37 mol, 1.144 L, 37 %) follows, while the mixture is still at about 40 °C until pH of the mixture is around 3. During addition of the acid, a solid is formed. The solids are to be filtered off using e.g. a Büchner funnel with a paper filter and dried in vacuum stove. Yield exemplarily: 1129 g yellowish solid (107 % yield, still contains some water).
4) Synthesis of compound 4 (see Scheme 5 below) preferably follows after the Dean-Stark destillation of step 3b) in toluene followed by the preparation of the acid chloride using thionyl chloride. Subsequently, a solvent swap from toluene to DCM and removal of the excess thionyl chloride follows for the subsequent Weinreb amide synthesis.
4a) The acid chloride synthesis may be performed by using a suitable glass reactor with mechanical stirring under nitrogen atmosphere. The nitrogen outlet of the reactor may be quenched by a NaOH solution. The amide synthesis may be performed while using a suitable glass reactor with mechanical stirring under nitrogen atmosphere, as well (see scheme 5 below): 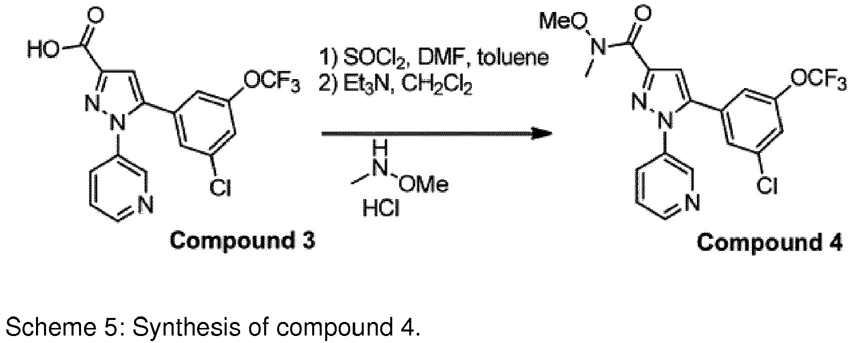
4b) Preferably, for the synthesis of compound 4, a suspension of 5-(3-chloro-5-(trifluoromethoxy)phenyl)-1-(pyridin-3-yl)-1H-pyrazole-3-carboxylic acid (exemplarily 919 g, 2395 mmol) in toluene (dry) (exemplarily 7500 ml) is heated to reflux under Dean-Stark conditions to remove any residual water. Followed by cooling the mixture to approximately 70°C and then carefully thionyl chloride (exemplarily 570 g, 4790 mmol, 350 ml) and DMF (exemplarily 4.38 g, 59.9 mmol, 4.64 ml) are added. The mixture is warmed to reflux and stirred for approx. 4 hours. A yellow suspension remains. The reaction mixture is concentrated to dryness *in vacuo.* The obtained distillate containing the excess of SOCl2 is discarded. The obtained oil/solid is transferred to exemplarily a 10 L flask fitted with a mechanical top stirrer using dichloromethane (exemplarily 5000 ml). N,O-dimethylhydroxylamine hydrochloride (exemplarily 280 g, 2874 mmol) is added and the mixture is cooled to approx. 0 °C. Then slowly neat triethylamine (exemplarily 727 g, 7185 mmol, 1001 ml) is added dropwise. The cooling bath is removed and the mixture is stirred at room temperature. Water (exemplarily 1 L) is added and the mixture is stirred vigorously. The layers are separated and the organic phase is washed with 50% diluted sat. aq. Na2CO3 (1 L), dried on Na2SO4 and concentrated *in vacuo.* The crude oil is triturated in heptane (exemplarily 1.5 L). The formed solids are filtered off and dried in a vacuum stove to exemplarily yield 872 g of a yellow solid (85%).
5) A Grignard reaction towards the intermediate 5 follows using methylmagnesium chloride (see scheme 6 below): 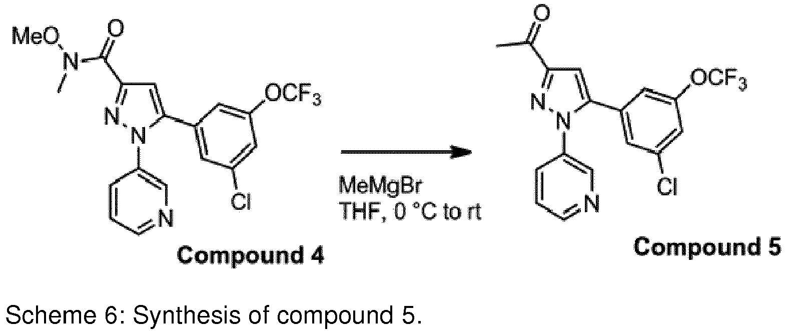
5a) Compound 5 is obtained following extraction, concentration and trituration procedure using conventional laboratory glassware.
5b) Preferably, to obtain compound 5 with the context of the present invention, in exemplarily a 10 L three necked glass roundbottom flask with mechanical stirring under nitrogen atmosphere, to a stirred solution of 5-(3-chloro-5-(trifluoromethoxy)phenyl)-N-methoxy-N-methyl-1-(pyridin-3-yl)-1 H-pyrazole-3-carboxamide (exemplarily 954 g, 2235 mmol) in tetrahydrofuran (dry) (exemplarily 6 L) is slowly added methylmagnesium chloride in THF (3M) (exemplarily 2682 mmol, 0.894 L) at approx. 0 °C dropwise over a period of approx. 45 minutes. After approx. 30 minutes, a precipitate is formed; the mixture remains stirrable. The reaction mixture is poured in a mixture of approx. 2.5 L sat. aq. NH4Cl and 2.5 L water. The layers are separated and the organic phase is dried on Na2SO4 and then concentrated *in vacuo* to afford a brown oil. The oil is triturated with MTBE (exemplarily 50 mL) and heptane (exemplarily 2 L). The formed solid is filtered and washed with heptane (exemplarily 500 mL) to exemplarily yield 713 g of a beige solid (84 %).

### Analysis of exemplary batches of compound 5 synthesised by the first synthesis phase described above

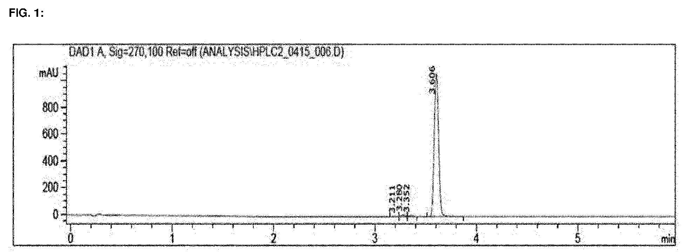

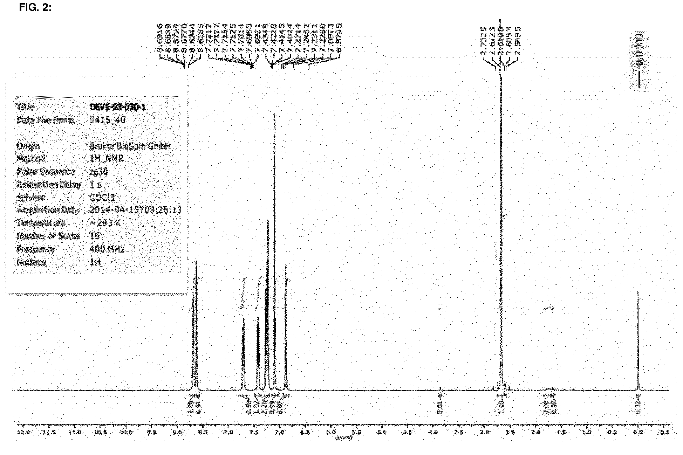

All prepared batches of compound 5 according to the above-described synthesis phase I were combined and mixed thorougly. Five approx. 1 g samples were taken from the test container, combined and ground into a fine powder using a mortar and pestle. Analysis of this material with HPLC (see Figure 1) showed the purity to be 97.9% and analysis with 1H-NMR (see Figure 2) confirmed the structure.

In total, in an exemplary synthesis according to synthesis phase I, 4.18 kg of compound 5 was prepared from 4.98 kg of 1-(3-chloro-5-(trifluoromethoxy)phenyl)ethanone and 3.32 kg 3-hydrazinylpyridine dihydrochloride.

### Synthesis Phase II

### Synthesis of 4-[5-[3-Chloro-5-(trifluoromethoxy) phenyl]-1-(pyridin-3-yl)-1 H-pyrazol-3-yl]-5,6-dihydro-7H-pyrrolo[3,4-d]pyrimidin-7-one (i.e. compound A)

The below schemes depict exemplarily the process of synthesis phase II:

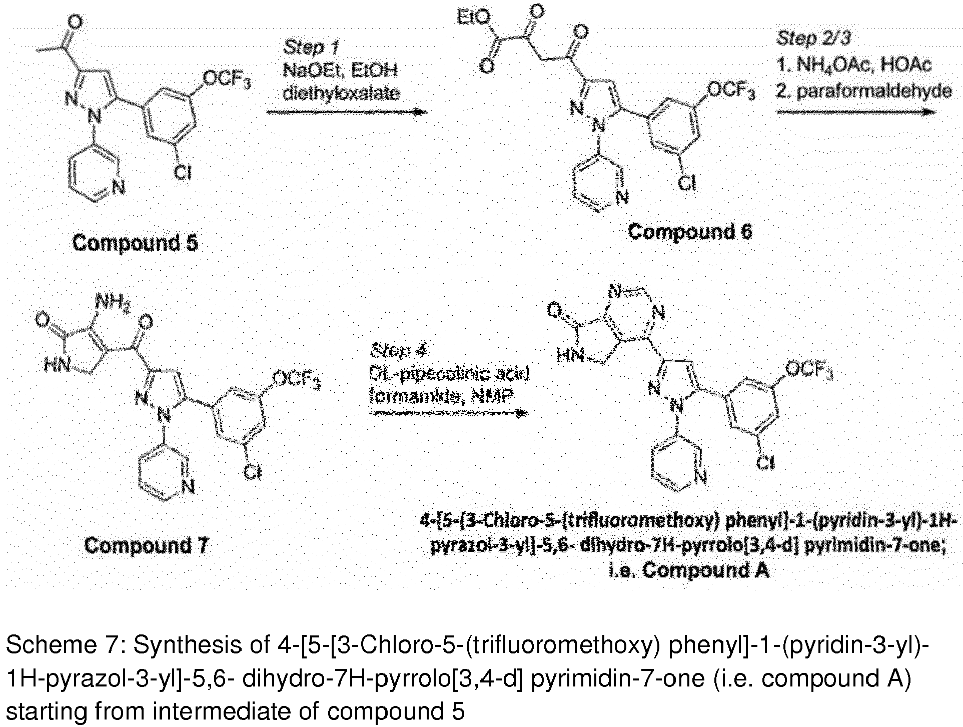

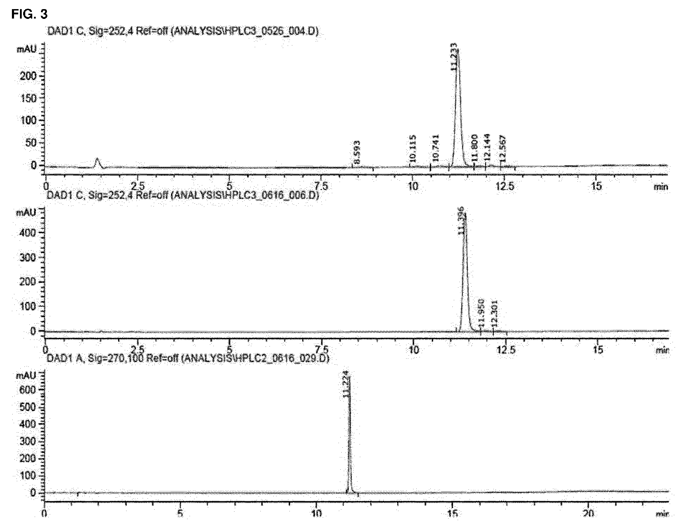

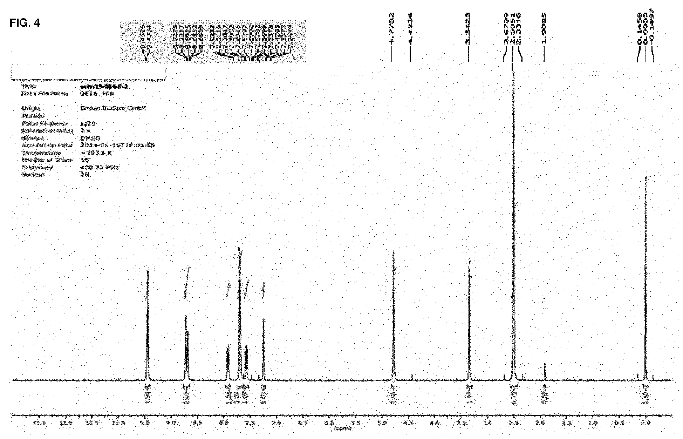

The synthesis phase II starts from intermediate compound 5:
6) A solution of compound 5 and diethyloxalate in THF is treated with a solution of sodium ethoxide in ethanol. Followed by quenching the solution according to step 1) with acetic acid, aqueous work-up and subsequent trituration with ethanol, compound 6 is obtained.
6a) Preferably, with the context of the present invention, to a solution of 1-(5-(3-chloro-5-(trifluoromethoxy)phenyl)-1-(pyridin-3-yl)-1 H-pyrazol-3-yl)ethanone (exemplarily 580 g, 1519 mmol) in tetrahydrofuran (dry) (exemplarily 3.5 L) is added diethyl oxalate (exemplarily 278 g, 1899 mmol, 0.258 L). Then a solution of sodium ethoxide in ethanol (exemplarily 689 g, 2127 mmol, 0.794 L, 21 %) is added dropwise at room temperature in approx. 20 minutes. The mixture is stirred for 1 hour. Acetic acid (exemplarily 137 g, 2279 mmol, 0.130 L) is added to quench the reaction and the mixture was stirred for approx. 15 minutes. The reaction mixture is poured into water (exemplarily 8 L) while stirring vigorously to form a sticky solid. After stirring over approx. 48 hours, the mixture is allowed to settle. The liquid phase is decanted off and the obtained sticky solids are vigorously stirred in ethanol (exemplarily 800 mL). The suspension is filtered and the solid is washed with a minimal amount of fresh ethanol on the filter. The solid is then dried in a vacuum stove to exemplarily yield 540 g yellow solid.
7) Following this, compound 6 is added to a solution of an excess of ammonium acetate in acetic acid and the mixture is reacted at approx. 35°C, preferably overnight, resulting into the enamine compound 6a (see scheme 8 below): 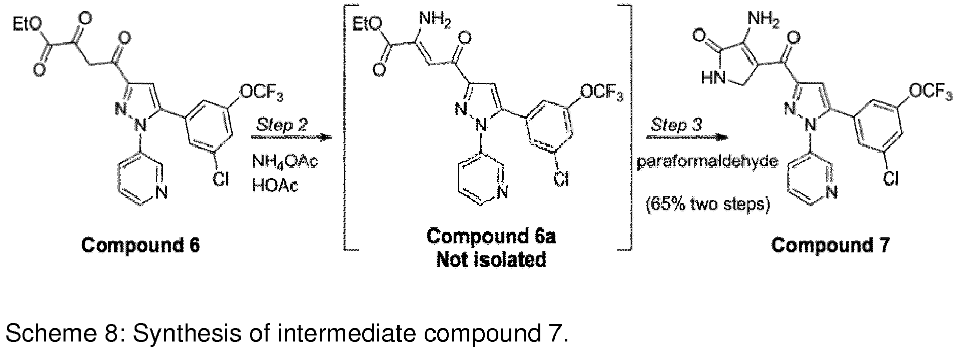 The enamine intermediate compound 6a is reacted with paraformaldehyde at room temperature (rt). After reacting, preferably overnight, typically a complete conversion towards compound 7 takes place as the main reaction product. After aqueous work-up and subsequent trituration with 2-propanol, compound 7 is obtained as an off-white powder.
7a) Preferably, with the context of the present invention, to a warm (approx. 30°C) solution of ammonium acetate (exemplarily 1123 g, 14.6 mol) in acetic acid (exemplarily 5.4 L) is added ethyl 4-(5-(3-chloro-5-(trifluoromethoxy)phenyl)-1-(pyridin-3-yl)-1H-pyrazol-3-yl)-2,4-dioxobutanoate (exemplarily 540 g, 1121 mmol). The mixture is stirred at approx. 35°C (internal temperature) overnight, so to afford a light brown solution. After nearly complete conversion, the mixture is cooled to room temperature. Then, paraformaldehyde (exemplarily 53.8 g, 1793 mmol) is added and the mixture is stirred overnight to afford a yellow suspension. The reaction mixture is poured into approx. 10 L water (optionally divided over two flasks). The content of each flask is stirred for approx. 15 minutes and the solids are filtered off (exemplarily 4L glass filter P3, rinsed with 2 x 1 L water, stirred). The solids are combined into exemplarily a 5 L flask and exemplarily 1500 mL of 2-propanol is added. The resulting suspension is stirred over approx. 48 hours and the solids are isolated by filtration. The isolated material is subsequently washed on the filter with 2-propanol (exemplarily 2 x 500 mL) and dried in vacuum stove to exemplarily yield 345 g of an off white solid.
8) Synthesis of 4-[5-[3-Chloro-5-(trifluoromethoxy) phenyl]-1-(pyridin-3-yl)-1 H-pyrazol-3-yl]-5,6- dihydro-7H-pyrrolo[3,4-d] pyrimidin-7-one (i.e. compound A) follows:
8a) Preferably, with the context of the present invention, an exemplary 4 L three necked round bottom flask (fitted with reflux condenser and a nitrogen inlet at the stirrer adapter to prevent clogging) is charged with 3-amino-4-(5-(3-chloro-5-(trifluoromethoxy)phenyl)-1-(pyridin-3-yl)-1H-pyrazole-3-carbonyl)-1H-pyrrol-2(5H)-one (exemplarily 203.8 g, 439 mmol), DL-pipecolinic acid (exemplarily 283 g, 2197 mmol), N-methyl-2-pyrrolidinone (exemplarily 1 L) and formamide (exemplarily 1 L). The reflux condenser is closed with a bubble counter. The suspension is heated using a heating mantle to afford a dark brown solution at approx. 130 °C. The mixture is carefully heated further to approx. 150 - 155 °C (internal temperature) and it is to be closely monitored that the reaction temperature remains in this specific temperature region. The reaction is therefore not heated overnight, but allowed to cool to room temperature, so to afford a thick suspension and re-heated after approx. 10-12 hours to again form a solution.
   When less than 1 area % of the starting material (compound 7) is observed with HPLC analysis (generally requiring approx. 17 hours of reaction time), the reaction is stopped and the hot reaction mixture (approx. 140°C) is poured gently to 10 L of warm water (approx. 60°C, divided over two flasks) while stirring vigorously to form brown suspensions. The suspensions were allowed to cool to room temperature overnight. The solids from each flask were filtered and washed with 4 x 1 L water on the filter. The crude material is dried in a vacuum stove to afford a brown powder (see scheme 9 below): 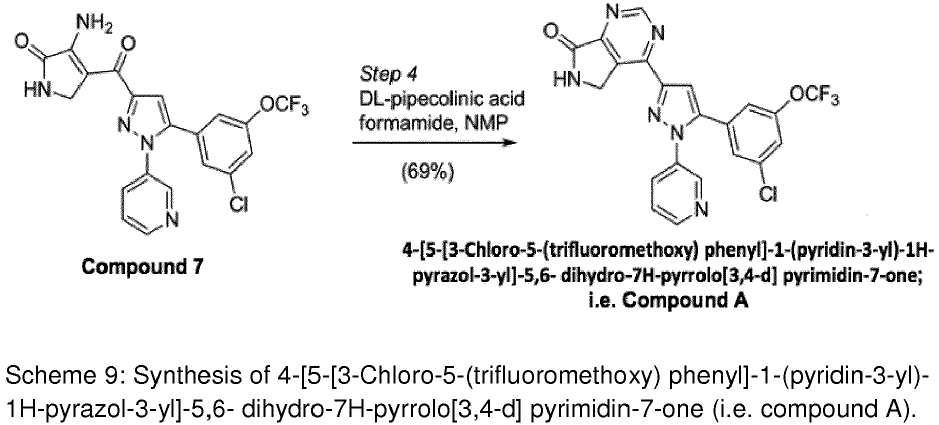 Because the yield is typically > 100 % it is assumed that the crude material still contains some water. Because water is used in the subsequent purification step as solvent, said water can be used for purification.
9) Purification of 4-[5-[3-Chloro-5-(trifluoromethoxy) phenyl]-1-(pyridin-3-yl)-1H-pyrazol-3-yl]-5,6- dihydro-7H-pyrrolo[3,4-d] pyrimidin-7-one (i.e. compound A) follows:
   As stated above, water is used in the subsequent purification step as solvent without any preceding drying step of the crude 4-[5-[3-Chloro-5-(trifluoromethoxy) phenyl]-1-(pyridin-3-yl)-1 H-pyrazol-3-yl]-5,6- dihydro-7H-pyrrolo[3,4-d] pyrimidin-7-one (i.e. compound A) obtained in step 4).
   Crude 4-[5-[3-Chloro-5-(trifluoromethoxy) phenyl]-1-(pyridin-3-yl)-1 H-pyrazol-3-yl]-5,6- dihydro-7H-pyrrolo[3,4-d] pyrimidin-7-one (i.e. compound A) is dissolved in acetic acid under gentle warming to approx. 45 °C (internal temperature) and then water is added dropwise to the warm solution, preferably over approx. 2 hours. The dark brown solution obtained is then allowed to cool slowly to room temperature, preferably overnight.
   The resulting suspension is filtered off and the collected solids are rinsed on the filter with acetic acid/water mixture (1:1) followed by washing on the filter with water.
   The solids are suspended in water and saturated NaHCO3 solution is added (pH 8). The resulting suspensions are allowed to cool down and filtered off. The collected solids are washed on the filter with water.
9a) Preferably, with the context of the present invention, the purification of 4-[5-[3-Chloro-5-(trifluoromethoxy)phenyl]-1-(pyridin-3-yl)-1H-pyrazol-3-yl]-5,6-dihydro-7H-pyrrolo[3,4-d] pyrimidin-7-one (i.e. compound A) comprises the following steps:
   The crude compound A is exemplarily divided in 3 batches of approx. 600 g (containing approx. 90 g water). Each batch is dissolved in exemplary 2590 mL of acetic acid under gentle warming to approx. 45 °C (internal temperature) and then exemplary 2500 mL water is added dropwise to the warm solution over approx. 2 hours. The dark brown solutions are then allowed to cool down slowly to room temperature overnight. The resulting suspensions are filtered off (exemplary two 4 L filters) and the collected solids were rinsed on the filter with 1 L acetic acid/water (1:1) followed by washing on the filter with 3 x 2 L water/filter.
   The solids, collected on the two filters by the above described method, were analyzed with HPLC, which showed that the purity of the material in all cases was > 97 % (see Figure 3). 1H-NMR analysis showed that the material contained 30-35 mol % acetic acid (see Figure 4).
10) The final compound is isolated as the free base.

In total, exemplarily 1.13 kg of compound A (purity > 98 %) were prepared from 3.48 kg of 1-(5-(3-chloro-5-(trifluoromethoxy)phenyl)-1-(pyridin-3-yl)-1H-pyrazol-3-yl)ethanone (compound 5). Because the quality of the first two batches of crude compound A was not sufficient, those were held separate and were not combined with the other batches for preparation of the final 1.13 kg of compound A. As a result, the 1.13 kg of compound A was prepared from 1.6 kg of compound 7 (2.1 kg prepared in total).

Based on the above described synthesis routes I and II, in another aspect of the invention, the inventors found the following generalized process to obtain the compound A in kg-amounts; see scheme 10 below.

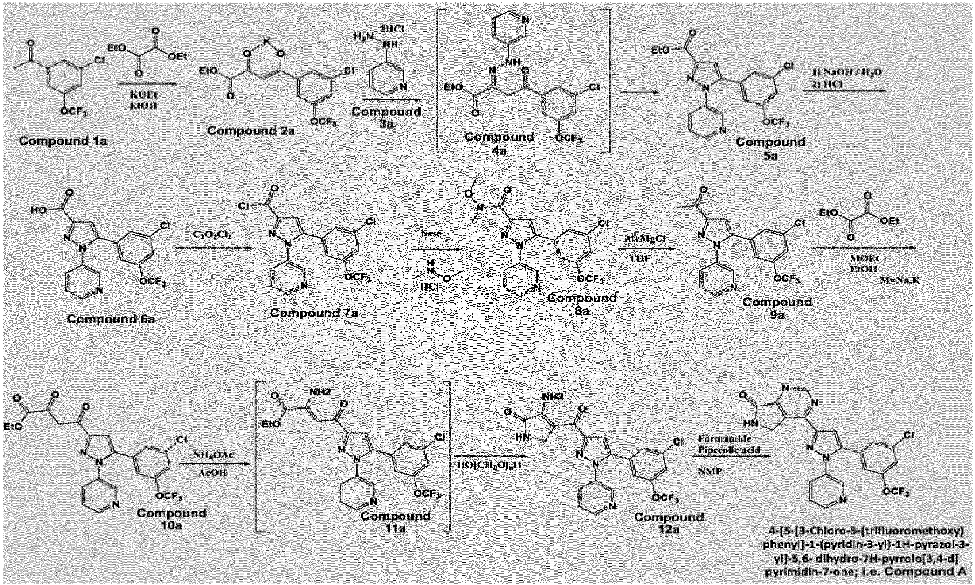

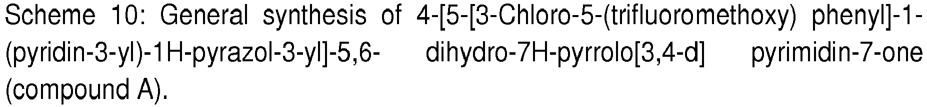

### General process description for synthesizing compound A

The general process comprises 11 chemical transformations and follows a linear synthesis approach starting from 3-chloro-5-(trifluoromethoxy)acetophenone (i.e. compound 1 a, see Scheme 10). The second key raw material used is 3-hydrazinepyridine dihydrochloride (i.e. compound 3a; see Scheme 10). 5-(3-chloro-5-(trifluoromethoxy)phenyl)-1-(pyridin-3-yl)-1H-pyrazole-3-carboxylic acid (i.e. compound 6a; See Scheme 10) represents the GMP process starting material. 5-(3-chloro-5-(trifluoromethoxy)phenyl)-N-methoxy-N-methyl-1-(pyridin-3-yl)-1H-pyrazole-3-carboxamide (i.e. compound 8a; see Scheme 10) represents another common intermediate of the general synthesis process for obtaining 4-[5-[3-Chloro-5-(trifluoromethoxy) phenyl]-1-(pyridin-3-yl)-1H-pyrazol-3-yl]-5,6- dihydro-7H-pyrrolo[3,4-d] pyrimidin-7-one (i.e. compound A) in accordance with the invention.

Step 1) Starting from the commercially available raw material 3-chloro-5-(trifluoromethoxy)acetophenone (i.e. compound 1 a, see Scheme 10), the chemical transformation towards 5-(3-chloro-5-(trifluoromethoxy)phenyl)-1-(pyridin-3-yl)-1H-pyrazole-3-carboxylic acid (i.e. compound 6a; See Scheme 10) comprises 4 chemical steps, performed as two distinct process steps.

Step 1a) The first step describes the production and isolation of compound 2a (see Scheme 11) out of compound 1 a, the second process step is designed to produce compound 6a as free acid (see 12).

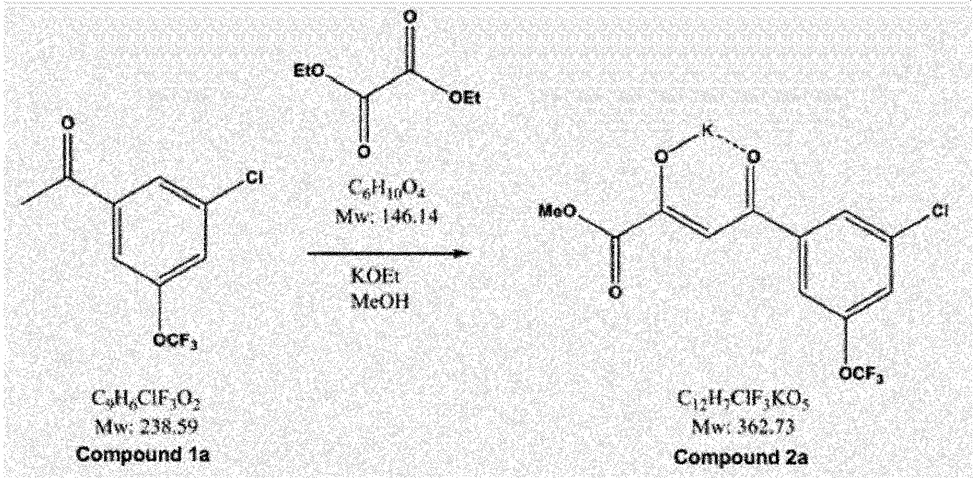

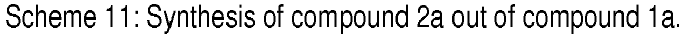

The product compound 2a is isolated in solid form as its potassium salt, and used without further purification in the next process steps (see below). For the heredescribed general synthesis in kg-amounts, the co-solvent methanol is used. The presence of methanol improves both the mobility of the obtained suspension, and improves the filtration speed during the isolation of the desired product.

Further details for the above described first general process step can be derived from the example portion; see Example 1.

Step 2) Step 2 describes the synthesis of compound 6a starting from the intermediate compound 2a (see Scheme 12 below). The reaction as depicted below in Scheme 12 is a two-step single pot reaction. The first step is a condensation reaction of compound 2a with the second key starting material compound 3a. The hydrazone intermediate compound 4a is formed at ambient temperature, but the formation of compound 5a out of compound 4a occurs at elevated temperature. The reaction mixture needs to be heated by slow rate heating. Compound 2a reacts rapidly with compound 3a, resulting in a condensation product, which is not purged in the subsequent reaction steps. The resulting pyridyl-aryl-pyrazole ester compound 5a is a mixture of the methyl and ethyl ester which is not isolated, but converted to the corresponding carboxylic acid compound 6a using an aqueous sodium hydroxide solution.

After adjusting the pH, the product compound 6a precipitates out of solution and can be isolated in solid form. The addition of hydrochloric acid solution needs to be monitored on pH, as reaction mixture needs to be of pH ≥ 2. The obtained dry compound 6a can be used without further purification in the next process step.

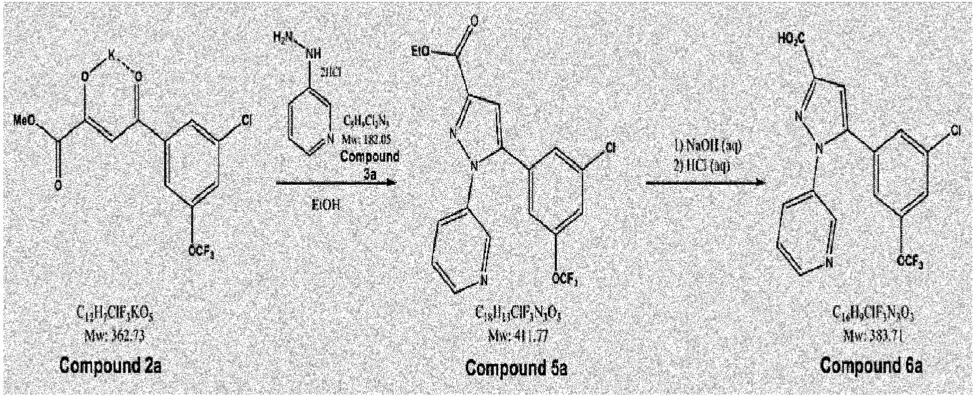

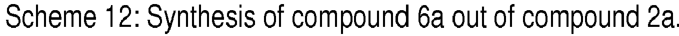

Step 2a - alternative) Optionally, compound 6a could also be isolated as either oxalate or hydrochloric acid adduct. The oxalate adduct can be prevented by isolating intermediate compound 2a in solid form by filtration. This filtration step purges all remaining diethyl oxalate, which is the source of the oxalate anion. By controlling the pH during production of compound 6a a hydrochloric acid adduct formation can be prevented.

Step 3) This process step describes the synthesis of compound 8a starting from the GMP starting material compound 6a (see Scheme 13 below). The intermediate acid chloride compound 7a is not isolated, but the process is performed as a two-step one pot reaction. The product is isolated in solid form, and used without further purification in the next process step (see below). This reaction is conducted with oxalyl chloride at ambient temperature. Preferably, slow dosing of said reagent oxalyl chloride is performed. The N,O-dimethylhydroxyl amine is added as its HCl salt. No reaction should occur, until this reagent is liberated by dosing of the base. The preferred base with the context of the present invention is diisopropylethylamine (DiPEA).

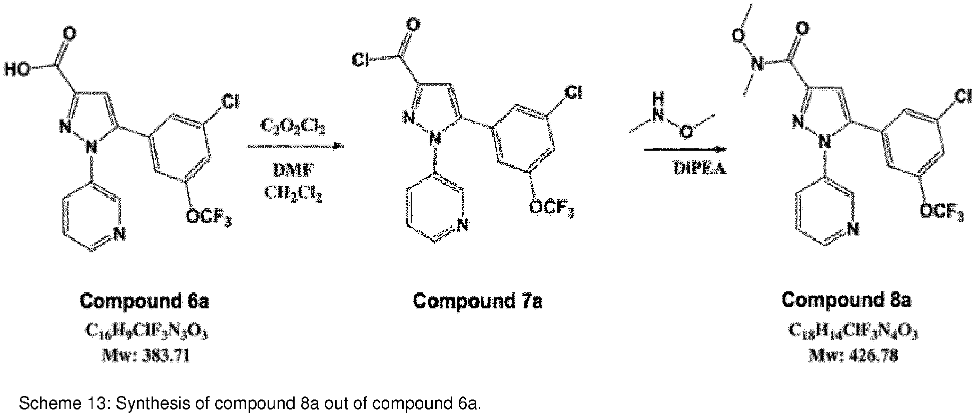

Step 4) This process step 4 describes the synthesis of compound 9a starting from the Weinreb amide compound 8a (see Scheme 14 below). Compound 9a is isolated as a solution in THF. This solution can be used in the next step of the synthesis without further purification. The reagent methylmagnesiumchloride can be commercially obtained as a tetrahydrofuran solution (22 %-wt active material). This solution can be transferred directly from the pressurized container into the reactor.

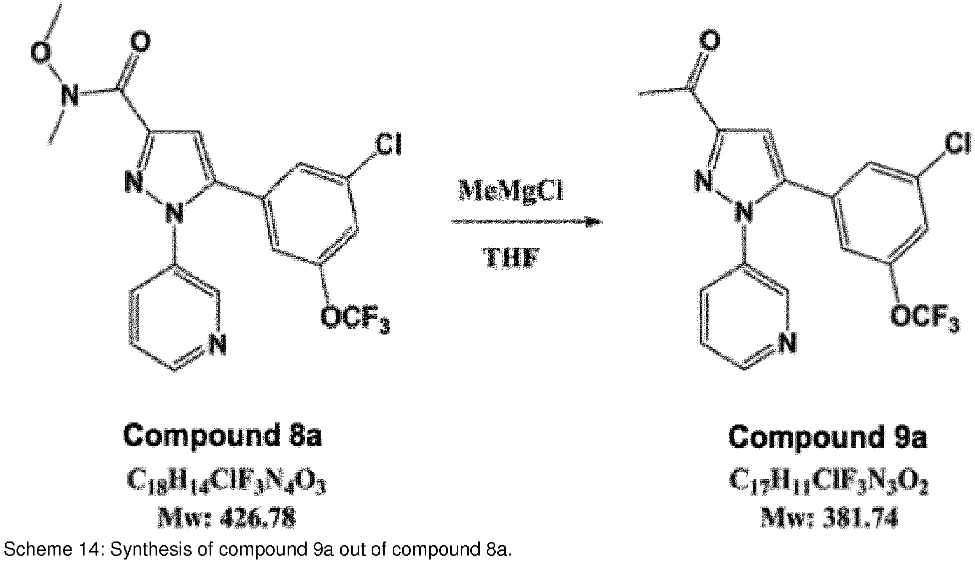

Step 5) Process step 5 is characterized by the synthesis of compound 10a starting from the ketone derivative compound 9a, which is harvested as a tetrahydrofuran solution (see Scheme 15 below). For the GMP synthesis of compound 10a a sodium ethoxide as 21 %-wt solution in ethanol is used.

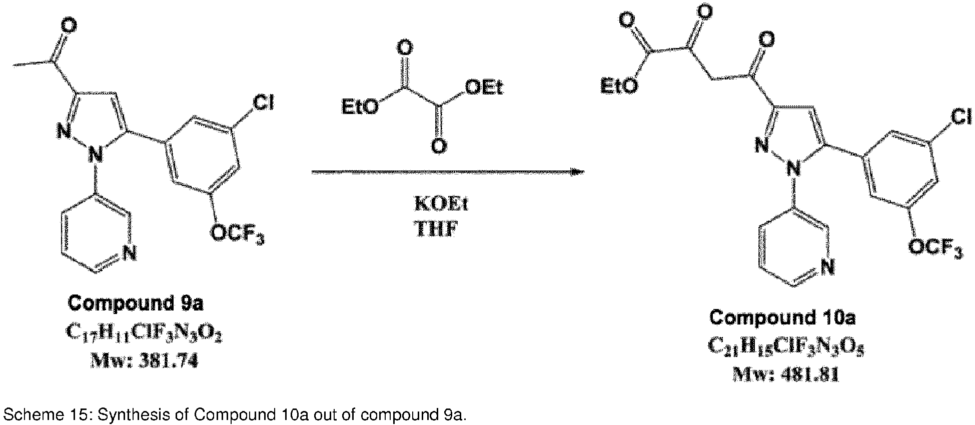

Step 6) The synthesis step 6 describes the synthesis of compound 12a starting from the intermediate compound 10a (see Scheme 16 below). The chemistry is a two-step process in which first a keto-imine (i.e. compound 11 a) is formed, which is not isolated, followed by a Mannich reaction yielding the compound 12a. In both steps the solvent acetic acid is used. The ammonia donor is in both cases ammonium acetate. The paraformaldehyde is dosed as a solid to the reaction mixture, in order to minimize the required amounts of solvents. The first step is performed at approx. 45 ± 5 °C, while the second step is performed at approx. 35 ± 5°C.

Dissolution of the process intermediate compound 11 a and the intermediate product compound 10a occurs at temperatures above approx. 30 - 35 °C. The product compound 12a precipitates out of solution by addition of ethanol to the reaction mixture.

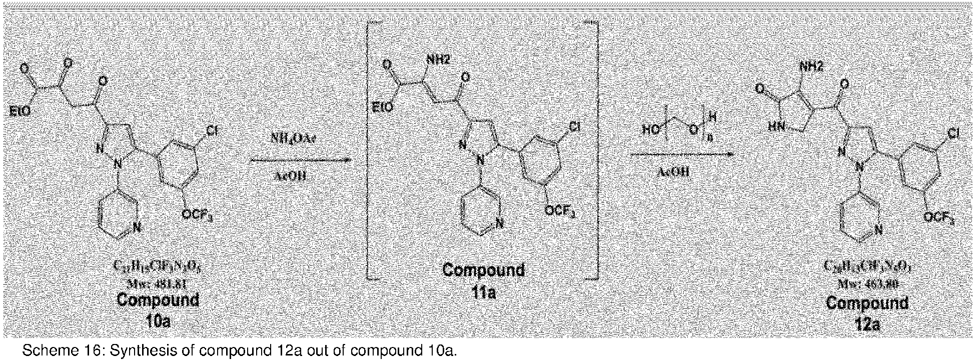

Step 7) Step 7 describes the synthesis of compound A as crude product, starting from the intermediate compound 12a (see Scheme 17 below). In order to prevent large amounts of salts in the condenser the reaction mixture is heated and kept under pressure. A positive side effect of performing the reaction under pressure is the fact that the crude isolated compound A is of higher chromatographic purity.

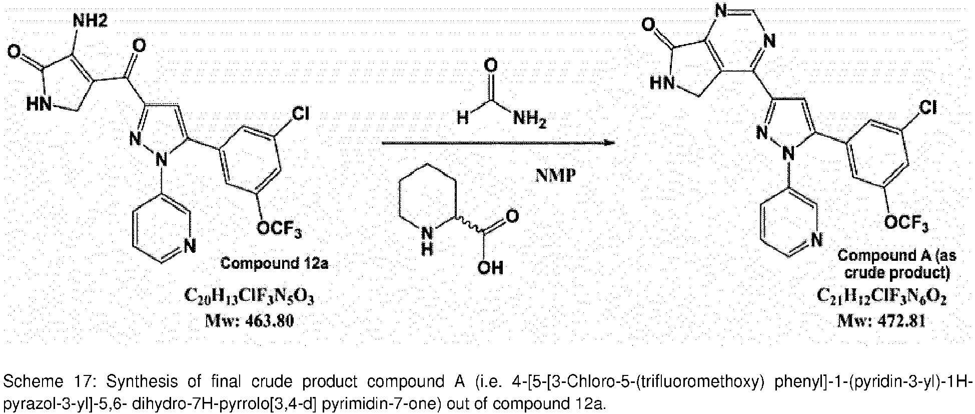

Step 7a) Purification Isolation and release testing of compound A as crude product:

The finalization of the synthesis process is characterized by two distinct purification steps.

The first step describes the crystallization of the crude product compound A out of an acetic acid solution, using water as anti-solvent. This step is intended to remove organic impurities formed during the last steps of production, and reduce the amounts of compound 12a, if still present as impurity in the final product. Compound A can be also obtained as acetate adduct. The acetate adduct can be identified by its distinct XRPD pattern. Dosing water, however, yields compound A as a free base with the correct polymorphic form.

The second step consists of dissolution of the purified compound A in a mixture of dichloromethane and methanol in order to perform a screening filtration designed to remove any foreign particles, if present. The product can be isolated by filtration after a solvent switch to methanol.

Final release testing may also be performed.

In contrast to the previous described synthesis phases I and II, the above described alternative route does not comprise any chromatographic purification steps of 4-[5-[3-Chloro-5-(trifluoromethoxy) phenyl]-1-(pyridin-3-yl)-1 H-pyrazol-3-yl]-5,6-dihydro-7H-pyrrolo[3,4-d] pyrimidin-7-one (compound A).

Further details on the above described processes for the synthesis of compound A can be derived from Example 1.

For the person skilled in the art, by the above described synthesis processes, it is immediately apparent how to modify this processes to synthesize the closely related, methyl containing analouge compound C of the invention; i.e. 4-[5-[3-Chloro-5-(trifluoromethoxy) phenyl]-1-(pyridin-3-yl)-1H-pyrazol-3-yl]-5,6-dihydro-2-methyl-7H-pyrrolo[3, 4-d]pyrimidin-7-one.

With the above context, in another aspect of the invention, the inventors surprisingly found new synthesis processes for the compound A, and likewise for compound C of the invention, characterized by:
- a larger production capability of kg-amounts of compounds A or C, respectively;
- less chemical reaction steps in a linear way (11 steps only);
- higher isolated total yield;
- higher purity of final isolated material (i.e. compound A or likewise compound C);
- use of less hazardous agents that are also more cost-effective; and
- the avoidance of several chromatographic purification steps.

The compounds of the general formula (I) for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD in accordance with the present invention can act systemically and/or locally. For this purpose, they can be administered in a suitable way, such as, e.g., orally, parenterally, pulmonarily, nasally, sublingually, lingually, bucally, rectally, dermally, transdermally, conjunctivally, otically or as an implant or stent.

In accordance with the present invention the oral and parenteral administration routes for the compounds of general formula (I) in the methods of treatment and/or prevention of the aforementioned diseases are preferred.

For these application routes, the compounds according to the invention can be administered in suitable forms of application.

For oral application, forms of application that release the compounds according to the invention in a quick-acting and/or modified way according to the state of the art and that contain the compounds according to the invention in crystalline and/or amorphized and/or dissolved form and/or in the form of co-crystals or polymorphs of said co-crystals, are selected from a group comprising tablets (uncoated or coated tablets, for example with gastric juice-resistant or slow-dissolving or insoluble coatings, which control the release of the compound according to the invention), tablets or films/wafers that quickly dissolve in the oral cavity, films/lyophilisates, multilayered tablets, capsules (for example, hard- or soft-gelatin capsules), granulates, pellets, powder, emulsions, microemulsions, nanoemulsions, suspensions, nanosuspensions, aerosols or solutions, are suitable.

Parenteral administration can be realized by bypassing a resorption step (e.g., by intravenous, intraarterial, intracardial, intraspinal or intralumbar means) or by including resorption (e.g., by intramuscular, subcutaneous, intracutaneous, percutaneous or intraperitoneal means). For parenteral administration, *inter alia,* injection and infusion preparations in the form of solutions, suspensions, nanosuspensions, emulsions, microemulsions, nanoemulsions, lyophilisates, liposomes, polymeric micelles or sterile powders are suitable as forms of application in accordance with the instant invention.

For the other administration routes, as e.g., inhalation forms of medication *inter alia* powder inhalers, nebulizers, nose drops, nasal solutions, nasal sprays; e.g. for oral administration tablets that are to be administered lingually, sublingually or buccally; films/wafers or capsules, suppositories, aqueous suspensions (lotions, shaking mixtures), aqueous nanosuspensions, lipophilic suspensions; ear or eye preparations, or e.g. for topical application forms of medication ointments, creams, transdermal therapeutic systems (such as, for example, patches), milk, pastes, foams, scattered powders; implants or stents, are suitable in accordance with the invention.

The compounds of the general formula (I) according to the invention can be converted into the above-recited forms of application. This can take place in a way that is known in the art by mixing with inert, nontoxic, pharmaceutically suitable adjuvants. These adjuvants include, *inter alia,* vehicles (for example, microcrystalline cellulose, lactose, mannitol), solvents (e.g., liquid polyethylene glycols), emulsifiers and dispersing agents or wetting agents (for example, sodium dodecyl sulfate, polyoxysorbitanoleate), binders (for example, polyvinylpyrrolidone), synthetic and natural polymers (for example, albumin), stabilizers (e.g., antioxidants, such as, for example, ascorbic acid), dyes (e.g., inorganic pigments, such as, for example, iron oxides), and flavoring and/or odor correctives.

In general, it has been shown to be advantageous in both human and veterinary medicine to administer the compounds of general formula (I) as active pharmaceutical ingredient (API) in a method of treating and/or preventing cardiovascular diseases and the aforementioned particular diseases of the invention in total quantities of 0.0005 to 50 mg/kg, preferably of 0.005 to 40 mg/kg, more preferably from 0.05 to 30 mg/kg, even more preferably of 0.1 to 20 mg/kg, even more preferably of 1 to 10 mg/kg of body weight per 24-hour period, potentially in the form of multiple single doses.

A single dose preferably contains a compound of general formula (I) as API in quantities from 0.005 to 50 mg/kg, more preferably from 0.005 to 40 mg/kg, even more preferably from 0.05 to 30 mg/kg, even more preferably from 0.05 to 20 mg/kg, even more preferably from 0.05 to 10 mg/kg, most preferred of 0.05 to 5 mg/kg of body weight.

Nonetheless, the person skilled in the art is aware that sometimes it can be necessary to deviate from said quantities, namely depending on body weight, administration route, individual response to the active substance, nature of the preparation and time or interval at which the administration takes place. For example, in certain cases it may be sufficient to get by with less than the aforementioned minimum amount, while in other cases the stated upper limit has to be exceeded. When administering large amounts it may be recommendable to distribute these in several individual doses over the course of a day.

In another aspect, subject matter of the invention are pharmaceutical agents that contain at least one compound of the general formula (I) according to the invention, usually together with one or more inert, non-toxic, pharmaceutically suitable adjuvants, as well as their use for the above-mentioned purposes.

In addition, in another aspect, the present invention is directed to pharmaceutical combination compositions comprising:
- a therapeutically effective amount of a composition comprising a first compound, said first compound being a compound of the general formula (I) or a pharmaceutically acceptable salt, or a solvate or a solvate of the salt of said compound or a pharmaceutically acceptable co-crystal of said compound or a polymorph of said co-crystal;
- and at least a second compound, said second compound being selected from a group comprising pharmaceutically acceptable antithrombotic agent, blood pressure lowering agent, lipid metabolism agent, calcium antagonist / calcium channel blockers, alpha 1 receptor blocker, beta-receptor blocker, angiotensin A-II antagonist, ACE inhibitor, endothelin antagonist, cholesterol absorption inhibitor, polymeric bile;
- and a pharmaceutically acceptable carrier, vehicle or diluent.

Thus, with the above context, another object of the present invention are pharmaceutical compositions comprising at least one compound of the general formula (I) or a pharmaceutically acceptable salt or solvate or a solvate of the salt of said compound or a pharmaceutically acceptable co-crystal of said compound or a polymorph of said co-crystal, and one or more other active ingredients, in particular for its use in methods of treatment and/or prevention of the aforementioned diseases. Specific examples are preferably suitable combination active ingredients comprising:
- antithrombotic agents, for example and preferably selected from the group comprising platelet aggregation inhibitors, of anticoagulants or of per fibrino lyrical substances; and/or
- blood pressure lowering agents, as for example, but not being limited thereto, selected from the group comprising calcium antagonists, angiotensin antagonists, ACE inhibitors, endothelin antagonists, renin inhibitors, alpha-receptor blockers, beta-receptor blockers, mineralocorticoid receptor antagonists diuretics; and/or
- to alter lipid metabolism agents, for example and preferably selected from a group comprising thyroid receptor agonists, cholesterol synthesis inhibitors such as for example and preferably HMG-CoA reductase or squalene synthesis inhibitors, of ACAT inhibitors, CETP inhibitors, MTP inhibitors, PPAR -alpha, PPAR gamma and/or PPAR-delta agonists, cholesterol absorption inhibitors, lipase inhibitors, polymeric bile acid reabsorption and lipoprotein antagonist.

Agents having antithrombotic activity preferably mean compounds selected from the group of platelet aggregation inhibitors, anticoagulants or fibrinolytic agents.

Thus, in another aspect of the invention the compounds of the general formula (I) or a pharmaceutically acceptable salt or solvate or a solvate of the salt of said compound or a pharmaceutically acceptable co-crystal of said compound or a polymorph of said co-crystal are to be administered in a method of treatment and/or prevention of the aforementioned diseases/conditions in combination with a platelet aggregation inhibitor, being selected for example and preferably from a group comprising aspirin, clopidogrel, ticlopidine or dipyridamole.

Among the antihypertensive agents used in combination with the compounds of the general formula (I) or a pharmaceutically acceptable salt or solvate or a solvate of the salt of said compound or a pharmaceutically acceptable co-crystal of said compound or a polymorph of said co-crystal in a method of treatment and/or prevention of the aforementioned diseases/conditions are preferably compounds selected from the group comprising calcium antagonists, angiotensin A-II antagonists, ACE inhibitors, endothelin antagonists, renin inhibitors, alpha-receptor blockers, beta-receptor blockers, mineralocorticoid receptor antagonists, and of diuretics.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with a calcium antagonist such as by way of example and preferably selected from a group comprising nifedipine, amlodipine, verapamil or diltiazem.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with an alpha 1 receptor blocker such as by way of example and preferably prazosin.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with a beta-receptor blocker, such as for example and not being limited to, propranolol, atenolol, timolol, pindolol, alprenolol, oxprenolol, penbutolol, bupranolol, metipranolol, nadolol, mepindolol, Carazalol, sotalol, metoprolol, betaxolol, celiprolol, bisoprolol, carteolol, esmolol, labetalol, carvedilol, adaprolol, Landiolol, nebivolol, Epanolol or bucindolol.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with an angiotensin A-II antagonist such as, for example and preferably selected from a group comprising losartan, candesartan, valsartan, telmisartan or embursatan.
In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with an ACE inhibitor such as by way of example and preferably enalapril, captopril, lisinopril, ramipril, delapril, fosinopril, quinopril, perindopril or trandopril.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with an endothelin antagonist, such as for example and preferably selected from a group comprising bosentan, darusentan, ambrisentan or sitaxsentan.

With the context of the invention, lipid metabolism-modifying agents are preferably compounds selected from the group comprising CETP inhibitors, thyroid receptor agonists, cholesterol synthesis inhibitors such as HMG-CoA reductase or squalene synthesis inhibitors, ACAT inhibitors, MTP inhibitors, PPAR-alpha, PPAR-gamma and/or PPAR-delta agonists, cholesterol absorption inhibitors, polymeric bile acid reabsorption inhibitors, lipase inhibitors and lipoprotein antagonists.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with a cholesterol absorption inhibitor such as, for example and preferably selected from a group comprising ezetimibe, tiqueside or pamaqueside.

In yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with a lipase inhibitor such as, for example and preferably orlistat.

With the above context, in yet another aspect of the invention, the compounds of the general formula (I) or a pharmaceutically acceptable salt or solvate or a solvate of the salt of said compound or a pharmaceutically acceptable co-crystal of said compound or a polymorph of said co-crystal for use in a method of treatment and/or prevention of the aforementioned diseases/conditions are to be combined with a polymeric bile as for example and preferably selected from a group comprising cholestyramine, colestipol, colesolvam, CholestaGel or colestimid.

In addition, the present invention is directed to pharmaceutical combination compositions comprising: a therapeutically effective amount of a composition comprising
- a first compound, said first compound being a compound of formula (I) or a pharmaceutically acceptable salt or solvate or a solvate of the salt of said compound or a pharmaceutically acceptable co-crystal of said compound or a polymorph of said co-crystal;
- a second compound, said second compound being a lipid modulating agent; and
- a pharmaceutically acceptable carrier, vehicle or diluent.

Examples of lipid modulating agents include a lipase inhibitor, an HMG-CoA reductase inhibitor, an HMG-CoA synthase inhibitor, an HMG-CoA reductase gene expression inhibitor, an HMG-CoA synthase gene expression inhibitor, an MTP/Apo B secretion inhibitor, a CETP inhibitor, a bile acid absorption inhibitor, a cholesterol absorption inhibitor, a cholesterol synthesis inhibitor, a squalene synthetase inhibitor, a squalene epoxidase inhibitor, a squalene cyclase inhibitor, a combined squalene epoxidase/squalene cyclase inhibitor, a fibrate, niacin, a combination of niacin and lovastatin, an ion-exchange resin, an antioxidant, an ACAT inhibitor and a bile acid sequestrant.

With the above context, the compounds of the invention may be used in the herein dislcosed methods for treatment and/or prophylaxis of the aforementioned diseases alone or in combination, if necessary, with other agents.

Said other agents may be preferably selected from the classes of statins, prile, beta blocker, acetylsalicylic acid, sartanes, ACE-inhibitor, AT-1 receptor antagonists, diuretics, calcium-channel antagonists/calcium-channel blocker.

The compounds of the general formula (I) or a pharmaceutically acceptable salt or solvate or a solvate of the salt of said compound or a pharmaceutically acceptable co-crystal of said compound or a polymorph of said co-crystal may also be used in conjunction with other pharmaceutical agents (e.g., LDL-cholesterol lowering agents, triglyceride lowering agents) in the methods of treatment and/or prevention as described herein. For example, they may be used in combination with lipid modulating agents, antidiabetic agents and further cardiovascular agents.

Lipid modulating agents may be used as a combination agent in conjunction with compounds of the general formula (I) or a pharmaceutically acceptable salt or solvate or a solvate of the salt of said compound or a pharmaceutically acceptable co-crystal of said compound or a polymorph of said co-crystal. Any HMG-CoA reductase inhibitor may be used in the combination aspect of this invention. The conversion of 3-hydroxy-3-methylglutaryl-coenzyme A (HMG-CoA) to mevalonate is an early and rate-limiting step in the cholesterol biosynthetic pathway. This step is catalyzed by the enzyme HMG-CoA reductase. Statins inhibit HMG-CoA reductase from catalyzing this conversion. The following paragraphs describe exemplary stati ns.

The term HMG-CoA reductase inhibitor refers to compounds, which inhibit the bioconversion of hydroxymethylglutaryl-coenzyme A to mevalonic acid catalyzed by the enzyme HMG-CoA reductase. Such inhibition is readily determined by those skilled in the art according to standard assays (e.g., Meth. Enzymol. 1981; 71:455-509 and references cited therein). A variety of these compounds are described and referenced below, however, other HMG-CoA reductase inhibitors will be known to those skilled in the art. U.S. Pat. No. 4,231,938 describes certain compounds isolated after cultivation of a microorganism belonging to the genus Aspergillus, such as lovastatin. Also, U.S. Pat. No. 4,444,784 describes synthetic derivatives of the aforementioned compounds, such as simvastatin. Also, U.S. Pat. No. 4,739,073 describes certain substituted indoles, such as fluvastatin. Also, U.S. Pat. No. 4,346,227 describes ML-236B derivatives, such as pravastatin. Also, EP-491226A describes certain pyridyldihydroxyheptenoic acids, such as cerivastatin. In addition, U.S. Pat. No. 5,273,995 describes certain 6-[2-(substituted-pyrrol-1-yl)alkyl]pyran-2-ones such as atorvastatin and any pharmaceutically acceptable form thereof (i.e. Lipitor^{®}). Additional HMG-CoA reductase inhibitors include rosuvastatin and pitavastatin.

Atorvastatin calcium (i.e., atorvastatin hemicalcium), described in U.S. Pat. No. 5,273,995 is currently sold under the trademark Lipitor^{®}.

Statins also include such compounds as rosuvastatin described in U.S. RE37,314 E, pitivastatin described in EP 304063 B1 and U.S. Pat. No. 5,011,930, simvastatin, described in U.S. Pat. No. 4,444,784; pravastatin, described in U.S. Pat. No. 4,346,227; cerivastatin, described in U.S. Pat. No. 5,502,199; mevastatin, described in U.S. Pat. No. 3,983,140; velostatin, described in U.S. Pat. No. 4,448,784 and U.S. Pat. No. 4,450,171; fluvastatin, described in U.S. Pat. No. 4,739,073; compactin, described in U.S. Pat. No. 4,804,770; lovastatin, described in U.S. Pat. No. 4,231,938; dalvastatin, described in European Patent Application Publication No. 738510 A2; fluindostatin, described in European Patent Application Publication No. 363934 A1; and dihydrocompactin, described in U.S. Pat. No. 4,450,171.

Given the association between diabetes and atherosclerosis (e.g., in case of metabolic syndrome), in another aspect of the invention, the compounds of the general formula (I) or a pharmaceutically acceptable salt or solvate or a solvate of the salt of said compound or a pharmaceutically acceptable co-crystal of said compound or a polymorph of said co-crystal may be administered with antidiabetic compounds in methods of treating and/or preventing the comorbidity diabetes.

Diabetes can be treated by administering to a patient having diabetes (especially Type II), insulin resistance, impaired glucose tolerance, metabolic syndrome, or the like, or any of the diabetic complications such as neuropathy, nephropathy, retinopathy or cataracts, a therapeutically effective amount of a compound of the present invention in combination with other agents (e.g., insulin) that can be used to treat diabetes. This includes the classes of anti-diabetic agents (and specific agents) described herein.

With the above context, in another aspect of the invention, any glycogen phosphorylase inhibitor can be used as the second agent in combination with a compound of the present invention or a pharmaceutically acceptable salt or solvate or a solvate of the salt of said compound or a pharmaceutically acceptable co-crystal of said compound or a polymorph of said co-crystal. The term glycogen phosphorylase inhibitor refers to compounds that inhibit the bioconversion of glycogen to glucose-1-phosphate, which is catalyzed by the enzyme glycogen phosphorylase. Such glycogen phosphorylase inhibition activity is readily determined by those skilled in the art according to standard assays (e.g., J. Med. Chem. 41 (1998) 2934-2938). A variety of glycogen phosphorylase inhibitors are known to those skilled in the art including those described in WO 96/39384 and WO 96/39385.

With the above context, in another aspect of the invention, any aldose reductase inhibitor can be used in combination with a compound of the present invention. The term aldose reductase inhibitor refers to compounds that inhibit the bioconversion of glucose to sorbitol, which is catalyzed by the enzyme aldose reductase. Aldose reductase inhibition is readily determined by those skilled in the art according to standard assays (e.g., J. Malone, Diabetes, 29:861-864 (1980). "Red Cell Sorbitol, an Indicator of Diabetic Control"). A variety of aldose reductase inhibitors are known to those skilled in the art, such as those described in U.S. Pat. No. 6,579,879, which includes 6-(5-chloro-3-methyl-benzofuran-2-sulfonyl)-2H-pyridazin-3-one.

With the above context, in another aspect of the invention, any sorbitol dehydrogenase inhibitor can be used in combination with a compound of the present invention or a pharmaceutically acceptable salt or solvate or a solvate of the salt of said compound or a pharmaceutically acceptable co-crystal of said compound or a polymorph of said co-crystal. The term sorbitol dehydrogenase inhibitor refers to compounds that inhibit the bioconversion of sorbitol to fructose, which is catalyzed by the enzyme sorbitol dehydrogenase. Such sorbitol dehydrogenase inhibitor activity is readily determined by those skilled in the art according to standard assays (e.g., Analyt. Biochem (2000) 280: 329-331). A variety of sorbitol dehydrogenase inhibitors are known, for example, U.S. Pat. Nos. 5,728,704 and 5,866,578 describe compounds and a method for treating or preventing diabetic complications by inhibiting the enzyme sorbitol dehydrogenase.

With the above context, calcium channel blockers which are within the scope of the present invention include, but are not limited to: bepridil, which may be prepared as described in U.S. Pat. No. 3,962, 238 or U.S. Reissue No. 30,577; clentiazem, which may be prepared as described in U.S. Pat. No. 4,567,175; diltiazem, which may be prepared as described in U.S. Pat. No. 3,562, fendiline, which may be prepared as described in U.S. Pat. No. 3,262,977; gallopamil, which may be prepared as described in U.S. Pat. No. 3,261,859; mibefradil, which may be prepared as described in U.S. Pat. No. 4,808,605; prenylamine, which may be prepared as described in U.S. Pat. No. 3,152,173; semotiadil, which may be prepared as described in U.S. Pat. No. 4,786,635; terodiline, which may be prepared as described in U.S. Pat. No. 3,371,014; verapamil, which may be prepared as described in U.S. Pat. No. 3,261,859; aranipine, which may be prepared as described in U.S. Pat. No. 4,572,909; barnidipine, which may be prepared as described in U.S. Pat. No. 4,220,649; benidipine, which may be prepared as described in European Patent Application Publication No. 106,275; cilnidipine, which may be prepared as described in U.S. Pat. No. 4,672,068; efonidipine, which may be prepared as described in U.S. Pat. No. 4,885,284; elgodipine, which may be prepared as described in U.S. Pat. No. 4,952,592; felodipine, which may be prepared as described in U.S. Pat. No. 4,264,611; isradipine, which may be prepared as described in U.S. Pat. No. 4,466,972; lacidipine, which may be prepared as described in U.S. Pat. No. 4,801,599; lercanidipine, which may be prepared as described in U.S. Pat. No. 4,705,797; manidipine, which may be prepared as described in U.S. Pat. No. 4,892,875; nicardipine, which may be prepared as described in U.S. Pat. No. 3,985,758; nifedipine, which may be prepared as described in U.S. Pat. No. 3,485,847; nilvadipine, which may be prepared as described in U.S. Pat. No. 4,338,322; nimodipine, which may be prepared as described in U.S. Pat. No. 3,799,934; nisoldipine, which may be prepared as described in U.S. Pat. No. 4,154,839; nitrendipine, which may be prepared as described in U.S. Pat. No. 3,799,934; cinnarizine, which may be prepared as described in U.S. Pat. No. 2,882,271; flunarizine, which may be prepared as described in U.S. Pat. No. 3,773,939; lidoflazine, which may be prepared as described in U.S. Pat. No. 3,267,104; lomerizine, which may be prepared as described in U.S. Pat. No. 4,663,325; bencyclane, which may be prepared as described in Hungarian Patent No. 151,865; etafenone, which may be prepared as described in German Patent No. 1,265,758; and perhexiline, which may be prepared as described in British Patent No. 1,025,578. Examples of presently marketed products containing antihypertensive agents include calcium channel blockers, such as Cardizem^{®}, Adalat^{®}, Calan^{®}, Cardene^{®}, Covera^{®}, Dilacor^{®}, DynaCirc^{®}, Procardia XL^{®}, Sular^{®}, Tiazac^{®}, Vascor^{®}, Verelan^{®}, Isoptin^{®}, Nimotop^{®}, Norvasc^{®}, and Plendil^{®}; angiotensin converting enzyme (ACE) inhibitors, such as Accupril^{®}, Altace^{®}, Captopril^{®}, Lotensin^{®}, Mavik^{®}, Monopril^{®}, Prinivil^{®}, Univasc^{®}, Vasotec^{®} and Zestril^{®}.

With the above context, angiotensin converting enzyme inhibitors (ACE-Inhibitors) which are within the scope of the present invention include, but are not limited to: alacepril, which may be prepared as described in U.S. Pat. No. 4,248,883; benazepril, which may be prepared as described in U.S. Pat. No. 4,410,520; captopril, which may be prepared as described in U.S. Pat. Nos. 4,046,889 and 4,105,776; ceronapril, which may be prepared as described in U.S. Pat. No. 4,452,790; delapril, which may be prepared as described in U.S. Pat. No. 4,385,051; enalapril, which may be prepared as described in U.S. Pat. No. 4,374,829; fosinopril, which may be prepared as described in U.S. Pat. No. 4,337,201; imadapril, which may be prepared as described in U.S. Pat. No. 4,508,727; lisinopril, which may be prepared as described in U.S. Pat. No. 4,555,502; moveltopril, which may be prepared as described in Belgian Patent No. 893,553; perindopril, which may be prepared as described in U.S. Pat. No. 4,508,729; quinapril, which may be prepared as described in U.S. Pat. No. 4,344,949; ramipril, which may be prepared as described in U.S. Pat. No. 4,587,258; spirapril, which may be prepared as described in U.S. Pat. No. 4,470,972; temocapril, which may be prepared as described in U.S. Pat. No. 4,699,905; and trandolapril, which may be prepared as described in U.S. Pat. No. 4,933,361.

With the above context, angiotensin-II receptor antagonists (A-II antagonists) which are within the scope of the present invention include, but are not limited to: candesartan, which may be prepared as described in U.S. Pat. No. 5,196,444; eprosartan, which may be prepared as described in U.S. Pat. No. 5,185,351; irbesartan, which may be prepared as described in U.S. Pat. No. 5,270,317; losartan, which may be prepared as described in U.S. Pat. No. 5,138,069; and valsartan, which may be prepared as described in U.S. Pat. No. 5,399,578.

With the above context, beta-adrenergic receptor blockers (beta- or β-blockers) which are within the scope of the present invention include, but are not limited to: acebutolol, which may be prepared as described in U.S. Pat. No. 3,857,952; alprenolol, which may be prepared as described in Netherlands Patent Application No. 6,605,692; amosulalol, which may be prepared as described in U.S. Pat. No. 4,217,305; arotinolol, which may be prepared as described in U.S. Pat. No. 3,932,400; atenolol, which may be prepared as described in U.S. Pat. No. 3,663,607 or 3,836,671; befunolol, which may be prepared as described in U.S. Pat. No. 3,853,923; betaxolol, which may be prepared as described in U.S. Pat. No. 4,252,984; bevantolol, which may be prepared as described in U.S. Pat. No. 3,857,981; bisoprolol, which may be prepared as described in U.S. Pat. No. 4,171,370; bopindolol, which may be prepared as described in U.S. Pat. No. 4,340,541; bucumolol, which may be prepared as described in U.S. Pat. No. 3,663,570; bufetolol, which may be prepared as described in U.S. Pat. No. 3,723,476; bufuralol, which may be prepared as described in U.S. Pat. No. 3,929,836; bunitrolol, which may be prepared as described in U.S. Pat. Nos. 3,940,489 and 3,961,071; buprandolol, which may be prepared as described in U.S. Pat. No. 3,309,406; butiridine hydrochloride, which may be prepared as described in French Patent No. 1,390,056; butofilolol, which may be prepared as described in U.S. Pat. No. 4,252,825; carazolol, which may be prepared as described in German Patent No. 2,240,599; carteolol, which may be prepared as described in U.S. Pat. No. 3,910,924; carvedilol, which may be prepared as described in U.S. Pat. No. 4,503,067; celiprolol, which may be prepared as described in U.S. Pat. No. 4,034,009; cetamolol, which may be prepared as described in U.S. Pat. No. 4,059,622; cloranolol, which may be prepared as described in German Patent No. 2,213,044; dilevalol, which may be prepared as described in Clifton et al., Journal of Medicinal Chemistry, 1982, 25, 670; epanolol, which may be prepared as described in European Patent Publication Application No. 41,491; indenolol, which may be prepared as described in U.S. Pat. No. 4,045,482; labetalol, which may be prepared as described in U.S. Pat. No. 4,012,444; levobunolol, which may be prepared as described in U.S. Pat. No. 4,463,176; mepindolol, which may be prepared as described in Seeman et al., Helv. Chim. Acta, 1971, 54, 241; metipranolol, which may be prepared as described in Czechoslovakian Patent Application No. 128,471; metoprolol, which may be prepared as described in U.S. Pat. No. 3,873,600; moprolol, which may be prepared as described in U.S. Pat. No. 3,501,7691; nadolol, which may be prepared as described in U.S. Pat. No. 3,935,267; nadoxolol, which may be prepared as described in U.S. Pat. No. 3,819,702; nebivalol, which may be prepared as described in U.S. Pat. No. 4,654,362; nipradilol, which may be prepared as described in U.S. Pat. No. 4,394,382; oxprenolol, which may be prepared as described in British Patent No. 1,077,603; perbutolol, which may be prepared as described in U.S. Pat. No. 3,551,493; pindolol, which may be prepared as described in Swiss Patent Nos. 469,002 and 472,404; practolol, which may be prepared as described in U.S. Pat. No. 3,408,387; pronethalol, which may be prepared as described in British Patent No. 909,357; propranolol, which may be prepared as described in U.S. Pat. Nos. 3,337,628 and 3,520,919; sotalol, which may be prepared as described in Uloth et al., Journal of Medicinal Chemistry, 1966, 9, 88; sufinalol, which may be prepared as described in German Patent No. 2,728,641; talindol, which may be prepared as described in U.S. Pat. Nos. 3,935,259 and 4,038,313; tertatolol, which may be prepared as described in U.S. Pat. No. 3,960,891; tilisolol, which may be prepared as described in U.S. Pat. No. 4,129,565; timolol, which may be prepared as described in U.S. Pat. No. 3,655,663; toliprolol, which may be prepared as described in U.S. Pat. No. 3,432,545; and xibenolol, which may be prepared as described in U.S. Pat. No. 4,018,824.

In yet another aspect, the present invention is further directed to the use of the compounds of the general formula (I) or a pharmaceutically acceptable salt or solvate or a solvate of the salt of said compound or a pharmaceutically acceptable co-crystal of said compound or a polymorph of said co-crystal as a medicament (such as a unit dosage tablet or unit dosage capsule, or a dual, tripple or polypill with at least one of the additional pharmaceutical agents as recited herein) in a method of treatment and/or prevention of one or more of the conditions previously identified in the above sections discussing its use in methods of treatment and/or prevention.

With the above context of the invention, a "polypill" is a medication that is a drug product in pill form (i.e., tablet or capsule) that combines multiple active pharmaceutical ingredients. The prefix "poly" means "multiple," referring to the multiplicity of distinct drugs in a given "pill." An occasional synonym with the context of the invention is combopill.

It is commonly manufactured as a fixed-dose combination drug product targeting treatment or prevention of e.g. chronic diseases. Polypills may be administered in methods of treatment/prevention of the aforementioned diseases and / or comorbidities of the invention as a means of preventive medicine, and/or treating actual pathophysiological condition(s), the former typically involving lower dosages than the latter. With the context of the invention, polypills may reduce the number of tablets or capsules (e.g. when orally administered) that need to be taken, which in turn may facilitate handling and administration of pharmaceuticals as well as alleviate patient pill-burden.

In a specific aspect of the invention, the multiple drugs in a given polypill might all be aimed at a single underlying condition (or, group of related conditions).

With the context of the instant invention, polypill(s) is/are a combinatorial drug product(s) of the aforemention pharmaceutical drug combinations.

In addition to the fixed-dose types of polypills, with the context of the invention, polypills can also be individually custom-made for specific patients through so-called pharmacy compounding.
In yet another aspect, the present invention comprises the use of the compounds of the general formula (I) or a pharmaceutically acceptable salt or solvate or a solvate of the salt of said compound or a pharmaceutically acceptable co-crystal of said compound or a polymorph of said co-crystal for the manufacture of a medicament (such as a unit dosage tablet or unit dosage capsule, or a dual, tripple or polypill with at least one of the additional pharmaceutical agents as recited herein) for use in a method of treatment and/or prevention of one or more of the conditions previously identified in the above sections discussing its use in methods of treatment and/or prevention.

The present invention is further directed to pharmaceutical compositions of the compounds of the general formula (I) or a pharmaceutically acceptable salt or solvate thereof, particularly a hydrate thereof, or a solvate of the salt of said compound or a pharmaceutically acceptable co-crystal of said compound or a polymorph of said co-crystal.

In accordance with the instant invention, a pharmaceutical composition may be prepared, packaged, or sold in bulk, as a single unit dose, or as a plurality of single unit doses.

As used herein, a "unit dose" is a discrete amount of the pharmaceutical composition comprising a predetermined amount of the active ingredient; which is at least a compound of the general formula (I) or a pharmaceutically acceptable salt or solvate or a solvate of the salt of said compound or a pharmaceutically acceptable co-crystal of said compound or a polymorph of said co-crystal of the invention. The amount of said active ingredient is generally equal to the dosage of the active ingredient, which would be administered to a subject or a convenient fraction of such a dosage such as, for example, one-half or one-third of such a dosage.

Furthermore, a pharmaceutical composition of the invention may be prepared, packaged, or sold in form of a dual, tripple or polypill with one or more of the additional pharmaceutical agents as recited above.

Thereby, the amount of the active ingredient is also generally equal to the dosage of the active ingredient, which would be administered to a subject or a convenient fraction of such a dosage such as, for example, one-half or one-third of such a dosage.

The present invention is further directed to advantageous pharmaceutical formulations of the compounds of the general formula (I) or a pharmaceutically acceptable salt or solvate or a solvate of the salt of said compound or a pharmaceutically acceptable co-crystal of said compound or a polymorph of said co-crystal.
Given that the compounds of the general formula (I) of the invention are poorly water-soluble, the inventors surprisingly and unexpectedly found specific pharmaceutical formuliations that significantly increase solubility of the compounds of the general formula (I), and particularly of the compounds A and C, or a pharmaceutically acceptable salt or solvate or a solvate of the salt of said compounds or a pharmaceutically acceptable co-crystal of said compounds or a polymorph of said co-crystal, which results in an advatageous dissolution behavior and thus increased bioavailability after being administered in the methods of treatment and/or prevention of the aforementioned diseases/conditions and / or its comorbidities.

For instance, compound A is a synthetic heterocyclic compound with molecular weight around 500 and pKa at 2.3. Typically, this compound exhibits very poor solubility in aqueous media (pH 2.0: 0.4 mg/ml, pH 7.4: 50 - 70 µg/ml). Also, compound A shows very low solubility in the commonly used pharmaceutical excipients, such as oils, cosolvents, and surfactants. But compound A is stable in aqueous and physiological fluids as well as organic solvents.

For the above outlined problem, the instant inventors found specific pharmaceutical formulations which enhance solubility of the compounds of general formula (I), particularly of the compounds A and C, and thus enhance its dissolution profiles and bioavailability properties, whereby the stability of the compounds of the invention remains.

With the above context of the invention, the compounds of general formula (I) or a pharmaceutically acceptable salt or solvate or a solvate of the salt of said compound or a pharmaceutically acceptable co-crystal of said compound or a polymorph of said co-crystal may be administered as a pharmaceutical formulation comprising a pharmaceutically effective amount of a compound of general formula (I) or a pharmaceutically acceptable salt or solvate or a solvate of the salt of said compound or a pharmaceutically acceptable co-crystal of said compound or a polymorph of said co-crystal in association with one or more pharmaceutically acceptable excipients including carriers, vehicles and diluents in the methods of treatment/prevention as disclosed herein.

The compounds as disclosed herein may be formulated for oral, buccal, intranasal, parenteral (e.g., intravenous (i.v.), intramuscular (i.m.) or subcutaneous (s.c.)), transdermal or rectal administration or in a form suitable for administration by inhalation. The compounds of the invention may also be formulated for sustained delivery.

In a specific aspect of the invention, the inventors found the use of hot-melt extrusion suitable to formulate the compounds of the general formula (I), particularly of compounds A and C, or a pharmaceutically acceptable salt or solvate or a solvate of the salt of said compounds or a pharmaceutically acceptable co-crystal of said compounds or a polymorph of said co-crystal as poorly water-soluble API_{S}.
The inventors found the following solid dispersions to improve bioavailability of the poorly soluble drugs by converting a crystalline API into its corresponding amorphous form.

Thus, yet in a specific aspect of the invention, solid dispersion formulations for the compounds of general formula (I), and particularly for the compounds A and C, or a pharmaceutically acceptable salt or solvate or a solvate of the salt of said compounds or a pharmaceutically acceptable co-crystal of said compounds or a polymorph of said co-crystal are provided that are based on
- HPMC-AS with either citric or maleic acid of 20 wt %, and
- containing up to 30 wt % of a compound of the general formula (I), particularly of a compound A or C, or a pharmaceutically acceptable salt or solvate or a solvate of the salt of said compounds or a pharmaceutically acceptable co-crystal of said compounds or a polymorph of said co-crystal.

With the above context, particularly, the hot-melt extrusion process was able to fully convert crystalline API; i.e. a compound of general formula (I), particularly a compound A or C, or a pharmaceutically acceptable salt or solvate or a solvate of the salt of said compound or a pharmaceutically acceptable co-crystal of said compound or a polymorph of said co-crystal into its corresponding amorphous form for the following pharmaceutical formulation of the invention:
- a compound of the general formula (I), particularly a compound A or C, or a pharmaceutically acceptable salt or solvate or a solvate of the salt of said compound or a pharmaceutically acceptable co-crystal of said compound or a polymorph of said co-crystal as API with 10 wt %
- citric acid with 30 wt %, and
- HPMC-AS with 60 wt %.

With the above context of the invention, the inventors further found that a stable solid dispersion of the compounds of general formula (I), particularly of compound A or C, or a pharmaceutically acceptable salt or solvate or a solvate of the salt of said compound or a pharmaceutically acceptable co-crystal of said compound or a polymorph of said co-crystal is obtainable by using the following ranges of API and relevant excipients:
- a compound of general formula (I), particularly a compound A or C, or a pharmaceutically acceptable salt or solvate or a solvate of the salt of said compound or a pharmaceutically acceptable co-crystal of said compound or a polymorph of said co-crystal as API with a drug load of 2,5 wt % to 40 wt %,
- citric acid with 5 wt % to 40 wt %
- HPMC-AS with 50 wt % to 90 wt %.

With the context of the present invention, preferred polymers for preparing said solid dispersion of a compound of general formula (I), particularly of compound A or C, or a pharmaceutically acceptable salt or solvate or a solvate of the salt of said compound or a pharmaceutically acceptable co-crystal of said compound or a polymorph of said co-crystal are selected from a group comprising celluloses / modified cellulose, Eudragits and polyvinyl pyrrolides, poloxamers, and PEGs.

With the above context of the invention, preferred organic acids that are to be added to improve solubilization of compounds of general formula (I), particularly of compound A or C, or a pharmaceutically acceptable salt or solvate or a solvate of the salt of said compound or a pharmaceutically acceptable co-crystal of said compound or a polymorph of said co-crystal in the polymeric matrix are selected from a group comprising citric acid, maleic acid, tartaric acid, ascorbic acid, adipic acid, aspartatic acid, benzenesulfonic acid, glucoheptonic acid, D-gluconic acid, L-glutamic acid, lactic acid, L-lysine, methanesulfonic acid, p-toluenesulfonic acid.

The inventors also surprisingly found that nanomilling allows to formulate a stable nanoparticular suspension with a compound of general formula (I), particularly of a compound A or C, or a pharmaceutically acceptable salt or solvate or a solvate of the salt of said compounds or a pharmaceutically acceptable co-crystal of said compounds or a polymorph of said co-crystal and so to decrease the particle size, whereby simultaneously the particle surface increases significantly, which improves bioavailability of the compound of general formula (I), particularly of compound A or C or a pharmaceutically acceptable salt or solvate or a solvate of the salt of said compounds or a pharmaceutically acceptable co-crystal of said compounds or a polymorph of said co-crystal as API.

With the above context, the instant inventors surprisingly found that a stable nanosuspension is obtainable with nanosuspension formulations comprising:
- a compound of general formula (I), particulary of compound A or C, or a pharmaceutically acceptable salt or solvate or a solvate of the salt of said compounds or a pharmaceutically acceptable co-crystal of said compounds or a polymorph of said co-crystal with 1 wt % to 40 wt %, and/or
- a surfactant(s) selected from the group comprising:
   - Tween 80: 0.1 wt % to 10 wt %
   - Sodium deoxycholate: 0.1 wt % to 10 wt %
   - Sodium taurocholate: 0.1 wt % to 10 wt %
   - Sodium glycocholate: 0.1 wt % to 10 wt %
   - Solutol HS 15: 0.1 wt % to 10 wt %
   - Spans: 0.1 wt % to 10 wt %
   - Soluplus: 0.1 wt % to 10 wt %
   - TPGS:0.1 wt%to 10 wt %
   - SLS:0.1 wt % to 10 wt %
   - polyoleate: 0.1 wt % to 10 wt %
      - added either alone or in combination, and/or
- polymers selected from the group comprising:
   - Polyvinylpyrridone (povidone): 0.5 wt % to 5 wt %
   - Poloxamer 188: 0.5 wt % to 5 wt %
   - Cellolosics: 0.5 wt % to 5 wt %
      - added either alone or in combination.

In accordance with the instant invention both above mentioned surfactants and polymers represent stabilizers for the herein disclosed nanosuspension formulations of a compound of general formula (I), particularly of a compound A or C or a pharmaceutically acceptable salt or solvate or a solvate of the salt of said compounds or a pharmaceutically acceptable co-crystal of said compounds or a polymorph of said co-crystal.

With the above context, according to the invention, a preferred nanosuspension comprises at least one compound of the general formula (I) or a pharmaceutically acceptable salt or solvate or a solvate of the salt of said compound or a pharmaceutically acceptable co-crystal of said compound or a polymorph of said co-crystal and Sodiumglycocholate (SGC), Poloxamer 188, and water.

Another preferred nanosuspension of the invention comprises at least one compound of the general formula (I), particularly of a compound A or C, or a pharmaceutically acceptable salt or solvate or a solvate of the salt of said compounds or a pharmaceutically acceptable co-crystal of said compounds or a polymorph of said co-crystal and Sodiumdeoxycholate (SDC), Povidone (PVP K25) and water.

In yet another aspect of the invention, for oral, transdermal, subcutaneous, intramuscular, parenteral injection/application the above disclosed nanosuspensions, particularly aqueous nanosuspensions, are provided as formulations for the compounds of the general formula (I), particularly for the compounds A and C or a pharmaceutically acceptable salt or solvate or a solvate of the salt of said compounds or a pharmaceutically acceptable co-crystal of said compounds or a polymorph of said co-crystal.

In yet another aspect of the invention, the here disclosed stable nanosuspensions of the compounds of general formula (I), particularly of the compounds A or C, or a pharmaceutically acceptable salt or solvate or a solvate of the salt of said compounds or a pharmaceutically acceptable co-crystal of said compounds or a polymorph of said co-crystal are provided for a long acting parenteral, for subcutaneous, for transdermal as well as for oral application thereof in the the methods of treatment/prevention for the herein disclosed cardiovascular diseases and / or comorbidities thereof.

In yet another aspect of the invention, the inventors found a mesoporous silica particle based formulation of the compounds of general formula (I), in particular of a compound A or C or a pharmaceutically acceptable salt or solvate or a solvate of the salt of said compounds or a pharmaceutically acceptable co-crystal of said compounds or a polymorph of said co-crystal as API to convert the crystalline API into the corresponding amorphous form, which surprisingly and unexpectedly increases dissolution rate of the API and thus its bioavailability.

Mesoporous silica particles have typical diameter in the range of approx. 50-250 µm and a narrow particle size distribution. The structure and morphology of these particles can be controlled at nano to micro scale and enable to generate high surface areas and pore volumes allowing for high drug loading; e.g. of 40 wt % - 50 wt % API load. The entrapment of drugs in the mesopores leads to conversion of crystalline drug into its amorphous form.

Thus, in yet another aspect of the invention, the instant inventors provide a stable mesoporous silica particle based formulation of the compounds of general formula (I), in particular of a compound A or C, or a pharmaceutically acceptable salt or solvate or a solvate of the salt of said compounds or a pharmaceutically acceptable co-crystal of said compounds or a polymorph of said co-crystal which is obtainable by impregnation of approx. 5 wt % to 40 wt % of a compound of general formula (I) as API, particularly of a compound A or C, or a pharmaceutically acceptable salt or solvate or a solvate of the salt of said compounds or a pharmaceutically acceptable co-crystal of said compounds or a polymorph of said co-crystal onto silica material, preferably OMS-7 silica material.

The inventors further found that the concentration of silica material in the pharmaceutical formulations based on mesoporous silica particles can range from 10 wt % to 95 wt %.

To further enhance the release of a compound of general formula (I), particularly of a compound A or C or a pharmaceutically acceptable salt or solvate or a solvate of the salt of said compounds or a pharmaceutically acceptable co-crystal of said compounds or a polymorph of said co-crystal of the invention as API from the mesoporous silica material, various polymers and surfactants can be added to the above formulation. Thereby, the concentration of said polymers and surfactants may range from 0.01 wt % to 5 wt %.

With the above context, in accordance with the invention, said surfactants are preferably selected from a group comprising polysorbates, spans, poloxmaers, TPGS, lecithin.

With the above context, in accordance with the invention, said polymers are preferably selected from a group comprising celluloses, polyvinylpyrrolidones (PVP), polythethylene glycols (PEG), Soluplus.

Typically, in accordance with the invention, said surfactants and polymers can range in concentration between 1 wt % and 40 wt %, provided that a 1:1 ratio with the compound of general formula (I), particularly of a compound A or C, or a pharmaceutically acceptable salt or solvate or a solvate of the salt of said compounds or a pharmaceutically acceptable co-crystal of said compounds or a polymorph of said co-crystal is given.

In this regard, the inventors surprisingly found that sodium lauryl sulphate (SLS) is advantageous as surfactant and that hydroxypropylmethyl cellulose (HPMC) is advantageous as polymer. Preferably, the ratio of said surfactant and polymer with a compound of general formula (I) or a pharmaceutically acceptable salt or solvate or a solvate of the salt of said compounds or a pharmaceutically acceptable co-crystal of said compounds or a polymorph of said co-crystal as API is 1:1 in accordance with the present invention.

With the above context, the here disclosed silica-based drug delivery technology for the compounds of general formula (I), particularly for a compound A or C, or a pharmaceutically acceptable salt or solvate or a solvate of the salt of said compounds or a pharmaceutically acceptable co-crystal of said compounds or a polymorph of said co-crystal provides for a solubility-enhancing formulation for the otherwise poorly water-soluble compounds of general formula (I), particularly compound A or C, or a pharmaceutically acceptable salt or solvate or a solvate of the salt of said compounds or a pharmaceutically acceptable co-crystal of said compounds or a polymorph of said co-crystal as API of the invention.

In yet another aspect of the invention, moreover the inventors found that for the compounds of the invention in higher concentrations of ≥ 200 mg/kg, preferably ≥ 300 mg/kg, more preferably ≥ 400 mg/kg, most preferred ≥ 500 mg/kg, the addition of lipophilic vehicles such as Capmul^{®} MCM EP C8 can be used for pharmaceutical formulation in accordance with the present invention.

Thus, in another aspect of the invention, Capmul^{®}-MCM EP C8 based formulations of the compounds of general formula (I), particulary of a compound A or C, or a pharmaceutically acceptable salt or solvate or a solvate of the salt of said compounds or a pharmaceutically acceptable co-crystal of said compounds or a polymorph of said co-crystal are disclosed, said Capmul^{®}-based formulations comprising:
- a compound of general formula (I), particularly of compound A or C, or a pharmaceutically acceptable salt or solvate or a solvate of the salt of said compounds or a pharmaceutically acceptable co-crystal of said compounds or a polymorph of said co-crystal as API
- a vehicle Capmul^{®}-MCM EP C8 or a similar vehicle
- acetic acid or a similar acid, such as lactic acid.

Thereby, for the Capmul^{®}-based formulations as found by the inventors,
- the drug loading of a compound of general formula (I), particularly of a compound A or C, or a pharmaceutically acceptable salt or solvate or a solvate of the salt of said compounds or a pharmaceutically acceptable co-crystal of said compounds or a polymorph of said co-crystal can range from 0.1 to 10 mg/ml,
- the vehicle Capmul^{®}-MCM EP C8 can range in these formulations between 5 wt % to 85 wt %;
- whereas the added acetic acid can range from 1 wt % to 15 wt %.

In yet another aspect, the inventors further found that for the compounds of the invention in higher concentrations of ≥ 200 mg/kg, preferably ≥ 300 mg/kg, more preferably ≥ 400 mg/kg, most preferred ≥ 500 mg/kg, suspension formulation with the vehicle Capmul^{®} allows for suitable administration thereof; whereby the Labrafil concentration is not higher than 50 %.

During testing, the suspension formulations of the present invention allowed to administer compounds of general formula (I), particularly of a compound A or C, or a pharmaceutically acceptable salt or solvate or a solvate of the salt of said compounds or a pharmaceutically acceptable co-crystal of said compounds or a polymorph of said co-crystal with doses up to 500 mg/kg - without the need of a concentration of Labrafil above 50 %.

Thereby, in this specific embodiment of the invention for the Capmul^{®}-based suspension formulations as found by the inventors, the drug loading of a compound of general formula (I) or a pharmaceutically acceptable salt or solvate or a solvate of the salt of said compounds or a pharmaceutically acceptable co-crystal of said compounds or a polymorph of said co-crystal can range from 0.1 to 50 mg/ml and the Capmul MCM EP C8 concentration can range from 5% to 70%, whereby the acetic acid component in this formulation can range from 1% to 15%, and the Labrafil^{®} component can range from 5% to 50%.

Since the present invention has an aspect that relates to the use of the compounds of general formula (I) or a pharmaceutically acceptable salt or solvate or a solvate of the salt of said compounds or a pharmaceutically acceptable co-crystal of said compounds or a polymorph of said co-crystal in methods of treatment and/or prevention of the disease/conditions described herein with a combination of active ingredients which may be administered separately (see above), the invention also relates to combining separate pharmaceutical compositions in form of a kit.

The kit of the invention comprises at least two separate pharmaceutical compositions: a compound of general formula (I) or a pharmaceutically acceptable salt or solvate or a solvate of the salt of said compounds or a pharmaceutically acceptable co-crystal of said compounds or a polymorph of said co-crystal and a second compound as described above.

The kit of the invention further comprises a means for containing the separate compositions such as a container, a divided bottle or a divided foil packet. Typically the kit comprises directions for the administration of the separate components.

In another aspect of the invention, a kit may comprise bilayered tablets sachet pellets filled in capsules (i.e. the so-called stick-pack technology).

With the context of the invention, the kit form is particularly advantageous when the separate components are preferably administered in different dosage forms (e.g., oral, parenteral, long acting parenteral), are administered at different dosage intervals, or when titration of the individual components of the combination is desired by the prescribing physician.

Also, as the present invention has an aspect that relates to the use of the compounds of general formula (I) or a pharmaceutically acceptable salt or solvate or a solvate of the salt of said compounds or a pharmaceutically acceptable co-crystal of said compounds or a polymorph of said co-crystal in methods of treatment and/or prevention of the disease/conditions described herein with a combination of active ingredients which may be administered jointly, the invention also relates to combining separate pharmaceutical compositions in a single dosage form, such as (but not limited to) a single tablet or capsule, a bilayer or multilayer tablet or capsule, or through the use of segregated components or compartments within a tablet or capsule.

Moreover, as the present invention has an aspect that relates to the use of the compounds of general formula (I) or a pharmaceutically acceptable salt or solvate or a solvate of the salt of said compounds or a pharmaceutically acceptable co-crystal of said compounds or a polymorph of said co-crystal in methods of treatment and/or prevention of the disease/conditions described herein with a combination of one or more active ingredients which may be administered jointly, the invention also relates to combining separate pharmaceutical compositions in a dual, tripple or polypill dosage form.

With the context of the invention, the active ingredient of the general formula (I) or a pharmaceutically acceptable salt or solvate or a solvate of the salt of said compounds or a pharmaceutically acceptable co-crystal of said compounds or a polymorph of said co-crystal in accordance with the invention may be delivered as a suspension or a nanosuspension in an aqueous or non-aqueous vehicle, with or without additional solvents, co-solvents, excipients, or complexation agents selected from pharmaceutically acceptable diluents, excipients, vehicles, or carriers.

The compounds of the general formula (I), particularly a compound A or C, or a pharmaceutically acceptable salt or solvate or a solvate of the salt of said compounds or a pharmaceutically acceptable co-crystal of said compounds or a polymorph of said co-crystal of the invention, for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD demonstrate an unexpected and surprising, valuable pharmacological action spectrum (see Example 7 for further details).

With the context of the instant invention, exemplarily compound A was formulated in different pharmaceutical formulations of the present invention.

Thereby, compound A was orally administered as:
- non-micronized material,
- micronized material and
- ethane sulfonate of compound A suspended in Labrafil^{®} M1944CS, and
- as non-micronized material in three further formulations; i.e.
   mesoporous silica particles, aqueous nanosuspension and co-solvent based solution or suspension,
to mice and dogs in order to demonstrate exemplarily the highest systemic exposure of compound A.

Compound A was formulated as suspension in Labrafil^{®} M1944CS, mesoporous silica particles, aqueous nanosuspension and co-solvent based solution. Single doses of 30 or 500 mg/kg were administered to mice except the co-solvent based solution, which was administered at 100 or 500 mg/kg.

In addition, a single oral dose of 30 or 100 mg/kg was applied to dogs. Pharmacokinetic parameters were assessed and compared (see Example 7). Thereby, micronized, non-micronized compound A and the ethane sulfonate of compound A were orally administered to dogs formulated as a Labrafil^{®} M1944CS suspension at a dose of 30 mg/kg. The micronization of compound A and the use of an ethane sulfonate of compound A showed no increase of exposure when compared to the non-micronized material.

In general, compound A administered as Labrafil^{®} M1944CS suspension and as mesoporous silica particles showed the most favourable results in terms of Cₘₐₓ and maximal exposure in both species; dogs and mice. Similar plasma concentration time profiles were observed after oral administration of compound A formulated either as Labrafil^{®} M1944CS suspension or as mesoporous silica particles.

Therefore, in another aspect of the invention, the compounds of the general formula (I) or a pharmaceutically acceptable salt or solvate or a solvate of the salt of said compounds or a pharmaceutically acceptable co-crystal of said compounds or a polymorph of said co-crystal may be administered as Labrafil^{®} M1944CS suspension and as mesoporous silica particles formulation to achieve an improved pharmacokintetic spectrum in an individual who is in need of such administration.

The compounds of the general formula (I) or a pharmaceutically acceptable salt or solvate or a solvate of the salt of said compounds or a pharmaceutically acceptable co-crystal of said compounds or a polymorph of said co-crystal for use in methods of treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD in accordance with the invention did not show any adverse effects on cardiovascular parameters other than the reversible reduction in heart rate. No adverse effects on locomotion could be determined.

Specifically, as detailed in Example 4 below, no adverse effects on QTc-interval with administration of the exemplary compound A of the invention could be determined. Hereby, the person skilled in the art is aware that the QTc-interval is a medical indicator of the safety and tolerability aspects of a cardiovascular drug, when being administered to an individual. No effects on the QTc-interval of an administered drug prove *inter alia* excellent tolerability and safety thereof, whereas prolonged QTc causes premature action potentials during the late phases of depolarization. This generally would increase the risk of developing ventricular arrhythmias or fatal ventricular fibrillations.
In general, higher rates of prolonged QTc are seen in females, older patients, high systolic blood pressure or heart rate, and short stature. Prolonged QTc is also associated with ECG findings called Torsade de Pointes, which are known to degenerate into ventricular fibrillation, associated with higher mortality rates. There are many causes of prolonged QTc intervals, acquired causes being more common than genetic.

Given the problems underlying the invention as mentioned in the introduction portion of the present application, it is advantageous to provide for pure and very potent inhibitors of the I*_{f}* current, whereby the administration is well tolerated by patients which thus would suffer no or only minor side effects, when directly compared to e.g. ivabradine treatment.

In the below outlined animal study with beagle dogs despite the desired reversible effects on significant reduction of heart rate under treatment phase with a compound of general formula (I); i.e. exemplarily compound A, no other adverse events could be noticed. That is, the instant inventors foresee, that in contrast to ivabradine, by administering lower amounts of an API according to the general formula (I), contraindications in severe bradycardia, II or III degree atrio-ventricular block, ejection fraction < 35%, and severe hypotension, may be avoided, by the findings of the present invention.

Accordingly, no signs of too much slowing of the heart rate (severe bradycardia) nor atrial fibrillation could be recognized by administration of the exemplary compound A in the animal beagle dog study described below in more detail.

Thus, in another aspect of the invention, the compounds of the general formula (I), and particularly the compound A or C, or a pharmaceutically acceptable salt or solvate or a solvate of the salt of said compounds or a pharmaceutically acceptable co-crystal of said compounds or a polymorph of said co-crystal are therefore suitable for use as medicaments in a method for treating and/or preventing cardiovascular diseases and / or comorbidities thereof, in particular for use in methods of treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD in humans and animals.

As outlined above, the compounds of general formula (I) or a pharmaceutically acceptable salt or solvate or a solvate of the salt of said compounds or a pharmaceutically acceptable co-crystal of said compounds or a polymorph of said co-crystal of the invention are suitable for the use in methods of treatment and/or prevention of the aforementioned diseases.

With the above context, in yet another aspect of the invention, the compounds of the invention can therefore be also used in medicaments for the treatment and/or prevention of cardiovascular diseases such as high blood pressure (hypertension), resistant hypertension, acute and chronic heart failure, coronary heart disease, stable and unstable angina pectoris, peripheral and cardiac vascular disorders, arrhythmias, arrhythmia of the atria and the chambers and conduction disorders such as atrio-ventricular blockade grade I-III (AV block I-III), supraventricular tachyarrhythmia, atrial fibrillation, atrial fibrillation, ventricular fibrillation, ventricular flutter, ventricular tachyarrhythmia, torsades de pointes tachycardia, extrasystoles of atrium and the ventricle, AV junctional premature beats, sick sinus syndrome, syncope, Wolff-Parkinson-White syndrome, of acute coronary syndrome (ACS), autoimmune heart disease (pericarditis, endocarditis, Valvolitis, aortitis, cardiomyopathies), shock such as cardiogenic shock, septic shock, and anaphylactic shock, aneurysms, premature ventricular contraction (PVC), for the treatment and / or prophylaxis of thromboembolic disorders and ischaemias such as myocardial ischemia, myocardial infarction, stroke, cardiac hypertrophy, transitory and ischemic attacks, preeclampsia, inflammatory cardiovascular diseases, spasms of the coronary and peripheral arteries, edema, such as pulmonary edema, cerebral edema, renal edema or heart failure-related edema, peripheral circulatory disorders, reperfusion damage, arterial and venous thrombosis, microalbuminuria, heart failure, endothelial dysfunction, for the prevention of restenosis such as after thrombolysis treatments, percutaneous-transluminalen angioplasty (PTA), transluminal coronary angioplasties (PTCA), heart transplants and bypass operations, and micro- and macrovascular damage (vasculitis), increased levels of fibrinogen and lower LDL density and elevated levels of plasminogen activator inhibitor 1 (PAI-1).

With the context of the invention, in yet another aspect, the compounds of general formula (I) or a pharmaceutically acceptable salt or solvate or a solvate of the salt of said compounds or a pharmaceutically acceptable co-crystal of said compounds or a polymorph of said co-crystal of the invention are provided for the use in methods of treatment and / or prevention of cardiovascular comorbidities as atherosclerosis, lipid metabolism disorders, hypolipoproteinaemias, dyslipidemia, hypertriglyceridemia, hyperlipidemia, hypercholesterolaemia, abetelipoproteinaemia, sitosterolaemia, cholesterosis, Tangier disease, obesity (adiposity), and combined hyperlipidemia and the metabolic syndrome.

With the above context, in yet another aspect of the invention, the compounds of general formula (I) or a pharmaceutically acceptable salt or solvate or a solvate of the salt of said compounds or a pharmaceutically acceptable co-crystal of said compounds or a polymorph of said co-crystal of the invention are provided for the use in methods of the treatment and / or prevention of primary and secondary Raynaud's phenomenon, of microcirculation impairments, claudication, peripheral and autonomic neuropathy, diabetic microangiopathy, diabetic retinopathy, diabetic ulcers on the extremities, gangrene, CREST syndrome, erythematosis, onychomycosis, rheumatic diseases, and may be used to promote wound healing.

The compounds of the present invention are further characterised particularly by an advantageous HCN4 inhibition profile characterized by an IC₅₀ *in vitro* of below 1 µM, preferably below 0.8 µM, more preferably below 0.6 µM, even more preferably below 0.5 µM when determined in CHO cells transiently expressing the HCN4 channel by a suitable IC₅₀ measurement.

In yet another aspect, additional subject matter of the present invention is therefore the use of the compounds of general formula (I) or a pharmaceutically acceptable salt or solvate or a solvate of the salt of said compounds or a pharmaceutically acceptable co-crystal of said compounds or a polymorph of said co-crystal in accordance with the invention in a method for treating and/or preventing cardiovascular diseases and/or comorbidties thereof in a subject suffering therefrom, wherein said subject is characterized by having a resting daytime sinus rate of ≥ 90 beats/min. (bpm), preferably ≥ 100 beats/min. (bpm) and an average 24-hours heart rate of ≥ 90 beats/min. (bpm) when measured by e.g. Holter monitoring or any other suitable method the person skilled in the art is aware of that are otherwise ruled out for being related to physiologic demands or conditions known to increase heart rate.

In yet a preferred subject matter of the present invention is the use of the compounds of the general formula (I) or a pharmaceutically acceptable salt or solvate or a solvate of the salt of said compounds or a pharmaceutically acceptable co-crystal of said compounds or a polymorph of said co-crystal in a method for treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD.

Yet a more preferred subject matter of the present invention is the use of the compounds of the general formula (I) or a pharmaceutically acceptable salt or solvate or a solvate of the salt of said compounds or a pharmaceutically acceptable co-crystal of said compounds or a polymorph of said co-crystal in accordance with the invention in a method for treating and/or preventing cardiovascular diseases selected from the group comprising CA, HHD, IST, SAP, MI, CAD, HF, and stroke, and/or for use in methods of treating and/or preventing cardiovascular comorbidities selected from the group comprising diabetes, metabolic syndrome, and obesity.

A most preferred subject matter of the present invention is the use of the compounds of the general formula (I) or a pharmaceutically acceptable salt or solvate or a solvate of the salt of said compounds or a pharmaceutically acceptable co-crystal of said compounds or a polymorph of said co-crystal in accordance with the invention in a method for treating and/or preventing cardiovascular diseases selected from the group comprising IST, SAP, MI, CAD, HF, and stroke, and/or for use in methods of treating and/or preventing cardiovascular comorbidities selected from the group comprising diabetes and metabolic syndrome.

In another aspect, subject matter of the present invention is a method for treating and/or preventing cardiovascular diseases selected from the group comprising CA, HHD, tachycardia, IST, AP and SAP, MI, CAD, HF, and stroke, and/or for use in methods of treating and/or preventing cardiovascular comorbidities selected from the group comprising diabetes, metabolic syndrome, obesity, and CKD using a therapeutically effective amount of the compounds in accordance with the present invention or a pharmaceutically acceptable salt or solvate or a solvate of the salt of said compounds or a pharmaceutically acceptable co-crystal of said compounds or a polymorph of said co-crystal.

In this connection, an additional subject matter of the present invention is, in particular, the use of a therapeutically effective quantity of the compounds of general formula (I) or a pharmaceutically acceptable salt or solvate or a solvate of the salt of said compounds or a pharmaceutically acceptable co-crystal of said compounds or a polymorph of said co-crystal in accordance with the invention in a method to prevent and/or treat cardiovascular diseases caused by increased heart rates and/or heart arrhythmias and/or hypertension.

In view of the above, further preferred subject matter in accordance with the present invention is represented by the following consecutively numbered embodiments:
1. A compound of the general formula (I) 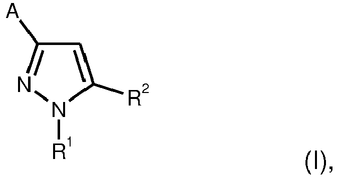 wherein
   - A: stands for a group of formula (i) or (ii) 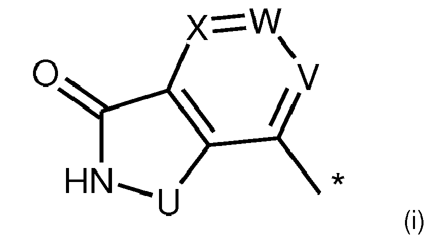
   or 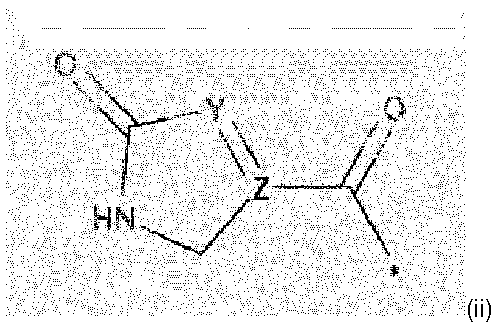 wherein in formula (i)
   - U: stands for carbon, whereby carbon can be substituted with 1 to 2 alkyl substituents, which are selected independently of one another, or with an oxo substituent,
   - V: stands for nitrogen,
   - W: stands for carbon, whereby carbon can be substituted with a substituent selected from the group consisting of halogen, amino, hydroxy, methoxy, and methyl,
   - X: stands for nitrogen or carbon, whereby carbon can be substituted with a substituent selected from the group consisting of halogen, amino, hydroxy, methoxy, and methyl, and
   - *: is the point of attachment to the carbon atom;
   wherein in formula (ii)
   - Y: stands for carbon,
   whereby carbon can be substituted with halogen, amino, hydroxy, methoxy, and methyl,
   - Z: stands for carbon or nitrogen, and
   - *: is the point of attachment to the carbon atom,
   and wherein
   - R¹: stands for phenyl or pyridyl,
   whereby phenyl is substituted with 1 to 2 substituents, the substituents being selected independently of one another from the group consisting of halogen, hydroxy, amino, cyano, and nitro
   - R²: stands for phenyl,
   whereby phenyl is substituted with 1 to 2 substituents, the substituents being selected independently of one another from the group consisting of halogen, hydroxy, amino, nitro, (C1-C4)-alkyl, (C1-C4)-alkylamino and (C1-C4)-alkoxy,
   wherein
   alkyl, alkylamino and alkoxy in turn may be substituted one to three times identically or differently with radicals selected from the group consisting of halogen, hydroxy, amino,
   and their co-crystals or the polymorphs of their co-crystals, and their salts, their solvates or the solvates of their salts
   for use in methods of treating and/or preventing cardiovascular diseases and / or comorbidities thereof.
2. The compound of formula (I) for the use in a method of treating and/or preventing cardiovascular diseases and / or comorbidities thereof according to embodiment 1,
   in which A stands for a group of said formula (i) or (ii) as represented above,
   wherein in formula (i)
   - U: stands for carbon,

   - W: stands for carbon,
   whereby carbon can be substituted with a methyl,
   - X: stands for nitrogen or carbon,
   whereby carbon can be substituted with a methyl;
   - X: preferably stands for nitrogen or carbon, and
   - *: is the point of attachment to the carbon atom,
   and wherein
   - R¹: stands for phenyl or pyridyl,
   whereby phenyl is substituted with 1 to 2 substituents, the substituents being selected independently of one another from the group consisting of halogen, cyano, and nitro,
   whereby said 1 to 2 substituents are more preferably selected independently of one another from the group consisting of halogen and cyano, and said halogen is more preferably selected independently of one another from Cl and F,
   whereby
   - R¹: preferably stands for pyridyl,
   wherein
   - R¹: more preferred stands for 3-pyridyl or 4-pyridyl,
   wherein
   - R¹: most preferred stands for 3-pyridyl,
   and wherein
   - R²: stands for phenyl,
   whereby phenyl is substituted with 1 to 2 substituents, the substituents being selected independently of one another from the group consisting of halogen, and (C1-C4)-alkoxy,
   wherein
   alkoxy in turn may be substituted one to three times identically or differently with radicals selected from the group consisting of halogen,
   whereby said 1 to 2 substituents are more preferably selected independently of one another from the group consisting of halogen and (C1-C4)-alkoxy, and said halogen is more preferably selected independently of one another from Cl and F, and the alkoxy on their part can be substituted one to three times with fluorine atoms, more preferred with two fluorine atoms, most preferred with three fluorine atoms,
   - R²: preferably stands for phenyl,
   whereby phenyl is substituted with two substituents, said two substituents being more preferably selected independently of one another from the group consisting of halogen and (C1-C3)-alkoxy, and the halogen more preferably being selected independently of one another from Cl and F, and the alkoxy on their part can be substituted with three fluorine atoms,
   - R²: most preferred stands for 3-Cl-5-CF₃O-phenyl;
   wherein in formula (ii)
   - Y: stands for carbon, whereby carbon can be substituted with amino,
   - Z: stands for carbon or nitrogen, and
   - *: is the point of attachment to the carbon atom,
   and wherein
   - R¹: stands for phenyl or pyridyl,
   whereby phenyl is substituted with two substituents, the substituents being selected independently of one another from the group consisting of halogen and cyano,
   - R¹: stands preferably for phenyl or 3-pyridyl,
   whereby phenyl is substituted with two substituents, the substituents being selected independently of one another from the group consisting of halogen and cyano, whereby said halogen is more preferably selected independently of one another from Cl and F, most preferred said halogen is fluorine,
   and wherein
   - R²: stands for phenyl,
   whereby phenyl is substituted with 1 to 2 substituents, the substituents being selected independently of one another from the group consisting of halogen, and (C1-C4)-alkoxy,
   wherein
   alkoxy in turn may be substituted one to three times identically or differently with radicals selected from the group consisting of halogen,
   whereby said 1 to 2 substituents are more preferably selected independently of one another from the group consisting of halogen and (C1-C4)-alkoxy, and said halogen is more preferably selected independently of one another from Cl and F, and the alkoxy on their part can be substituted one to three times with fluorine atoms, more preferred with two fluorine atoms, most preferred with three fluorine atoms,
   - R²: preferably stands for phenyl,
   whereby phenyl is substituted with two substituents, said two substituents being more preferably selected independently of one another from the group consisting of halogen and (C1-C3)-alkoxy, and the halogen more preferably being selected independently of one another from Cl and F, and the alkoxy on their part can be substituted with three fluorine atoms,
   - R²: most preferred stands for 3-Cl-5-CF₃O-phenyl,
   and their co-crystals or the polymorphs of their co-crystals, and their salts, their solvates or the solvates of their salts.
3. The compound of formula (I) for the use in a method of treating and/or preventing cardiovascular diseases and / or comorbidities thereof according to any of the embodiments 1 or 2,
   in which A stands for a group of said formula (i) or (ii) as represented above, 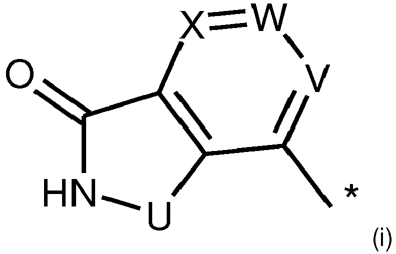 or 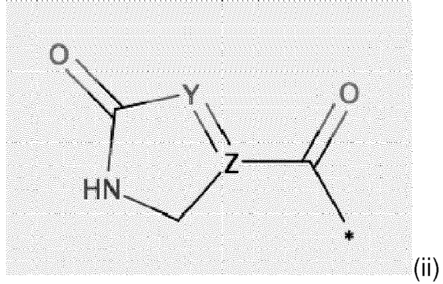 wherein in formula (i)
   - U: stands for CH2,
   - V: stands for N,
   - W: stands for CH or CMe, wherein CMe stands for C-CH3,
   - X: stands for N or CH, and
   - *: is the point of attachment to the carbon atom,
   and wherein
   - R¹: stands for phenyl or 3-pyridyl or 4-pyridyl,
   preferably for 3-pyridyl, and
   whereby phenyl is substituted with two substituents, whereby the substituents are selected independently of one another from the group consisting of halogen and cyano,
   - R²: stands for phenyl,
   whereby phenyl is substituted with two substituents, the substituents being selected independently of one another from the group consisting of halogen and (C1-C3)-alkoxy,
   wherein
   halogen is preferably chlorine, and
   alkoxy on their part can be substituted with three fluorine atoms;
   wherein in formula (ii)
   - Y: stands for CH2 or C-NH2,
   - Z: stands for CH or N, and
   - *: is the point of attachment to the carbon atom,
   and wherein
   - R¹: stands for phenyl or 3-pyridyl or 4-pyridyl,
   - R¹: preferably stands for 3-pyridyl, and
   whereby phenyl is substituted with two substituents, whereby the substituents are selected independently of one another from the group consisting of halogen and cyano,
   - R²: stands for phenyl,
   whereby phenyl is substituted with two substituents, the substituents being selected independently of one another from the group consisting of halogen and (C1-C3)-alkoxy,
   wherein
   halogen is preferably chlorine, and
   alkoxy on their part can be substituted with three fluorine atoms,
   and their co-crystals or the polymorphs of their co-crystals, and their salts, their solvates or the solvates of their salts.
4. The compound of formula (I) for the use in a method of treating and/or preventing cardiovascular diseases and / or comorbidities thereof according to any of the embodiments 1 to 3,
   in which A stands for a group of said formula (i) or (ii) as represented above, 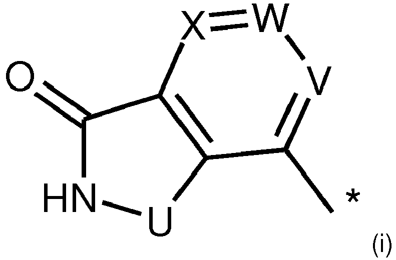 or 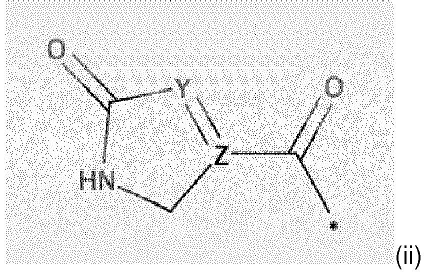 wherein in formula (i)
   - U: stands for CH2,
   - V: stands for N,
   - W: stands for CH or CMe, wherein CMe stands for C-CH3,
   - X: stands for N or CH, and
   - *: is the point of attachment to the carbon atom,
   and wherein
   - R¹: stands for 3-pyridyl,
   - R²: stands for phenyl,
   whereby phenyl is substituted with two substituents, the substituents being selected independently of one another from the group consisting of halogen and (C1-C2)-alkoxy,
   wherein
   halogen is preferably chlorine, and
   alkoxy on their part can be substituted with three fluorine atoms;
   wherein in formula (ii)
   - Y: stands for CH2 or C-NH2,
   - Z: stands for CH or N, and
   - *: is the point of attachment to the carbon atom,
   and wherein
   - R¹: stands for 3-pyridyl,
   - R²: stands for phenyl,
   whereby phenyl is substituted with two substituents, the substituents being selected independently of one another from the group consisting of halogen and (C1-C2)-alkoxy,
   wherein
   halogen is preferably chlorine, and
   alkoxy on their part can be substituted with three fluorine atoms,
   and their co-crystals or the polymorphs of their co-crystals, and their salts, their solvates or the solvates of their salts.
5. The compound of formula (I) for the use in a method of treating and/or preventing cardiovascular diseases and / or comorbidities thereof according to any of the preceding embodiments,
   in which A stands for a group of said formula (i) or (ii) as represented above, 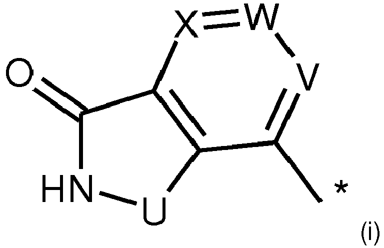 or 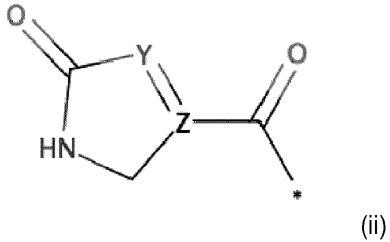 wherein in formula (i)
   - U: stands for CH2,
   - V: stands for N,
   - W: stands for CH,
   - X: stands for N, and
   - *: is the point of attachment to the carbon atom,
   and wherein
   - R¹: stands for 3-pyridyl, and
   - R²: stands for phenyl,
   whereby phenyl is substituted with two substituents, the substituents being selected independently of one another from the group consisting of halogen and (C1-C2)-alkoxy,
   wherein
   halogen is preferably chlorine, and
   alkoxy on their part is substituted with three fluorine atoms;
   wherein in formula (ii)
   - Y: stands for CH2 or C-NH2,
   - Z: stands for CH or N, and
   - *: is the point of attachment to the carbon atom,
   and wherein
   - R¹: stands for 3-pyridyl,
   - R²: stands for phenyl,
   whereby phenyl is substituted with two substituents, the substituents being selected independently of one another from the group consisting of halogen and (C1-C2)-alkoxy,
   wherein
   halogen is preferably chlorine, and
   alkoxy on their part can be substituted with three fluorine atoms,
   and their co-crystals or the polymorphs of their co-crystals, and their salts, their solvates or the solvates of their salts.
6. The compound of formula (I) for the use in a method of treating and/or preventing cardiovascular diseases and / or comorbidities thereof according to any of the preceding embodiments,
   in which A stands for a group of formula (i) or (ii) as represented above,
   wherein formula (i) is selected from a group consisting of 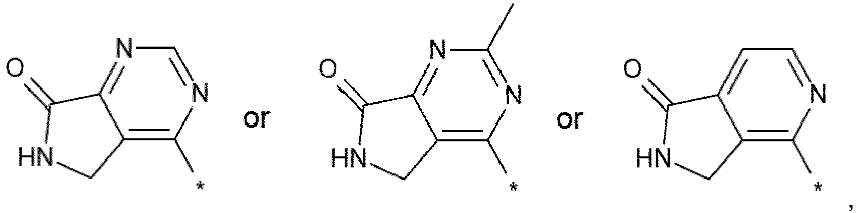 and wherein
   - R¹: is selected from a group consisting of 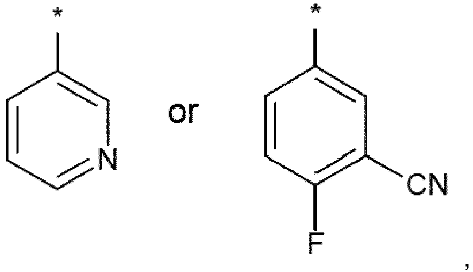
   and wherein
   - R²: is 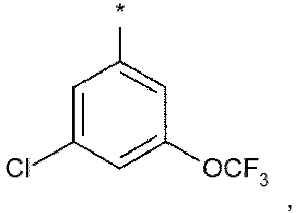
   and wherein
   - *: is the point of attachment to the carbon atom;
   wherein formula (ii) is selected from a group consisting of 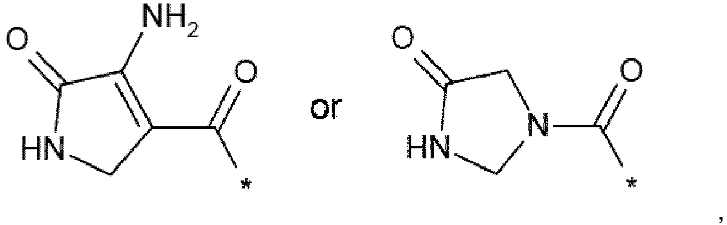 and wherein
   - R¹: is selected from a group consisting of 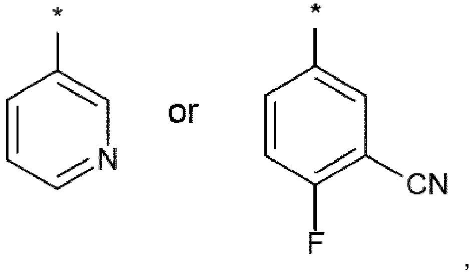
   and wherein
   - R²: is 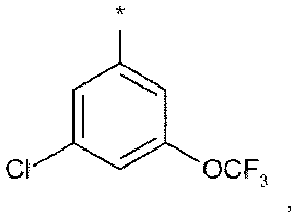
   and wherein
   - *: is the point of attachment to the carbon atom
   and their co-crystals or the polymorphs of their co-crystals, and their salts, their solvates or the solvates of their salts.
7. The compound of formula (I) for the use in a method of treating and/or preventing cardiovascular diseases and / or comorbidities thereof according to any of the preceding embodiments, with the proviso that residue A stands for formula (i),
   wherein said compound is 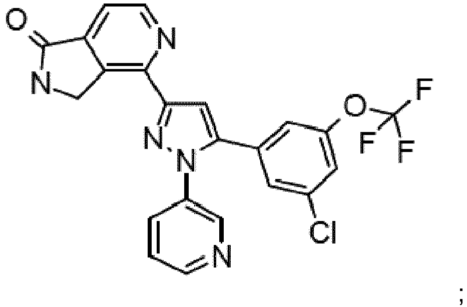 i.e. 4-[5-[3-Chloro-5-(trifluoromethoxy) phenyl]-1-(pyridin-3-yl)-1H-pyrazol-3-yl]-2,3-dihydro-1H-pyrrolo[3, 4-c]pyridin-1-one,
   and their co-crystals or the polymorphs of their co-crystals, and their salts, their solvates or the solvates of their salts.
8. The compound of formula (I) for the use in a method of treating and/or preventing cardiovascular diseases and / or comorbidities thereof according to any of the preceding embodiments, with the proviso that residue A stands for formula (i),
   wherein said compound is 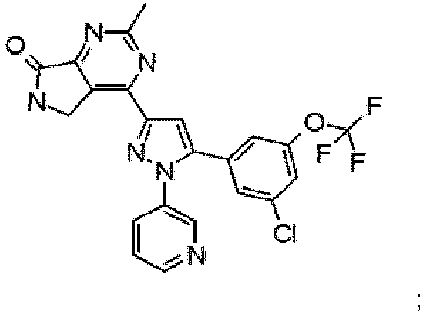 i.e. 4-[5-[3-Chloro-5-(trifluoromethoxy) phenyl]-1-(pyridin-3-yl)-1H-pyrazol-3-yl]-5,6-dihydro-2-methyl-7H-pyrrolo[3,4-d] pyrimidin-7-one,
   and their co-crystals or the polymorphs of their co-crystals, and their salts, their solvates or the solvates of their salts.
9. The compound of formula (I) for the use in a method of treating and/or preventing cardiovascular diseases and / or comorbidities thereof according to any of the preceding embodiments, with the proviso that residue A stands for formula (i),
   wherein said compound is 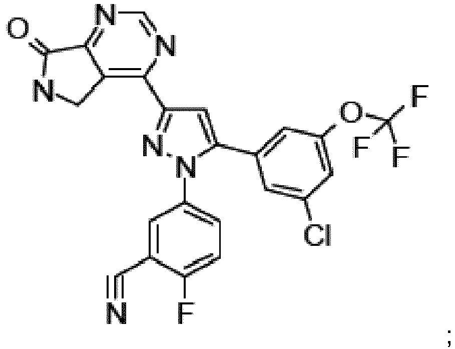 i.e. 4-[5-[3-Chloro-5-(trifluoromethoxy) phenyl]-1-(3-cyano-4-fluoro-phenyl)-1H-pyrazol-3-yl]-5,6-dihydro-7H-pyrrolo[3,4-d] pyrimidin-7-one,
   and their co-crystals or the polymorphs of their co-crystals, and their salts, their solvates or the solvates of their salts.
10. The compound of formula (I) for the use in a method of treating and/or preventing cardiovascular diseases and / or comorbidities thereof according to any of the preceding embodiments, with the proviso that residue A stands for formula (i),
   wherein said compound is 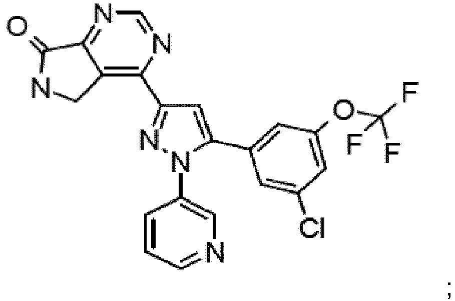 i.e. 4-[5-[3-Chloro-5-(trifluoromethoxy) phenyl]-1-(pyridin-3-yl)-1H-pyrazol-3-yl]-5,6-dihydro-7H-pyrrolo[3,4-d]pyrimidin-7-one,
   and their co-crystals or the polymorphs of their co-crystals, and their salts, their solvates or the solvates of their salts.
11. The compound of formula (I) for the use in a method of treating and/or preventing cardiovascular diseases and / or comorbidities thereof according to any of the preceding embodiments, with the proviso that residue A stands for formula (ii),
   wherein said compound is 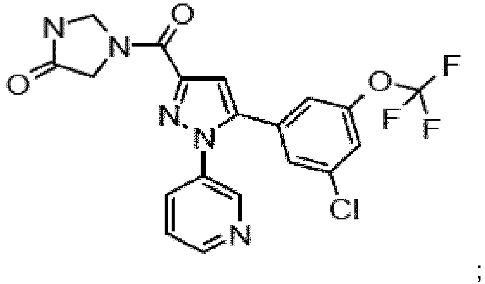 i.e. 1-[[5-[3-Chloro-5-(trifluoromethoxy) phenyl]-1-(pyridin-3-yl)-1H-pyrazol-3-yl]carbonyl]-imidazolidin-4-one,
   and their co-crystals or the polymorphs of their co-crystals, and their salts, their solvates or the solvates of their salts.
12. The compound of formula (I) for the use in a method of treating and/or preventing cardiovascular diseases and / or comorbidities thereof according to any of the preceding embodiments, with the proviso that residue A stands for formula (ii),
   wherein said compound is 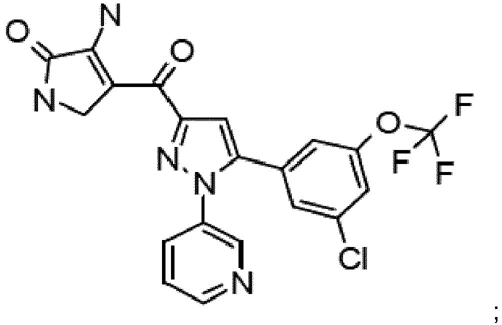 i.e. 3-Amino-4-[5-[3-chloro-5-(trifluoromethoxy) phenyl]-1-(pyridin-3-yl)-1H-pyrazol-3-yl-carbonyl]-1,5-dihydro-2H-pyrrol-2-one,
   and their co-crystals or the polymorphs of their co-crystals, and their salts, their solvates or the solvates of their salts.
13. The compound of formula (I) for the use in a method of treating and/or preventing cardiovascular diseases and / or comorbidities thereof according to any of the preceding embodiments, wherein said cardiovascular diseases are selected from a group comprising CA, HHD, tachycardia, IST, AP and SAP, MI, CAD, HF, and stroke, and wherein said comorbidities are selected from a group comprising diabetes, metabolic syndrome, obesity, and CKD.
14. Use of a compound in accordance with any of the embodiments 1 to 12 for the manufacture of a medicament for use in a method of treatment and/or prevention of cardiovascular diseases and / or comorbidities thereof.
15. The use of embodiment 14, wherein said cardiovascular diseases are selected from a group comprising CA, HHD, tachycardia, IST, AP and SAP, MI, CAD, HF, and stroke, and wherein said comorbidities are selected from a group comprising diabetes, metabolic syndrome, obesity, and CKD.
16. A pharmaceutical composition comprising at least one compound in accordance with any of the embodiments 1 to 12 in combination with at least one inert, nontoxic, pharmaceutically suitable adjuvant for the use in a method of treatment and/or prevention of cardiovascular diseases and / or comorbidities thereof.
17. The pharmaceutical composition of embodiment 16, wherein said cardiovascular diseases are selected from a group comprising CA, HHD, tachycardia, IST, AP and SAP, MI, CAD, HF, and stroke, and wherein said comorbidities are selected from a group comprising diabetes, metabolic syndrome, obesity, and CKD.
18. Method for treating and/or preventing cardiovascular diseases and / or comorbidities thereof in humans and animals, said method comprising the administration of an effective amount of at least one compound in accordance with any of the embodiments 1 to 12 or a pharmaceutical composition in accordance with any of the embodiments 16 or 17, to a human or animal requiring the same.
19. The method according to embodiment 18, wherein said cardiovascular disease and / or comorbidity thereof is treatable by HCN4 inhibition.
20. A pharmaceutical formulation, wherein said formulation is a nanosuspension comprising:
   - a compound of general formula (I) or a co-crystal thereof or the polymorph of a co-crystal thereof, or a salt thereof, or a solvate thereof or a solvate of a salt thereof according to any of the embodiments 1 to 12 with 1 wt % to 40 wt %, and further comprising
      - a surfactant(s) selected from the group comprising:
      - Tween 80: 0.1 wt % to 10 wt %,
      - Sodium deoxycholate: 0.1 wt % to 10 wt %,
      - Sodium taurocholate: 0.1 wt % to 10 wt %,
      - Sodium glycocholate: 0.1 wt % to 10 wt %,
      - Solutol HS 15: 0.1 wt % to 10 wt %,
      - Spans: 0.1 wt % to 10 wt %,
      - Soluplus: 0.1 wt % to 10 wt %,
      - TPGS:0.1 wt%to 10 wt %,
      - SLS: 0.1 wt % to 10 wt %,
      - polyoleate: 0.1 wt % to 10 wt %,
         added either alone or in combination, and/or further comprising
      - a polymer(s) selected from the group comprising:
      - Polyvinylpyrridone (povidone): 0.5 wt % to 5 wt %,
      - Poloxamer 188: 0.5 wt % to 5 wt %,
      - Cellolosics: 0.5 wt % to 5 wt %,
         added either alone or in combination.
21. A pharmaceutical formulation, wherein said formulation is a solid dispersion comprising:
   - HPMC-AS with either citric or maleic acid of 20 wt %, and
   - containing up to 30 wt % of a compound of the general formula (I) or a co-crystal thereof or the polymorph of a co-crystal thereof, or a salt thereof, or a solvate thereof or a solvate of a salt thereof in accordance with any of the embodiments 1 to 12.
22. A pharmaceutical formulation, wherein said formulation is a mesoporous silica based formulation, which is obtainable by impregnation of approx. 5 wt % to 40 wt % of a compound of general formula (I) or a co-crystal thereof or the polymorph of a co-crystal thereof, or a salt thereof, or a solvate thereof or a solvate of a salt thereof according to any of the embodiments 1 to 12 onto OMS-7 silica material, and wherein said mesoporous silica particles can range from 10 wt % to 95 wt %, and wherein polymers and surfactants can be added to said formulation, whereby the concentration of said polymers and surfactants may range from 0.01 wt % to 5 wt %.
23. A pharmaceutical formulation, wherein said formulation is a Capmul^{®} based co-solvent formulation, comprising:
   - a drug loading of a compound of general formula (I) or a co-crystal thereof or the polymorph of a co-crystal thereof, or a salt thereof, or a solvate thereof or a solvate of a salt thereof according to any of the embodiments 1 to 12 from 0.1 to 10 mg/ml,
   - the vehicle Capmul^{®} MCM EP C8 between 5 wt % to 70 wt %, and
   - acetic acid, which can range from 1 wt % to 15 wt %.
24. A pharmaceutical formulation according to any of the embodiments 20 to 23 for use in a method of treating and/or preventing cardiovascular diseases and / or comorbidities thereof, wherein said cardiovascular diseases are preferably selected from a group comprising CA, HHD, tachycardia, IST, AP and SAP, MI, CAD, HF, and stroke, and wherein said comorbidities are selected from a group comprising diabetes, metabolic syndrome, obesity, and CKD, further characterized by oral, parenteral, long acting parenteral, transdermal administration to a human and/or an animal in need thereof.
25. A process for the synthesis of the compound 4-[5-[3-Chloro-5-(trifluoromethoxy) phenyl]-1-(pyridin-3-yl)-1H-pyrazol-3-yl]-5,6-dihydro-7H-pyrrolo[3,4-d] pyrimidin-7-one of embodiment 10, said process comprises the steps of:
   Step 1)
      - reacting the starting material 3-chloro-5-(trifluoromethoxy)acetophenone (i.e. compound 1a) towards compound 2a in the presence of KOEt and MeOH as depicted below: 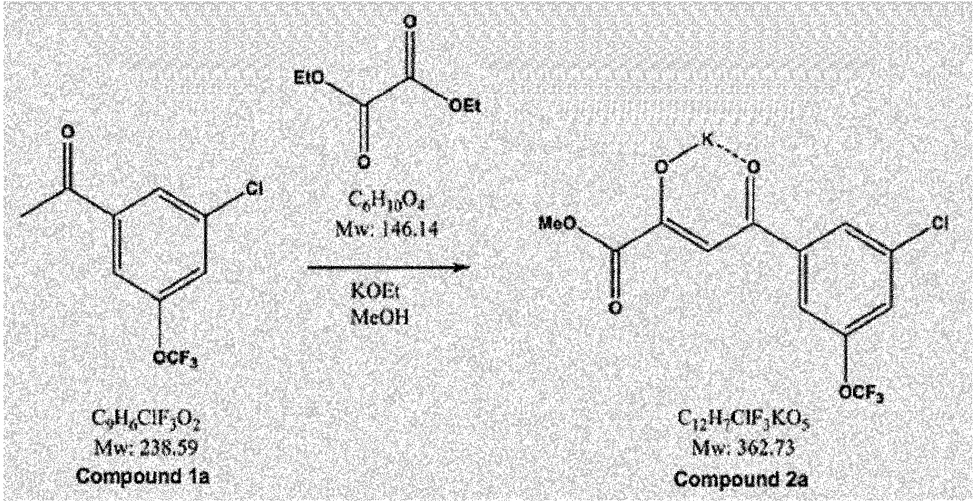
      - followed by isolation of compound 2a in solid form as its potassium salt
      - using said potassium salt without further purification in the next process step 2),
   Step 2)
      - reacting compound 2a obtained in step 1) in the presence of EtOH by a condensation reaction with compound 3a towards a compound 5a as depicted below, whereby said reaction mixture must be heated by slow rate heating,
      - followed by the reaction of intermediate compound 5a in the presence of NaOH and the addition of hydrochloric acid solution (HCl) at a pH ≥ 2, towards intermediate compound 6a,
      - after adjusting the pH, the product compound 6a precipitates out of solution and can be isolated in solid form,
      - the obtained dry compound 6a can be used without further purification in the next process step 3), 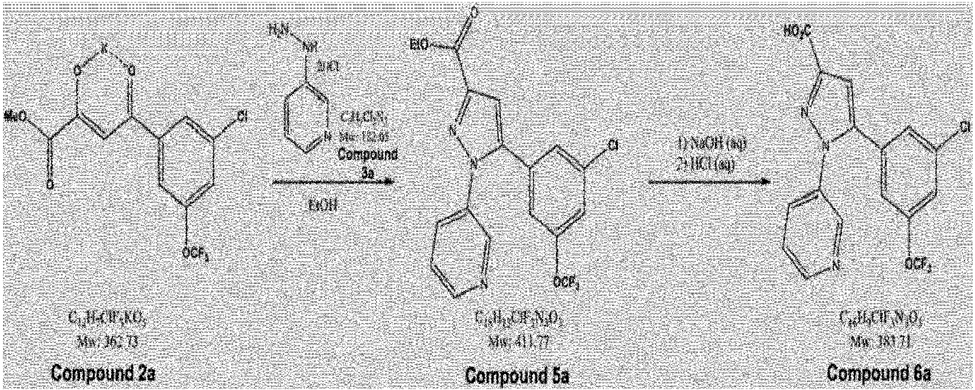
   Step 3)
      - reacting the starting material compound 6a via the intermediate acid chloride compound 7a in the presence of C2O2Cl2 and DMF as well as CH2Cl2 towards compound 8a in the presence of DiPEA as depicted below, and whereby this reaction is conducted with oxalyl chloride at ambient temperature and the N,O-dimethylhydroxyl amine is added as its HCl salt,
      - the reaction product 8a is isolated in solid form, and used without further purification in the next process step 4), 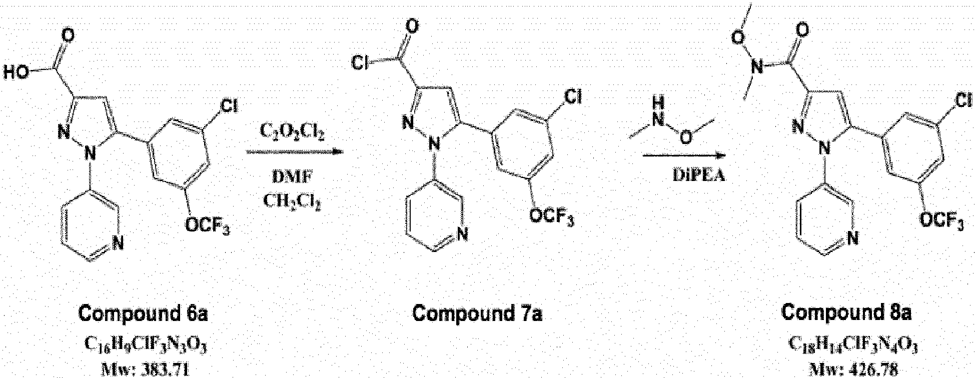
   Step 4)
      - the synthesis of compound 9a starting from the Weinreb amide compound 8a follows in the presence of MeMgCl and THF as depicted below,
      - compound 9a is isolated as a solution in THF,
      - this solution can be used in the next step of the synthesis without further purification, 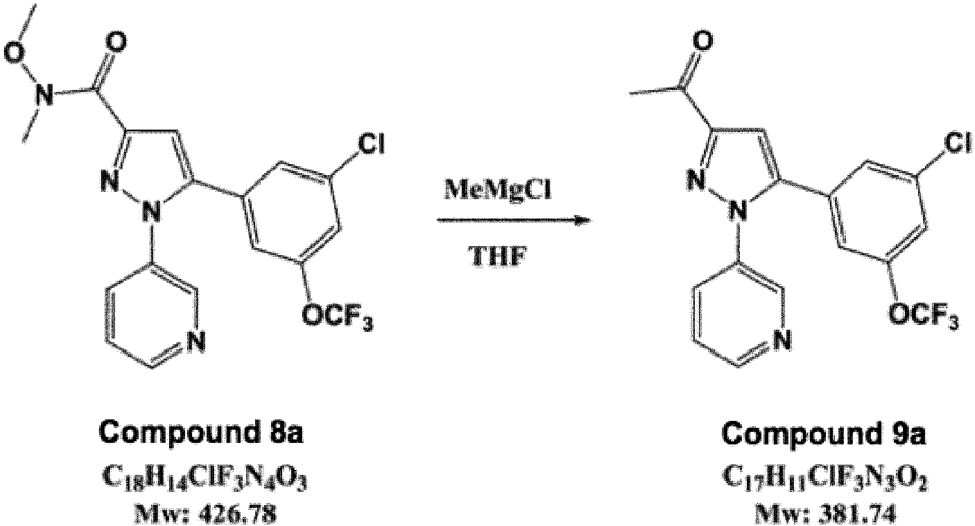
   Step 5)
      - as depicted below, the synthesis of compound 10a follows in the presence of a sodium ethoxide solution in ethanol, starting from the ketone derivative compound 9a, which is harvested as a tetrahydrofuran solution in step 4), 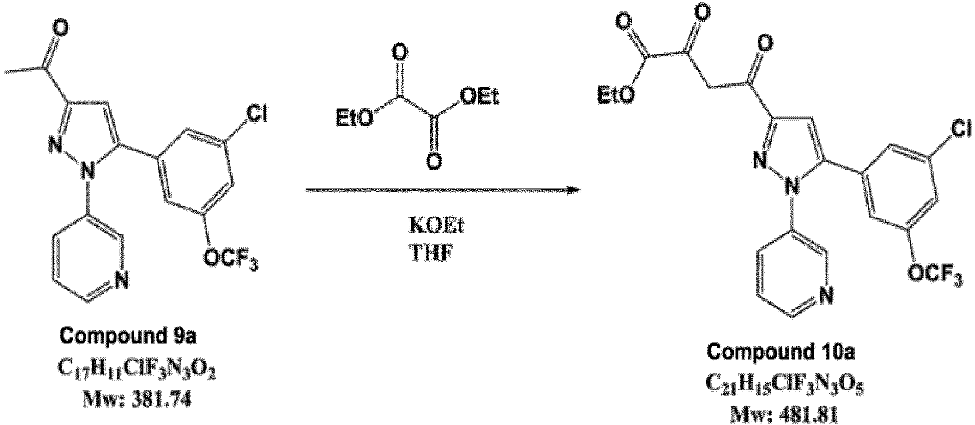
   Step 6)
      - the synthesis of compound 12a follows in a two-step process in which first a keto-imine (i.e. compound 11 a) is formed, which is not isolated, followed by a Mannich reaction yielding the compound 12a, as depicted below, whereby in both steps the solvent acetic acid is used, and whereby the ammonia donor is in both steps ammonium acetate, and whereby the first step is performed at approx. 45 ± 5°C, while the second step is performed at approx. 35 ± 5°C,
      - the product compound 12a precipitates out of solution by addition of ethanol to the reaction mixture, 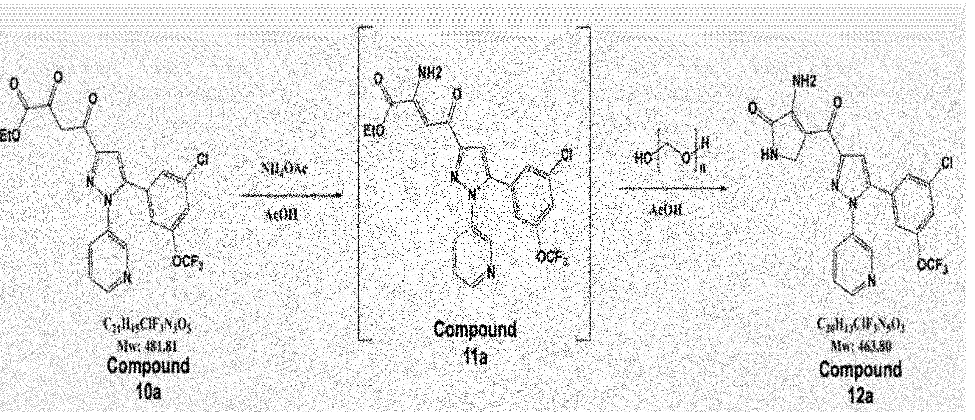
   Step 7)
      - the synthesis of compound 4-[5-[3-Chloro-5-(trifluoromethoxy) phenyl]-1-(pyridin-3-yl)-1 H-pyrazol-3-yl]-5,6- dihydro-7H-pyrrolo[3,4-d] pyrimidin-7-one as crude product follows as depicted below, starting from the intermediate compound 12a, thereby the reaction mixture is heated and kept under pressure, 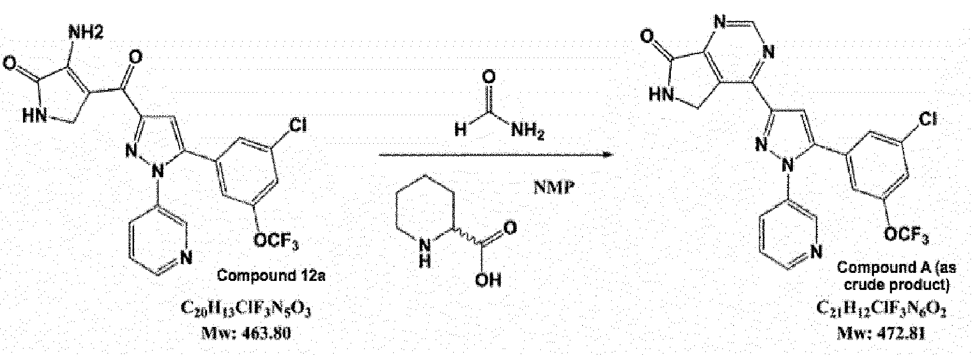
   Step 7a)
      - the finalization of the above synthesis process is characterized by two distinct purification steps, whereby
      - the first step comprises the crystallization of the crude product compound 4-[5-[3-Chloro-5-(trifluoromethoxy) phenyl]-1-(pyridin-3-yl)-1H-pyrazol-3-yl]-5,6- dihydro-7H-pyrrolo[3,4-d] pyrimidin-7-one out of an acetic acid solution, using water as anti-solvent, and
      - the second step consists of dissolution of the purified compound 4-[5-[3-Chloro-5-(trifluoromethoxy) phenyl]-1-(pyridin-3-yl)-1H-pyrazol-3-yl]-5,6-dihydro-7H-pyrrolo[3,4-d] pyrimidin-7-one in a mixture of dichloromethane and methanol in order to perform a screening filtration,
      - the purified product 4-[5-[3-Chloro-5-(trifluoromethoxy) phenyl]-1-(pyridin-3-yl)-1 H-pyrazol-3-yl]-5,6- dihydro-7H-pyrrolo[3,4-d] pyrimidin-7-one can be isolated by filtration after a solvent switch to methanol.

### Abbreviations

Throughout the entire application text, the following abbreviations do apply:
- AP: angina pectoris
- API: active pharmaceutical ingredient
- approx.: approximately
- bs: broad singlet (in NMR)
- bd: broad boublet (in NMR)
- cat.: catalytic
- CA: cardiac arrythmia
- CAD: coronary artery disease
- CKD: chronic kidney disease
- Cl: chemical ionisation (in MS)
- CMe: C-CH₃
- dd: doublet of doublet (in NMR)
- DMF: Dimethyl formamide
- DMSO: Dimethyl sulfoxide
- dt: doublet of triplet (in NMR)
- d. Th.: of theoretical yield
- EI: electron impact ionization (in MS)
- eq.: equivalent(s)
- ESI: electrospray-ionisation (in MS)
- Et: ethyl
- ges.: saturated
- h: hour(s)
- HF: heart failure
- HHD: hypertensive heart disease
- HPLC: high-pressure-, high performance liquid chromatography
- IST: inappropriate sinus tachycardia
- conc.: concentrated
- LC-MS: liquid chromatography-mass spectrometry
- LHMDS: lithium bis(trimethylsilyl)amide
- m: multiplet (in NMR)
- Me: methyl
- MI: myocardial ischemia
- min: minute(s)
- MS: mass spectrometry
- NMR: nuclear magnetic resonance
- Ph: phenyl
- q: quartet (in NMR)
- quint: quintet (in NMR)
- RT: room temperature
- Rₜ: retention time (in HPLC)
- SAN: sinoatrial node
- SAP: stable angina pectoris
- s: singlet (in NMR)
- TFA: trifluoroacetic acid
- THF: tetrahydrofuran
- UV: ultraviolet spectroscopy
- wässr.: aqueous, aqueous solution

- CC₅₀: Half cytotoxicity concentration
- DMSO: Dimethyl sulfoxide
- EC₅₀: Half maximal effective concentration
- FBS: Fetal Bovine Serum (Biochrom AG, Berlin, Germany)
- IC₅₀: Half maximal inhibitory concentration
- PBS: phosphate buffered saline
- Pen/Strep: Penicillin/Streptomycin
- RPMI: Roswell Park Memorial Institute
- MOI: multiplicity of infection
- MTP: microtitre plate
- ELISA: enzyme-linked immunosorbent assay

### Symbols

- -: not examined / no data
- 0: no pathological findings / normal
- 1 - n: reference to footnotes in the text
- #: reference to footnotes in the tables
- **: statistically significant at p ≤ 0.01
- *: statistically significant at p ≤ 0.05
- ( ): animal number (in the tables)

### Letters / Acronyms

- a/a: absorption/absorption
- a.m.: ante meridiem
- approx.: approximately
- BID, b.i.d.: bis in die (lat.; i.e. twice daily)
- b.w.: body weight
- EC: European Community
- EPA/USA: Environmental Protection Agency/United States of America
- EU: European Union
- f: female
- FDA: Food and Drug Administration
- GLP: Good Laboratory Practice
- HED: Human equivalent dose
- HTS: Head of Test Site
- i.e.: id est
- i.v.: intravenous administration
- LPT: Laboratory of Pharmacology and Toxicology
- LQAU: Leading Quality Assurance Unit (LPT)
- LUFA-ITL: Landwirtschaftliche Untersuchungs- und Forschungsanstalt, Institut für Tiergesundheit und Lebensmittelqualität
- m: male
- MAFF: Ministry of Agriculture, Forestry and Fisheries
- METI: Ministry of Economy, Trade and Industry
- MHLW: Ministry of Health, Labour and Welfare
- MS: main study
- no.: number
- ns: not significant at p ≤ 0.01 or p ≤ 0.05
- OECD: Organization for Economic Co-Operation and Development
- p.a.: post application
- Pl: Principal Investigator
- p.m.: post meridiem
- p.o.: per oral
- QAU: Quality Assurance Unit
- rel.: relative
- RP: recovery period
- RS: recovery sacrifice
- SD: standard deviation
- SOP: Standard Operating Procedure
- t: t-value according to STUDENT's t-test or DUNNETT's test
- TD 0 / TW 0: pre-drug examination
- TD: test day
- TS: Test Site / terminal sacrifice
- TSQAU: Test Site Quality Assurance Unit
- TW: test week

### Non-invasive telemetry

- ECG: electrocardiogram
- HR: heart rate
- PQ interval: beginning of atrioventricular and ventricular excitation
- QRS interval: deflections due to excitation of the ventricles
- QT interval: total electrocardiographic ventricular activity
- QTc value: QT interval corrected according to the formula of Bazett

### Haematology

- aPTT: activated partial thromboplastin time
- Baso: basophilic granulocytes
- Eos: eosinophilic granulocytes
- ESR: erythrocyte sedimentation
- HCT: haematocrit
- HGB: haemoglobin
- LUC: large unstained cells
- Lym: lymphocytes
- MCH: mean corpuscular haemoglobin
- MCHC: mean corpuscular haemoglobin concentration
- MCV: mean corpuscular volume
- Mono: monocytes
- Neut: neutrophilic granulocytes
- PLT: platelets
- RBC: erythrocytes (red blood cells)
- Reti: reticulocytes
- TPT: thromboplastin time
- WBC: leucocytes (white blood cells)

### Clinical biochemistry

- ALAT: alanine aminotransferase
- Alb.: albumin
- aP: alkaline phosphatase
- ASAT: aspartate aminotransferase
- BUN: blood urea nitrogen
- Creat.: creatinine
- Gamma-GT: gamma-glutamyltransferase
- Glob.: globulin
- Pot: potassium
- Sod: sodium
- TP: total protein

### Urinalysis

- A: crystalluria
- B: organisms (i.e. worm eggs, bacteria)
- C: further constituents (i.e. sperm, casts)
- E: epithelial cells
- ery: erythrocytes count
- L: leucocytes
- LC: lemon-coloured
- SC: straw-coloured
- R: erythrocytes
- neg.: negative/none found in any field examined
- norm.: normal
- pos.: positive
- +: 'small amount' of analyte/few in some fields examined
- ++: 'moderate amount' of analyte/few in all fields examined
- +++: 'large amount' of analyte/many in all fields examined

### Weights and Measures

- °C: degree Celsius
- µmol: micromol
- µL: microlitre
- Bq: Becquerel
- fL: femtolitre
- g: gram
- h: hour(s)
- kg: kilogram
- L: litre
- m: metre
- mg: milligram
- MJ: megajoule
- mL: millilitre
- mm: millimetre
- mmol: millimol
- pg: picogram
- ppb: parts per billion
- U: Unit

### Measuring units

body weight in kg
food intake in g/kg b.w./day
absolute organ weights in g
relative organ weights in g/kg b.w.

### Pharmacokinetic spectrum / PK data

| **Abbreviations** | **Full Descriptions** |
|---|---|
| AUC₀₋₂₄ | Area under the analyte vs. time concentration curve from time of administration up to 24h post dosing |
| AUCₗₐₛₜ | Area under the analyte vs. time concentration curve from time of administration up to the time of the last quantifiable concentration post dosing |
| AUC_{0-∞} | Area under the analyte vs. time concentration curve from time of administration up to infinity |
| AUC_{0-∞}/D | Dose normalized area under the analyte vs. time concentration curve from time of administration up to infinity |
| AUC_{%Extrap} | The percentage of area that is extrapolated in the estimation of AUC_{0-∞} |
| CL/F | Apparent total plasma clearance |
| Cₗₐₛₜ | Last quantifiable concentration |
| Cₘₐₓ | Maximal observed analyte concentration |
| Cₘₐₓ_D | Dose normalized maximal observed analyte concentration |
| Rsq | Correlation coefficient |
| SD | Standard deviation |
| SE | Standard error |
| T_{1/2z} | The apparent terminal elimination half-life |
| T_{lag} | Time prior to the first measurable (non-zero) concentration. |
| Tₗₐₛₜ | Time of the last quantifiable concentration |
| Tₘₐₓ | Time to reach the maximal observed analyte concentration |
| V_{z}/F | Apparent distribution volume |

Unless otherwise specified, the percentages in the following tests and examples are given as percentages by weight; parts are parts by weight. Solvent ratios, dilution ratios and concentration readings of liquid/liquid solutions refer in each case to the volume. The notation "w/v" means "weight/volume". Thus for example "10% w/v": means 100 mL solution or suspension contains 10 g substance.

### Examples

### Example 1 - Synthesis process for obtaining 4-[5-[3-Chloro-5-(trifluoromethoxy) phenyl]-1-(pyridin-3-yl)-1H-pyrazol-3-yl]-5,6-dihydro-7H-pyrrolo[3,4-d]pyrimidin-7-one (i.e. compound A)

### Step 1) Synthesis of a compound 2a out of compound 1a

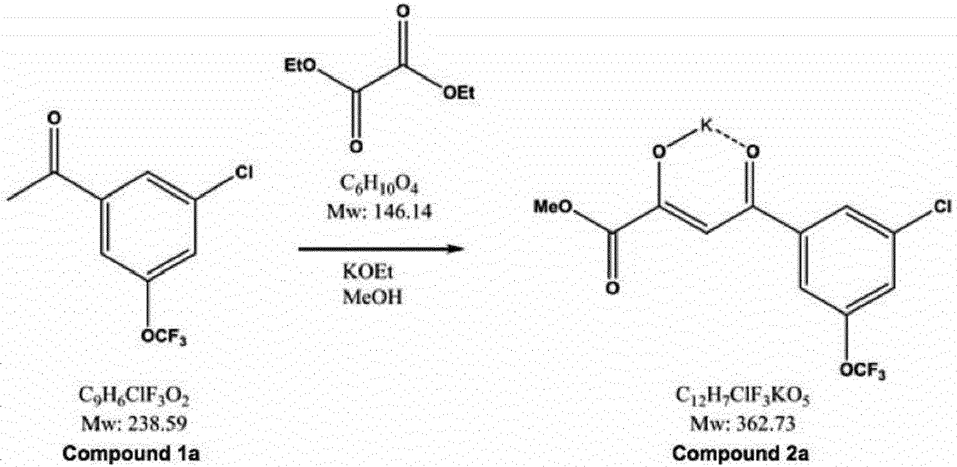

In the following, all volumes, weights and molar ratios are given relative to starting compound 1a. RX = 1000 L glass-lined reactor. FD = 500 liter Hastelloy-C filter dryer.

Process description for exemplary step 1, conversion of compound 1a to compound 2a (Intake 48.40 kg compound 1 a; Vmax approx. 950 L; 1 batch).
1. Inert reactor.
2. Charge diethyl oxalate (1.2 equiv.) to reactor.
3. Charge methanol (4.0 rel. volumes) to reactor.
4. Stir reactor contents until a homogeneous solution is obtained.
5. Harvest the obtained solution in new and clean drums labeled as "Diethyl oxalate solution".
6. Charge methanol (1.0 rel. volumes) to reactor.
7. Line rinse: discharge contents of the reactor to drum(s) labeled as "Diethyl oxalate solution".
8. Charge reactor with compound 1 a (1.0 equiv.).
9. Charge reactor with methanol (9.0 rel. volumes).
10. Start stirring the contents of the reactor until a homogeneous solution is obtained.
11. Adjust the temperature of the contents of the reactor to 20 ± 5°C.
12. Start dosing potassium ethoxide (1.3 equiv.) as 24 %-wt solution in ethanol in a dropwise fashion to the reactor while keeping the temperature of the reactor contents below 30°C.
13. Rinse the dosing bulb with ethanol (0.5 rel. volume).
14. Stir the contents of the reactor at 25 ± 5 °C for 30 ± 15 minutes.
15. Adjust the temperature of the contents of the reactor to 45 ± 5°C.
16. Dose over a period of 1.5 ± 0.5 h the contents of the drum(s) labeled as "Diethyl oxalate solution" to the reactor while keeping the contents of the reactor at 45 ± 5°C.
17. Line rinse: charge the reactor via the dosing bulb with ethanol (0.5 rel. volume).
18. Stir the contents of the reactor for a period of at least 2 h, but no longer than 18h at 45 ± 5°C.
19. IPC-1: conversion by HPLC.
20. Adjust the temperature of the reactor contents to 15 ± 5°C.
21. Stir the contents of the reactor at 15 ± 5°C for at least 2 h (hold point).
22. Transfer the contents of the reactor to the filter-dryer. Remove ML via filtration to a new and clean drum(s) labeled as "compound 2a".
23. Wash: charge filter dryer via the reactor with ethanol (2.0 rel. volumes). Remove washing liquid via filtration.
24. Charge the reactor with ethanol (2 rel. volumes).
25. Charge the reactor with n-heptane (2 rel. volumes).
26. Start stirring the contents of the reactor.
27. Wash: discharge the contents of the reactor via the filter dryer to the drum(s) of step 22; i.e. the drums labeled with "compound 2a".
28. Wash: charge the filter dryer via the reactor with n-heptane (2 rel. volumes). Remove washing liquid via filtration to the drum(s) of step 27; i.e. the drums labeled with "compound 2a".
29. Dry the filter cake under vacuum for at least 8h. (Hold point).
30. Harvest compound 2a into anti-static PE bag(s). Store the bag(s) in suitable container(s).
31. IPC-2: Release testing.
32. End of batch cleaning: rinse setup using potable water and methanol until visually clean. (Expected yield: 80 - 90%)

### Step 2) - Synthesis of a compound 6a out of compound 2a

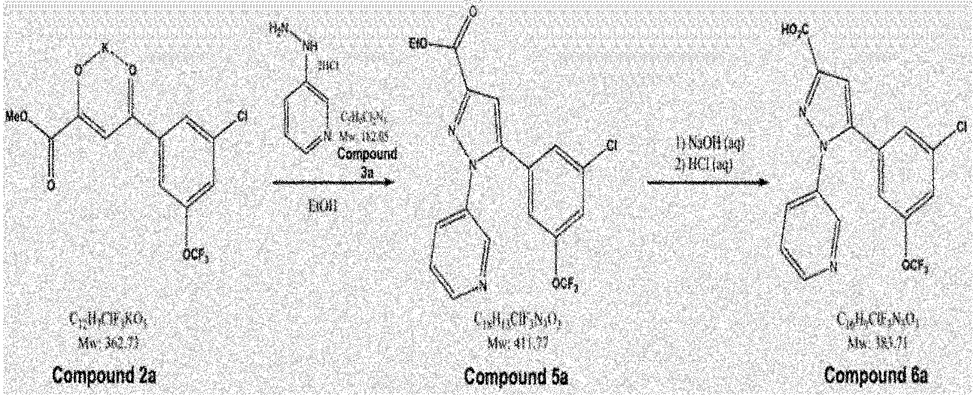

In the following, all volumes, weights and molar ratios are given relative to the dry intermediate compound 2a. RX = 1000 L glass-lined reactor. FD = 500 liter Hastelloy-C filter dryer, F60A = 60 L Hastelloy filter.

Process description for exemplary step 2, conversion of compound 2a to compound 6a (Intake 73.2 kg; Vmax approx. 1200L; 1 batch).
1. Inert reactor.
2. Charge dry intermediate compound 2a to reactor.
3. Inert reactor.
4. Charge ethanol (9 rel. volumes) to reactor of step 3.
5. Start stirring reactor contents.
6. Charge compound 3a (1.02 equiv.) to reactor as a solid.
7. Inert reactor.
8. Adjust temperature to 79 ± 5°C over a period of 1 h.
9. Stir the reaction mixture under reflux conditions for 2 to 4 hours.
10. IPC-1: conversion.
11. Adjust temperature to 25 ± 10 °C.
12. Charge purified water to reactor (8.0 rel. volumes).
13. Adjust temperature to 0 ± 5 °C.
14. Dose sodium hydroxide (2.0 equivalents) as 33 %-wt solution in water, while keeping the temperature of the reactor contents at 0 ± 5°C.
15. Rinse the dosing bulb with purified water (0.5 rel. volumes).
16. Stir at 0 ± 5° C for 45 ± 15 minutes.
17. Adjust temperature to 25 ± 5 °C.
18. Stir the reactor contents at 25 ± 5 °C for at least 4h, but no longer than 24h.
19. Adjust temperature of reactor contents to 40 - 45 °C.
20. Stir at 40 - 45 °C for 1 ± 0.5 h.
21. IPC-2: conversion by HPLC.
22. Transfer reaction mixture via a filter (coated with suitable amount of filter aid type Dicalite 478) and a 1 µm inline filter to new and clean drum(s) labelled as "compound 6a".
23. Charge reactor with purified water (0.5 rel. volumes).
24. Discharge reactor contents via filter and inline filter to the drum(s) labelled as "compound 6a".
25. Intermediate cleaning: rinse setup with purified water until visually clean.
26. Charge the contents of the drum(s) labelled as "compound 6a" to the reactor.
27. Line rinse using purified water (0.5 rel. volumes).
28. Adjust temperature of the reactor contents to 45 ± 5 °C.
29. Charge dosing bulb with 30 % hydrochloric acid solution.
30. Dose the hydrochloric acid solution in a slow to dropwise fashion until the pH of the reaction mixture is 3 ± 0.2. (Remainder of acid solution will be discarded as waste).
31. Stir the contents of the reactor at 40 - 45 °C for 30 ± 15 min.
32. Adjust the temperature of the reactor contents to 15 ± 5 °C.
33. Stir the contents of the reactor at 15 ± 5 °C for at least 4h, but do not exceed 18h.
34. Check, if pH is still 3.0 ± 0.2.
35. Discharge the contents of the reactor to the filter dryer, remove ML by filtration.
36. Charge filter via reactor with purified water (1.0 rel. volume). Discharge WL by filtration.
37. Charge reactor with purified water (1.0 rel. volume).
38. Charge reactor wit ethanol (1.0 rel. volume).
39. Stir the contents of the reactor until a homogeneous mixture is obtained.
40. Discharge the contents of the reactor via the filter dryer to the appropriate waste drum(s).
41. Charge reactor with purified water (1.0 rel. volume).
42. Charge reactor wit ethanol (1.0 rel. volume).
43. Stir the contents of the reactor until a homogeneous mixture is obtained.
44. Discharge the contents of the reactor via the filter dryer to the appropriate waste drum(s).
45. Dry the filter cake under vacuum for at least 8h. (Hold point).
46. Harvest compound 6a into an anti-static PE bag. Store the bag(s) in suitable container(s).
47. Perform release testing (IPC-3).
48. End of batch cleaning: rinse setup using potable water and methanol until visually clean.
49. Precondition the setup using ethanol. (Expected yield: 80-85%).

### Step 3) - Synthesis of compound 8a out of compound 6a

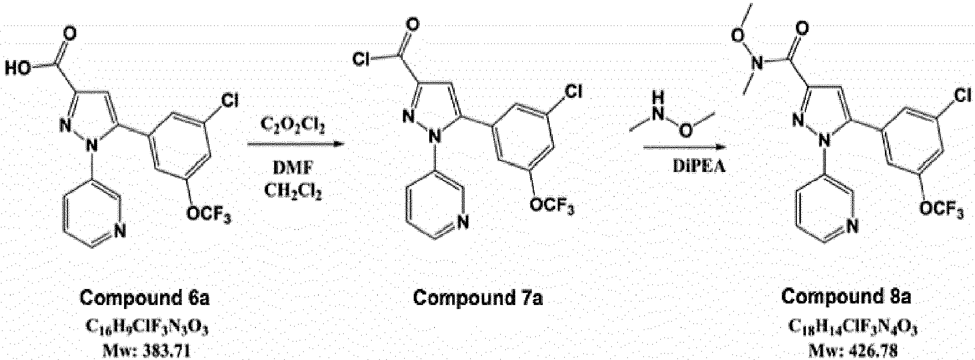

In the following, all volumes, weights and molar ratios are given relative to the intake of compound 6a. RX = 1000 L glass-lined reactor. FD = 500 liter Hastelloy-C filter dryer.

Process description for exemplary step 3, conversion of compound 6a to compound 8a (Intake 57.4 kg; Vmax approx. 950L; 1 batch).
1. Inert reactor.
2. Charge compound 6a to reactor as a solid.
3. Inert reactor.
4. Charge reactor with dichloromethane (9.0 rel. volumes).
5. Start stirring the reactor contents at medium speed.
6. IPC-1: Determine moisture levels of reaction mixture by KF-titration.
7. Adjust temperature to 15-20 °C.
8. Charge reactor via ball valve with N,N-dimethylformamide (0.15 equiv.).
9. Charge dosing bulb with oxalyl chloride (1.09 equiv.).
10. Dose oxalyl chloride in a slow to dropwise fashion while keeping the temperature at 20 ± 5 °C (slow dosing: foaming of reaction mixture).
11. Line rinse: charge reactor via dosing bulb with dichloromethane (1.0 rel. volume).
12. Post stir the reaction mixture at 20 ± 5 °C for 1.5 ± 0.5 h.
13. IPC-2: conversion by HPLC.
14. Adjust temperature to 30 ± 5 °C.
15. Distill off under reduced pressure a total of 4 relative volumes of solvent.
16. Break vacuum and charge dichloromethane (4.0 rel. volumes) to reactor.
17. Adjust temperature of the reactor contents to 0 ± 5 °C.
18. Charge N,O-dimethylhydroxylamine-HCl (1.20 equiv.) as a solid to the reactor.
19. Charge the dosing bulb with N,N-diisopropylethylamine (3.30 equiv.).
20. Dose the contents of the dosing bulb to the reaction mixture over a period of 45 ± 15 min while keeping the temperature at 0 ± 5 °C (temperature is leading in this step).
21. Stir the reaction mixture at 0 ± 5 °C for 1.5 ± 0.5 h.
22. IPC-3: conversion by HPLC.
23. Adjust temperature of reactor contents to 15 ± 5 °C.
24. Charge a solution of potassium carbonate (4.0 rel. volume) in water to reactor.
25. Stir biphasic mixture at high speed for 30 ± 15 min at 15 ± 5 °C.
26. Stop stirring the contents of the reactor and allow the phases to separate.
27. Check pH of aqueous phase.
28. Discharge organic phase into a new clean drum(s) labelled as "compound 8a".
29. Discharge aqueous phase as waste.
30. Charge product solution labelled as "compound 8a" to reactor.
31. Charge reactor with purified water (4.0 rel. volume).
32. Stir biphasic mixture at high speed for 15 ± 10 min at 20 ± 5 °C.
33. Stop stirring the contents of the reactor and allow the phases to separate.
34. Discharge organic phase via a filter containing a filter aid plug (Davisil LC60A 70-200 micron) and an inline filter (1µm PTFE) into a new clean drum(s) labelled as "compound 8a filtered".
35. Discharge aqueous phase as waste.
36. Charge reactor with dichloromethane (1.0 rel. volume).
37. Line rinse: discharge the contents of the reactor via the filter in inline filter to the drum(s) labelled as "compound 8a filtered".
38. Intermediate cleaning: rinse reactor setup with methanol until visually clean.
39. Precondition the reactor setup with dichloromethane.
40. Charge the contents of the drum(s) labelled as "compound 8a filtered" to the reactor.
41. Adjust temperature to 30 ± 5 °C.
42. Reduce the volume of the reactor contents to in total 2 rel. volumes by distilling of solvent (10 rel. volumes) under reduced pressure keeping the temperature of the reactor contents at 30 ± 5 °C.
43. Charge the reactor with diisopropyl ether (10.0 rel. volume).
44. Reduce the volume of the reactor contents to in total 2 rel. volumes by distilling of solvent (10 rel. volumes) under reduced pressure keeping the temperature of the reactor contents at 30 ± 5 °C.
45. Charge the reactor with diisopropyl ether (4.0 rel. volume).
46. Adjust the temperature of the reactor contents to 15 ± 5 °C.
47. Stir the reactor contents at 15 ± 5 °C for at least 4h (hold point).
48. Adjust the temperature to 0 ± 5 °C.
49. Stir the reactor contents at 0 ± 5 °C for at least 4h (hold point).
50. Discharge the contents of the reactor to the filter dryer. Remove ML by filtration.
51. Wash: Charge filter dryer via reactor with diisopropyl ether (1.0 rel. volume). Discharge washing liquid by filtration.
52. Wash: Charge filter dryer via reactor with diisopropyl ether (1.0 rel. volume). Discharge washing liquid by filtration.
53. Dry the filter cake under vacuum for at least 8h (hold point).
54. Harvest compound 8a into an anti-static PE bag.
55. IPC-4: release testing.
56. End of batch cleaning: rinse reactor with methanol until visually clean.
57. Precondition the reactor with either dichloromethane or THF. (Expected yield: 85-90%).

### Step 4) - Synthesis of compound 9a out of compound 8a

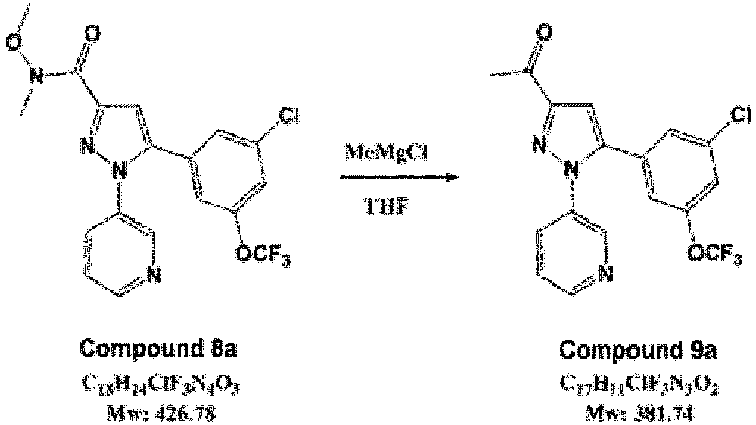

In the following, all volumes, weights and molar ratios are given relative to the intake of compound 8a. RX = 45 L glass-lined reactor.

Process description for exemplary step 4, conversion of compound 8a to compound 9a (Intake 23 kg; Vmax approx. 350 L; 1 batch).
1. Inert reactor.
2. Charge compound 8a to reactor as a solid.
3. Inert reactor.
4. Charge reactor with tetrahydrofuran (8.0 rel. volumes).
5. Start stirring the reactor contents at medium speed.
6. Adjust temperature to 0 ± 5 °C.
7. Dose methylmagnesiumchloride (1.20 equiv.) as 22 %-wt solution in tetrahydrofuran over a period of 45 ± 15 minutes while keeping the temperature of the reaction mixture at 0 ± 5 °C.
8. Rinse the dosing system with a minimum amount of tetrahydrofuran.
9. Stir the reaction mixture at 0 ± 5 °C for 1.5 ± 0.5 h.
10. Adjust temperature of the reactor contents to 20 ± 5 °C.
11. Stir the reaction mixture at 20 ± 5 °C for 1.0 ± 0.5 h.
12. IPC-1: Conversion by HPLC.
13. Adjust the temperature of the reaction mixture to 10 ± 5 °C.
14. Dose ammonium chloride to the reaction mixture (2.0 rel. volumes) as a solution in water over a period of about 15 minutes while keeping the temperature at 10 ± 5 °C (quench exothermic).
15. Post stir the reaction mixture at 10 ± 5 °C at medium to high speed for a period of 20 ± 10 minutes.
16. Stop stirring and allow the phases to separate.
17. Discard the aqueous phase as waste.
18. Charge a 20 % sodium chloride solution in water (2.0 rel. volumes) to reactor.
19. Charge purified water (2.0 rel. volumes) to reactor.
20. Stir the reactor contents at an appropriate speed for 15 ± 10 min at 15 ± 5 °C.
21. Stop stirring and allow the phases to separate.
22. Discard the aqueous phase as waste.
23. Harvest the product solution via an inline filter (1 µm PTFE) to new drum(s) labelled as "compound 9a intermediate".
24. Charge reactor with tetrahydrofuran (0.5 rel. volume).
25. Line rinse: discharge the contents of the reactor via the inline filter to the drum(s) labelled as "compound 9a intermediate".
26. Intermediate cleaning: rinse the reactor setup until visually clean. Use diluted hydrochloric acid solutions, purified water, and methanol.
27. Precondition the setup using tetrahydrofuran.
28. Charge reactor via the dosing bulb with the contents of the drum(s) "compound 9a intermediate".
29. Rinse intake line using tetrahydrofuran (0.5 rel. volume).
30. Start stirring the contents of the reactor and adjust the temperature to 40 ± 5 °C.
31. Reduce the total volume of the reactor contents to 4 relative volumes by distilling of 7 relative volumes of solvent under reduced pressure while keeping the temperature at 40 ± 5 °C.
32. Charge reactor with tetrahydrofuran (6.0 rel. volumes).
33. IPC-2: residual moisture by KF-titration.
34. Adjust temperature to 15 ± 5 °C.
35. Optional: harvest product solution to new and clean drum(s) labelled as "compound 9a".
36. Optional: charge reactor with tetrahydrofuran (0.5 rel. volume).
37. Optional: line rinse: discharge the contents of the reactor to the drum(s) labelled as "compound 9a".
38. IPC-3: release testing. (Expected yield based on HPLC-assay: 85-90%).

### Step 5) - Synthesis of compound 10a out of compound 9a

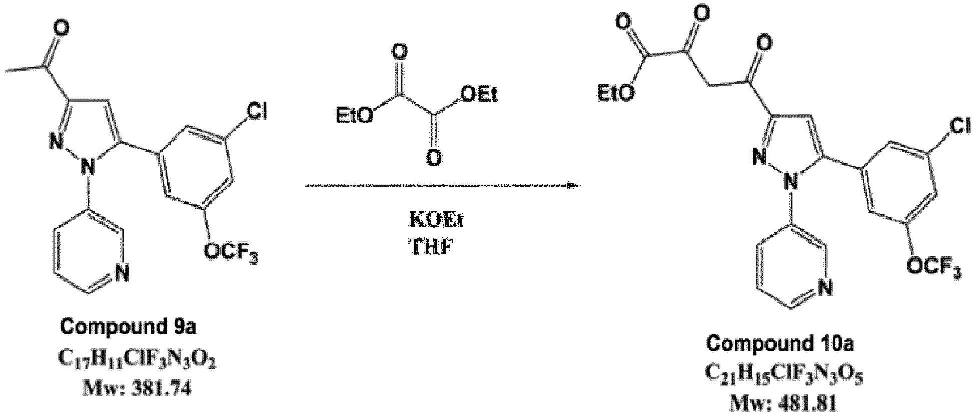

In the following, all volumes, weights and molar ratios are given relative to the intake of compound 9a. RX = 450 L glass-lined reactor. FX = 200 I glass lined filter.

Process description for exemplary step 5, conversion of compound 9a to compound 10a (Intake 22.1 kg; Vmax approx. 530L; 1 batch).
1. Optional: Inert reactor.
2. Optional: charge compound 9a.
3. Charge reactor with tetrahydrofuran (0.25 rel. volume).
4. Start stirring the reactor contents at medium to high speed.
5. Adjust the temperature of the reactor contents to 40 ± 5 °C.
6. Charge reactor wit diethyl oxalate (1.3 equiv.).
7. Line rinse: charge reactor with tetrahydrofuran (0.25 rel. volume).
8. Charge the dosing bulb with sodium ethoxide (1.5 equiv.) as 21 %-wt solution in ethanol.
9. Start dosing the contents of the dosing bulb over a period of 45 ± 15 min, while keeping the temperature at 40 ± 5 °C.
10. Line rinse: Charge the reactor via the dosing bulb with tetrahydrofuran (0.25 equiv.).
11. Stir the contents of the reactor at 40 ± 5 °C for a period of 1.5 ± 0.5 h.
12. IPC-1: conversion by HPLC.
13. Adjust temperature to 15 ± 5 °C.
14. Charge reactor with methyl-tert-butyl ether (5.0 rel. volume).
15. Charge reactor with purified water (2.0 rel. volume.).
16. Charge reactor via the dosing bulb with hydrochloric acid (1.2 equiv.) as 30 % solution in water.
17. Line rinse: charge reactor via the dosing bulb with purified water (1.0 rel. volume).
18. Stir the contents of the reactor at 15 ± 5 °C for 20 ± 10 min.
19. Stop stirring and allow the phases to separate.
20. Check pH of aqueous phase (expectation (pH ≤ 2).
21. Discharge the aqueous phase as waste.
22. Charge the reactor with purified water (2.0 rel. volume).
23. Stir the contents of the reactor at 15 ± 5 °C for 20 ± 10 min.
24. Stop stirring and allow the phases to separate.
25. Discard the aqueous phase as waste.
26. Charge the reactor with purified water (2.0 rel. volume).
27. Stir the contents of the reactor at 15 ± 5 °C for 20 ± 10 min.
28. Stop stirring and allow the phases to separate.
29. Discard the aqueous phase as waste.
30. Charge the reactor with methyl-tert-butyl Ether (1.0 rel. volume).
31. Discharge the contents of the reactor to new and clean drum(s) labelled as "compound 10a".
32. Intermediate cleaning: rinse the reactor setup with potable water and acetone until visually clean.
33. Precondition the setup with ethanol.
34. Charge the contents of the drum(s) labelled as "compound 10a" to reactor.
35. Line rinse: charge reactor with ethanol (0.5 rel. volume).
36. Start stirring and adjust the temperature of the reactor contents to 40 ± 5 °C.
37. Reduce the total volume of the reactor contents to 11 rel. volumes by distilling off 10 relative volumes of solvent under reduced pressure while keeping the temperature at 40 ± 5 °C.
38. Solvent switch: distil a total of 10 relative volumes of solvent under reduced pressure at 40 ± 5 °C while keeping the volume of the reactor contents constant by the addition of in total 10 rel. volumes of ethanol.
39. Adjust the temperature of reactor contents to 5 ± 5 °C.
40. Post stirr the reactor contents at 5 ± 5 °C for at least 2 hours.
41. Reduce stirring speed and release the contents of the reactor to the filter. Remove ML by filtration.
42. Charge the reactor with ethanol (2.0 rel. volumes).
43. Wash: release the contents of the reactor to the filter. Remove the WL by filtration.
44. Charge the reactor with ethanol (2.0 rel. volumes).
45. Wash: release the contents of the reactor to the filter. Remove the WL by filtration.
46. Dry the filter cake under vacuum for at least 8h (hold point).
47. Harvest compound 10a into an anti-static PE bag. Store the bag(s) in a suitable container.
48. IPC-2: release testing.
49. End of batch cleaning: rinse the setup with potable water and acetone until visually clean.
50. Pre-condition the reactors (Expected yield: 75-80%).

### Step 6) - Synthesis of compound 12a out of compound 10a

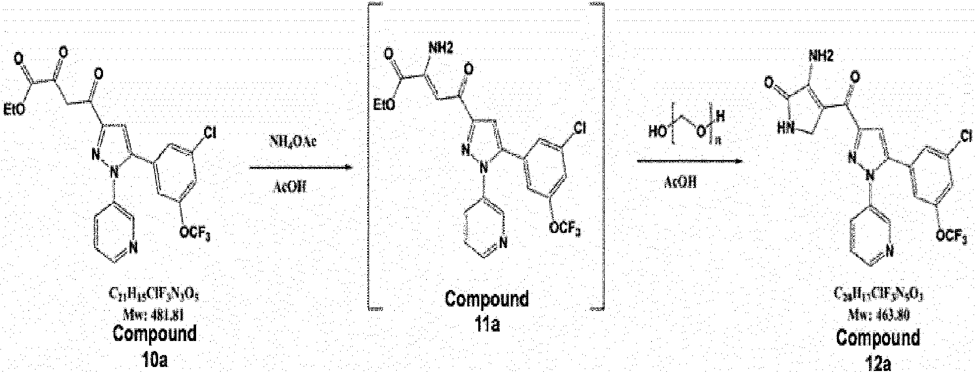

In the following, all volumes, weights and molar ratios are given relative to the intake of compound 10a. RX = 450 L glass-lined reactor.

Process description for exemplary step 6, conversion of compound 10a to compound 12a (Intake 22.5 kg; Vmax approx. 450L; 1 batch).
1. Inert reactor.
2. Charge dry intermediate compound 10a to the reactor as a solid.
3. Charge ammonium acetate (13.0 equiv.) to the reactor as a solid.
4. Inert reactor.
5. Charge glacial acetic acid (6.0 rel. volumes).
6. Start stirring the reactor contents at a low to moderate speed.
7. Adjust the temperature of the reactor contents to 45 ± 5 °C.
8. Stirr the contents of the reactor at 45 ± 5 °C for a period of 30 ± 15 min.
9. IPC-1: Conversion by HPLC.
10. Adjust temperature to 30 ± 5 °C.
11. Charge paraformaldehyde (2.0 equiv.) to the reactor as a solid.
12. Rinse dosing system with acetic acid (minimum amount required).
13. Inert reactor.
14. Stirr the contents of the reactor at 30 ± 5 °C for at least 6h, but do not exceed 18h.
15. IPC-2: conversion by HPLC.
16. Charge reactor with ethanol (10.0 rel. volumes).
17. Adjust the temperature of the reactor contents to 0 ± 5 °C.
18. Stirr the contents of the reactor for at least 2 h at 0 ± 5 °C (hold point).
19. Reduce the stirring speed and release the contents of the reactor to the filter. Remove the ML by filtration.
20. Charge the reactor with ethanol (2.0 rel. volumes).
21. Wash: discharge the contents of the reactor the filter. Remove the washing liquid by filtration.
22. Charge the reactor with ethanol (2.0 rel. volumes).
23. Wash: discharge the contents of the reactor to the filter. Remove the washing liquid by filtration.
24. Dry the filter cake under vacuum for at least 8 h (hold point).
25. IPC-3: volatiles by TGA.
26. Harvest compound 12a into an anti-static PE bag.
27. IPC-4: release testing. (Expected yield: 50-55%).

At the start of exemplary step 6) a very slow stirring speed needs to be applied due to the fact that ammonium acetate has a tendency to form large chunks of solids, which may damage the equipment. Dissolution of the reagent occurs upon heating. When full dissolution is obtained, the stirrer speed is increased to a medium speed. When full dissolution is observed the conversion from compound 10a towards compound 11 a is also nearly completed.

### Step 7) - Synthesis of final compound A out of compound 12a

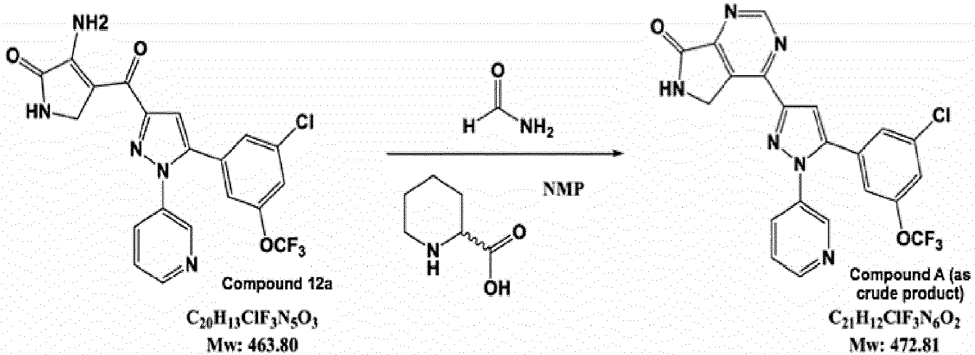

In the following, all volumes, weights and molar ratios are given relative to the intake of compound 12a. RX = FD = 200 L Hastelloy filter dryer.

Process description of exemplary step 7, conversion of compound 12a to compound A (Intake 10.6 kg; Vmax approx. 250L; 1 batch).
1. Inert reactor.
2. Charge dry intermediate compound 12a to the reactor as a solid.
3. Charge D,L-pipecolinic acid (5.0 equiv.) to the reactor as a solid.
4. Inert reactor.
5. Charge formamide (60 equiv.) to the reactor.
6. Charge N-methyl-2-pyrrolidone (5.0 rel. volumes) to the reactor.
7. Start stirring the reactor contents at a moderate speed.
8. Adjust the temperature of the reactor contents to 45 ± 5 °C.
9. Adjust temperature to 100 ± 10 °C.
10. Close the butterfly valve.
11. Adjust the temperature of the reactor contents to 140 ± 5 °C.
12. Post stir the reactor contents at 140 ± 5 °C for 2 ± 1 h.
13. Safety check: ensure the pressure is within the safe operating ranges of the reactor.
14. Continue stirring the reaction mixture at 140 ± 5 °C for at least an additional 10 h, but no longer than 24h.
15. Adjust the temperature to 90 ± 10 °C.
16. Open the butterfly valve.
17. IPC-1: conversion by HPLC.
18. Dose in a slow to dropwise fashion purified water (10.0 rel. volumes) to the reactor while keeping the temperature of the reaction mixture at 90 ± 10 °C.
19. Adjust the temperature to 15 ± 5 °C.
20. Stir the contents of the reactor for at least 2 h at 15 ± 5 °C (hold point).
21. Reduce the stirring speed and release the content of the reactor to the filter dryer. Remove the ML by filtration.
22. Charge the reactor with purified water (4.0 rel. volumes).
23. Release the contents of the reactor to the filter dryer. Remove the washing liquid by filtration.
24. Charge the reactor with purified water (4.0 rel. volumes).
25. Release the contents of the reactor to the filter dryer. Remove the washing liquid by filtration.
26. Charge the reactor with acetone (5.0 rel. volumes).
27. Adjust the temperature of the reactor contents to 50 ± 5 °C.
28. Discharge the contents of the reactor to the filter dryer. Remove the washing liquid by filtration.
29. Charge the reactor with acetone (5.0 rel. volumes).
30. Adjust the temperature of the reactor contents to 50 ± 5 °C.
31. Discharge the contents of the reactor to the filter dryer. Remove the washing liquid by filtration.
32. Dry the filter cake under vacuum for at least 8 h (hold point).
33. IPC-2: volatiles by TGA.
34. Harvest compound A as crude product into an anti-static PE bag.
35. IPC-3: release testing. (Expected yield: 75-80%).

### Step 7a) - Purification isolation and release testing of compound A as crude product

In the following, all volumes, weights and molar ratios are given relative to the intake of crude compound A. RX-1 = 450 L glass line reactor. RX-2 = 1000 L glass lined reactor. FD = 200 L Hastelloy filter dryer.

Process description for exemplary step 7a, purification of compound A (Intake 8.5 kg; Vmax approx. 250 L; 1 batch).
1. Inert reactor RX-1.
2. Charge reactor RX-1 with crude compound A.
3. Inert reactor RX-1.
4. Charge reactor RX-1 with glacial acetic acid (10.0 rel. volumes).
5. Start stirring the contents of the reactor RX-1 at moderate to high speed.
6. Adjust the contents of the reactor RX-1 to 50 ± 5 °C.
7. Stir the contents of the reactor RX-1 at 50 ± 5 °C for at least 15 min in order to obtain a solution.
8. Charge the dosing bulb with purified water (10.0 rel. volumes).
9. Dose the contents of the dosing bulb over a period of 45 ± 15 min to the reactor RX-1 while keeping the temperature at 50 ± 5 °C.
10. Adjust the temperature of the contents of RX-1 to 15 ± 5 °C.
11. Stir the contents of the reactor RX-1 for at least 2 h at 15 ± 5 °C (hold point).
12. Reduce the stirring speed and discharge the contents of the reactor RX-1 to the filter dryer. Remove the ML by filtration.
13. Charge the reactor RX-1 with purified water (2.0 rel. volumes).
14. Adjust the temperature of the contents of reactor RX-1 to 50 ± 5 °C.
15. Discharge the contents of the reactor RX-1 to the filter dryer. Remove the washing liquid by filtration.
16. Charge the reactor RX-1 with purified water (2.0 rel. volumes).
17. Adjust the temperature of the contents of reactor RX-1 to 50 ± 5 °C.
18. Discharge the contents of the reactor RX-1 to the filter dryer. Remove the washing liquid by filtration.
19. Charge the reactor RX-1 with methanol (2.0 rel. volumes).
20. Adjust the temperature of the contents of reactor RX-1 to 50 ± 5 °C.
21. Discharge the contents of the reactor RX-1 to the filter dryer. Remove the washing liquid by filtration.
22. Charge the reactor RX-1 with methanol (2.0 rel. volumes).
23. Adjust the temperature of the contents of reactor RX-1 to 50 ± 5 °C.
24. Discharge the contents of the reactor RX-1 to the filter dryer. Remove the washing liquid by filtration.
25. Dry the filter cake under vacuum for at least 8 h (hold point).
26. IPC-1: volatiles by TGA.
27. Charge reactor RX-1 with dichloromethane (20 rel. volumes).
28. Charge reactor RX-1 with methanol (5.0 rel. volumes).
29. Adjust temperature of the contents of reactor RX-1 to 40 ± 5 °C.
30. Transfer the contents of reactor RX-1 via the filter-dryer and inline filter (0.2 µm PTFE filter) to reactor RX-2.
31. Charge reactor RX-1 with dichloromethane (2.0 rel. volumes).
32. Charge reactor RX-1 with methanol (0.5 rel. volumes).
33. Adjust temperature of the contents of reactor RX-1 to 40 ± 5 °C.
34. Transfer the contents of reactor RX-1 via the filter-dryer and inline filter to reactor RX-2.
35. Start stirring the contents of reactor RX-2.
36. Adjust the temperature of the contents of reactor RX-2 to 40 ± 5 °C.
37. Reduce the total volume of the contents of reactor RX-2 to 7 relative volumes by distilling off 22 relative volumes of solvent by atmospheric distillation.
38. Solvent switch: distil a total of 5 relative volumes of solvent under atmospheric pressure while keeping the volume of the reactor contents of RX-2 constant by the addition of in total 5.0 rel. volumes of methanol.
39. Adjust the temperature of the reactor contents of reactor RX-2 to 15 ± 5 °C.
40. Post stir the contents of reactor RX-2 for at least 1 h at 15 ± 5 °C (hold point).
41. Intermediate cleaning: rinse the filter dryer with glacial acetic acid and methanol until visually clean and free of particles. Dry the filter dryer. Only continue after inspection and approval by a QA-officer.
42. Reduce the stirring speed and release the contents of reactor RX-2 to the filter dryer. Remove the ML by filtration.
43. Charge reactor RX-2 with methanol (2.0 rel. volumes).
44. Discharge the contents of reactor RX-2 to the filter dryer. Remove the washing liquid by filtration.
45. Start drying the filter cake under reduced pressure and elevated temperature (Tmax < 50 °C) for at least 48 h (hold point).
46. IPC-2: Volatiles by TGA; residual CH₂Cl₂ and MeOH by HS-GC-MS.
47. Harvest the product in PE-bag(s). Store the PE-bag in a second PE-bag. Store the bags in a suitable HDPE container.
48. IPC-3: final release testing. (Expected recovery: 90-95%).

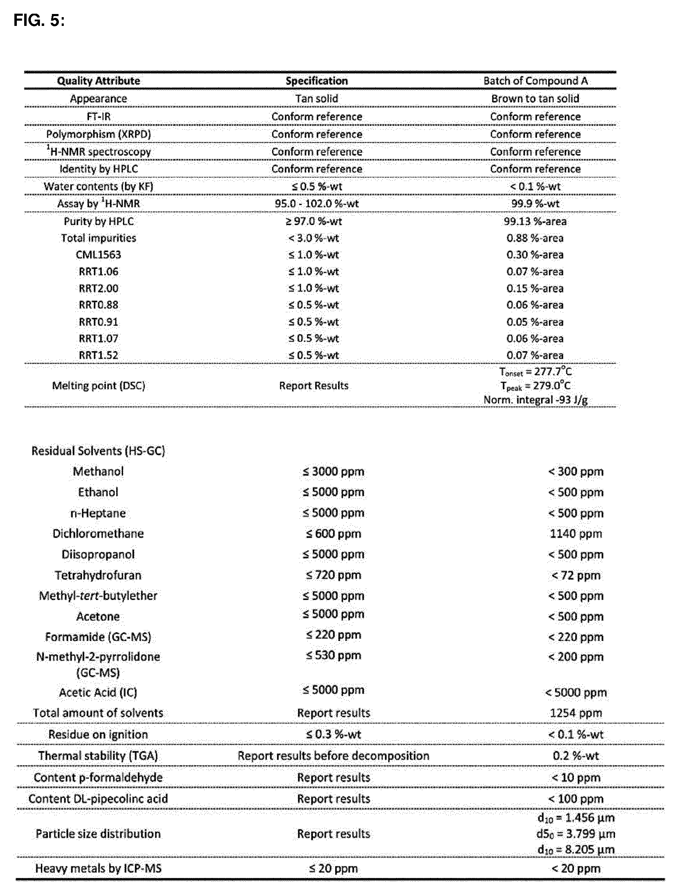

The analytical results for an exemplary batch synthesized by the above-described Example 1 can be dervided from FIG. 5 (i.e. final release testing).

The suitability of the compounds in accordance with the invention for use in methods for the treatment and/or prevention of cardiovascular diseases and / or comorbidities thereof can be also shown in the following assay systems:

The assay systems referred to below are to be regarded as examples and can be varied by one skilled in the art, by making modifications, or replaced by other suitable assay systems known to one skilled in the art for investigating the compounds in accordance with the invention against the background of retroviral diseases.

### Example 2 - In-vitro assays

### Example 2a - Effect on HCN4 currents recorded from transfected CHO

The following six compounds in accordance with the invention:
- Compound A: 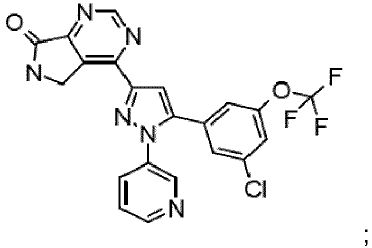 i.e. 4-[5-[3-Chloro-5-(trifluoromethoxy) phenyl]-1-(pyridin-3-yl)-1H-pyrazol-3-yl]-5,6-dihydro-7H-pyrrolo[3,4-d]pyrimidin-7-one,
- Compound B: 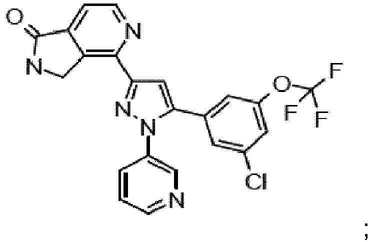 i.e. 4-[5-[3-Chloro-5-(trifluoromethoxy) phenyl]-1-(pyridin-3-yl)-1H-pyrazol-3-yl]-2,3-dihydro-1H-pyrrolo[3,4-c] pyridin-1-one,
- Compound C: 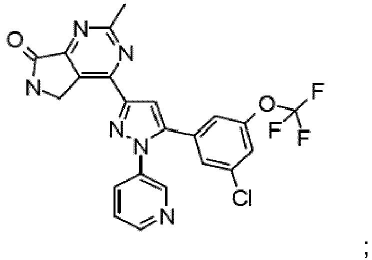 i.e. 4-[5-[3-Chloro-5-(trifluoromethoxy) phenyl]-1-(pyridin-3-yl)-1H-pyrazol-3-yl]-5,6-dihydro-2-methyl-7H-pyrrolo[3, 4-d] pyrimidin-7-one,
- Compound D: 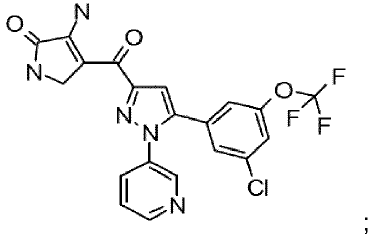 i.e. 3-Amino-4-[5-[3-chloro-5-(trifluoromethoxy) phenyl]-1-(pyridin-3-yl)-1H-pyrazol-3-yl-carbonyl]-1,5-dihydro-2H-pyrrol-2-one,
- Compound E: 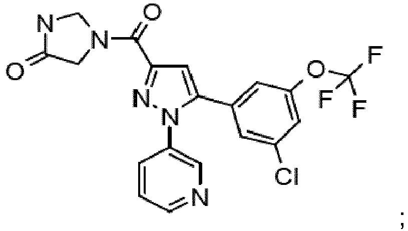 i.e. 1-[[5-[3-Chloro-5-(trifluoromethoxy) phenyl]-1-(pyridin-3-yl)-1H-pyrazol-3-yl]carbonyl]-imidazolidin-4-one,
- Compound F: 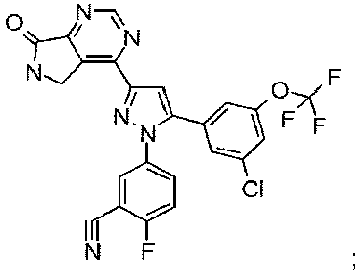 i.e. 4-[5-[3-Chloro-5-(trifluoromethoxy) phenyl]-1-(3-cyano-4-fluoro-phenyl)-1H-pyrazol-3-yl]-5,6-dihydro-7H-pyrrolo[3,4-d]pyrimidin-7-one,
were tested on their effects on human HCN4 currents recorded from transiently transfected CHO cells, in direct comparison to the reference compounds described below:
- Ivabradine: 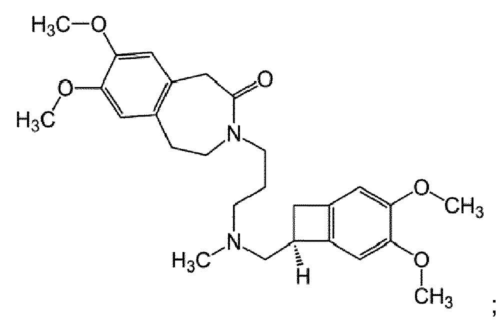 i.e. ((S)-3-{3-[([3,4-Dimethoxy-bicyclo [4.2.0]octa-1,3,5-trien-7-yl]methyl)methylamino]propyl}-1,3,4,5-tetrahydro-7,8-dimethoxy-2H-3-benzazepin-2-on),
- Compound X: 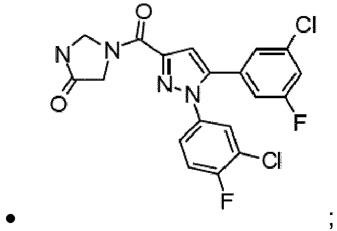 i.e. 1-[[1-(3-Chloro-4-fluorophenyl)-5-(3-chloro-5-fluorophenyl)-1 H-pyrazol-3-yl]carbonyl]-imidazolidin-4-one,
- Compound Y: 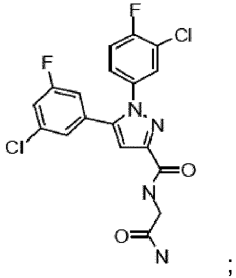 i.e. N-(2-Amino-2-oxoethyl)-1-(3-chloro-4-fluorophenyl)-5-(3-chloro-5-fluorophenyl)-1H-pyrazole-3-carboxamide.

Vehicle control (0.1% DMSO) and ZD7288 (100 µM), a suitable blocker of the HCN4 channel, were used as additional controls to assure accuracy of the method used.

### Methods

The whole-cell patch-clamp technique was used to demonstrate the effects of the six tested compounds of the invention in direct comparison to the above-mentioned reference compounds Ivabradine, and the compounds X and Y on HCN4 steady state currents. The pyrazole compounds X and Y as depicted above, and having structural similarities to the compounds of general formula (I) of the invention, were also tested to investigate whether the pure I*_{f}* current inhibitory effect of the compounds of the invention also extend to structurally closely related compounds.

Dependent on their solubility in the assay buffer, the compounds were tested first at one concentration of 10 µM (compound A, compound Y, compound X, compound B, compound E, compound F and ivabradine) or 1 µM for (compound C and D). If the current blocking of HCN4 at this concentration was higher than 50% the compounds were tested again at five concentrations of a half-logarithmic dilution series starting with either 10 µM or 1 µM as mentioned above (meaning 0.1 µM, 0.3 µM, 1 µM, 3 µM, and 10 µM or 0.01 µM, 0.03 µM 0.1 µM, 0.3 µM and 1 µM).

Due to the fact that compound B blocked the HCN4 currents at a concentration of 0.1 µM almost completely (see results below), a 100 times lower half-logarithmic dilution series with concentrations of 1 nM, 3 nM, 10 nM, 30 nM, and 100 nM were applied for this compound.

All compounds were supplied in DMSO solutions. The DMSO concentration of all test solutions was adapted to 0.1 % DMSO. All compounds were stored at thermostatic cabinet (18°C to 24°C) protected from light.

### Vehicle

< 0.1 % DMSO was used as vehicle (93.36 ± 2.49% relative steady state current, mean ± SEM of n=3 cells).

### Stock solution

The above described dose levels of the tested compounds were applied as follows. The tested compounds were weighed into a container on a calibrated precision balance. Vehicle was added to achieve a clear stock solution without precipitated test compounds. Dependending on compound solubility a stock solution of 10 mM or 1 mM (Compound C and D) was initially prepared (storage at 19 °C to 30 °C).

All compounds were admixed shortly before experimentation in their adequate formulation with DMSO. The dose formulations were maintained at room temperature (19 - 30 °C) when in use.

### Bath solution

The 1x bath solution was prepared by diluting 10x bath solution without glucose and 100x glucose solution with water at least every 7 days. Both stock solutions had been prepared prior to the experimental start and stored at 1°C to 9°C (10x bath solution) or -10 °C to -30 °C (100x glucose solution). When in use, the 1x bath solution was kept at room temperature (19 °C - 30 °C). When not in use, the 1x bath solution was stored at 1 °C to 9 °C. The final bath solution included the components: sodium chloride 137 mM; potassium chloride 4.0 mM; calcium chloride 1.8 mM; magnesium chloride 1.0 mM; HEPES 10 mM; D-Glucose 10 mM; pH (NaOH) 7.4.

### Pipette solution

The 1 x pipette solutions were thawed every day out of a frozen 1 x pipette solution, which had been prepared prior to the experimental start, aliquoted and stored at - 10 °C to - 30 °C. When in use, the 1x pipette solutions were filled into the patch-pipettes and kept at room temperature (19°C - 30°C). The 1x pipette solutions included the components outlined below: cesium fluoride 135 mM; sodium chloride 10 mM; calcium chloride 0.2 mM; HEPES 10 mM; EGTA 5 mM; cAMP 10 mM; pH (CsOH) 7.3.

### Test system / Equipment

Test system: CHO cells transiently expressing the HCN4 channel; NCBI Reference Sequence: NP005468.1 (HCN4 channel); Source CHO: ATCC, CCL-61 LGC Standards GmbH, Wesel, Germany were used.

Storage conditions: Maintenance of said CHO cells in culture or cryogenic storage; Passage number 23-32 was used for the tested compounds for its effects on the HCN4 channel expressed in CHO cells.

Equipment: Amplifier EPC-9/10, HEKA Electronics; Headstage: Preamplifier EPC-9/10, HEKA Electronics; Software: Patchmaster HEKA Electronics.

The test system was validated using the reference compound 100 µM ZD7288 (i.e. a selective HCN4 blocker), which effectively blocked the HCN4 steady state current (12.89 ± 1.19% relative steady state current, mean ± SEM of n=3 cells).

### Cell culture conditions

In general, CHO cells were passaged at a confluence of about 50 - 80 %. For electrophysiological measurements cells were seeded onto 35 mm sterile culture dishes containing 2 mL culture complete medium without antibiotics. Cells were cultivated at a density that enables the recording of single cells (without visible connections to other cells) to avoid electrical coupling.

Conditions: Standard Laboratory Conditions were applied. Cells were incubated at 37°C in a humidified atmosphere with 5 % CO₂ (rel. humidity about 95 %); Culture media: The cells were continuously maintained in and passaged in sterile culture flasks containing cell culture medium supplemented with 10 % fetal bovine serum and 1 % Penicillin/Streptomycin solution; Selection antibiotics: The complete medium as indicated above was supplemented with 100 µg/mL Zeocin.

### Experimental protocol

To achieve statistical significance the tested compounds were analyzed in sufficient numbers of *n*=3. As controls, three additional experiments (n=3) were conducted with bath solution containing 0.1 % DMSO (pure vehicle control). The same cells were treated with ZD7288; i.e. a suitable HCN4 channel blocker to assure accuracy of the method.

In 35 mm culture dishes CHO cells were seeded at a density allowing single cells to be recorded on the dish holder of the microscope and continuously perfused (at approximately 1 mL/min) with the bath solution described above. All solutions applied to cells including the pipette solution were maintained at room temperature (19 °C - 30 °C). After formation of a Gigaohm seal between the patch electrodes and individual cells (pipette resistance range: 2.0 MΩ - 7.0 MΩ; seal resistance range: > 1 GΩ) the cell membrane across the pipette tip was ruptured to assure electrical access to the cell interior (whole-cell patch-configuration). As soon as a stable seal was established, steady state currents were measured upon the hyperpolarization to -120 mV (2000 ms) from a holding potential of -40 mV. This voltage protocol (as shown in the scheme below) did run 5 times for control conditions and at least ten times for each test concentration. Pulse intervals were 20 s (0.05 Hz). If current amplitude was judged to be too low for measurement, another cell was recorded.

### Voltage protocol

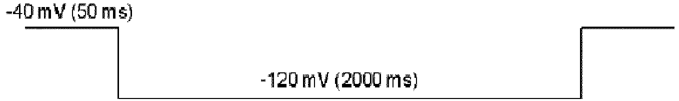

Once control recordings have been accomplished, cells were continuously perfused with bath solution containing the tested compounds as detailed above, 0.1 % DMSO or 100 µM ZD7288. During wash-in of the compounds, 0.1 % DMSO or 100 µM ZD7288 the voltage protocol indicated above did run continuously again at 20 s intervals until the steady-state level of block was reached.

### Data compilation and statistics

Values in Ampere (A) of the steady state current amplitudes were generated for each voltage step. The data obtained were printed for compilation and analysis. The recorded current amplitudes at the steady state level of current inhibition were compared to those from control conditions measured in the pre-treatment phase of the same cell. The amount of current block was calculated as percentage of control. To determine whether the observed current inhibition was due to a test compound interaction with the distinct ion channel HCN4 or due to current rundown, these residual currents were compared to those measured in vehicle treated cells. Data from at least 3 individual experiments were collected and the corresponding mean values and standard errors (SEM) calculated.

### Example 2a - Experimental results

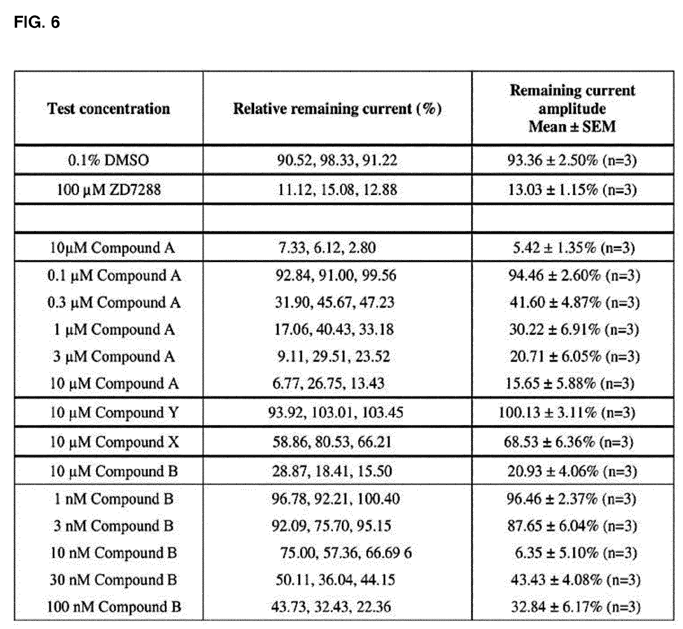

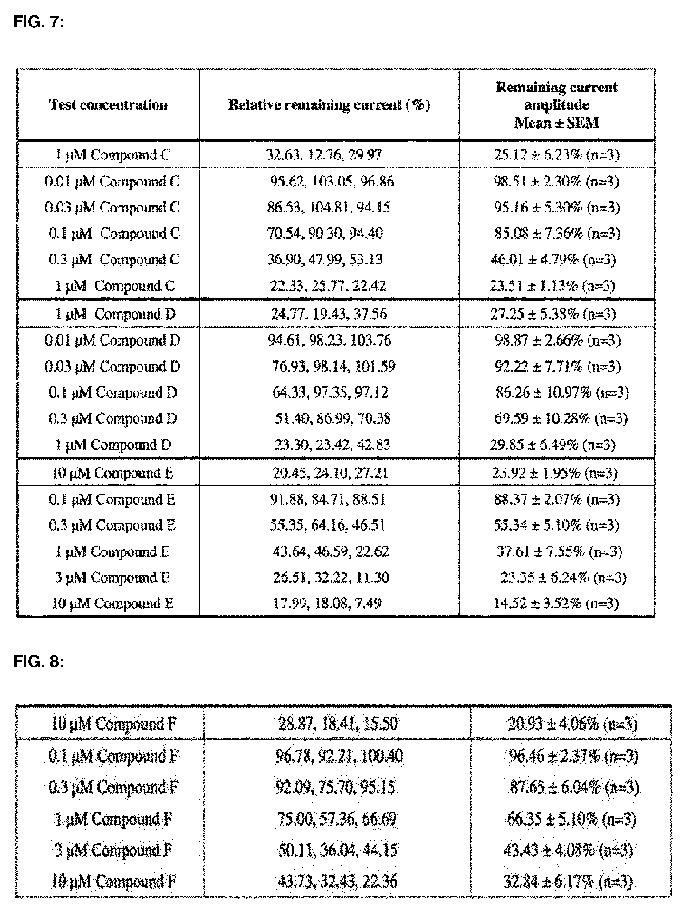

SigmaPlot 11 was used to calculate the means ± SEM of relative steady state current blockade for each concentration of the tested compounds. Analysis of Variance (ANOVA), multisample comparison (Dunnett) was conducted to test statistical significance of the test concentrations and the reference compounds versus vehicle (0.1 % DMSO in bath solution) using GraphPad Prism 5. Representative currents determined for the tested compounds in CHO cells are given in the Figures 6 to 9.

### Example 2b - IC50 determination of the tested compounds

The dose-response curves for the HCN4 channels treated with the tested compounds of Example 2a above were constructed with a sigmoidal two parameter equation: ${current}_{peak , relaive} = {/}_{1 + {\left(X/{IC}_{50}\right)}^{H}} 100 ,$ wherein X is the actual applied drug concentration, IC₅₀ is the concentration of drug at half maximal inhibition and H is the Hill coefficient. In biochemistry and pharmacology, the binding of a ligand (here the tested compounds) to a macromolecule (here the HCN4 channel) is often enhanced if there are already other ligands present on the same macromolecule (this is generally known as cooperative binding). The "Hill coefficient" provides a way to quantify this effect. It describes the fraction of the macromolecule saturated by ligand as a function of the ligand concentration; it is used in determining the degree of cooperativeness of the ligand binding to the enzyme or receptor.

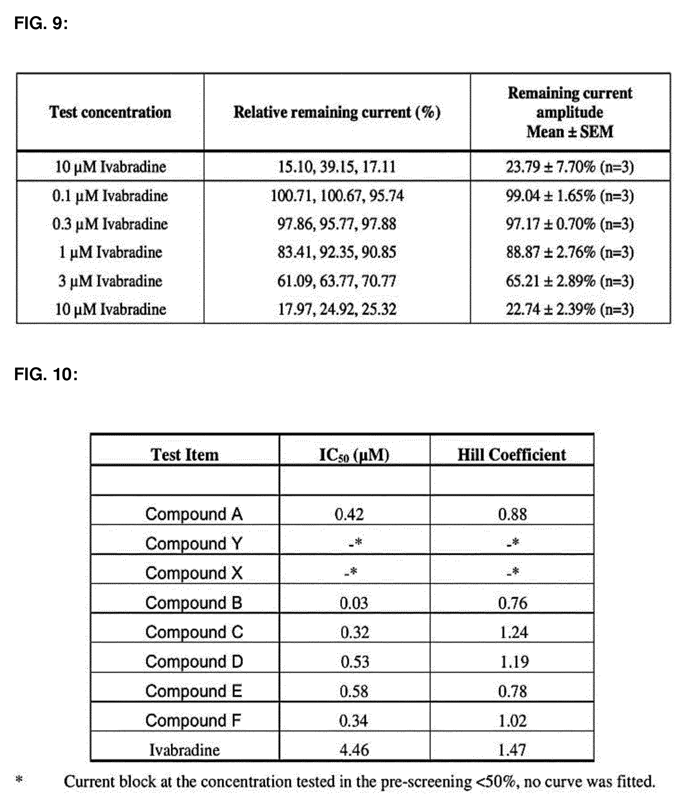

In Example 2b (see FIG. 10) the inhibitory effects of the six tested compounds in accordance with the invention (see above Example 2a for any details) on HCN4 currents are demonstrated versus ivabradine, and the reference compounds X and Y. Respective IC₅₀-values were determined for these tested compounds that blocked the HCN4 steady state currents by more than 50%. 0.1% DMSO was used as vehicle (93.36 ± 2.49% relative steady state current, mean ± SEM of n=3 cells). The test system was validated using the reference compound 100 µM ZD7288 (i.e. a selective HCN4 blocker), which effectively blocked the HCN4 steady state current (12.89 ± 1.19% relative steady state current, mean ± SEM of n=3 cells).

### Example 2b - Experimental results

As listed in FIG. 10, the compounds in accordance with the invention (i.e. compounds A, B, C, D, E and F) inhibited HCN4 currents with IC₅₀-values between 0.03 µM and 0.58 µM. Under the same test conditions ivabradine revealed an IC₅₀ of 4.46 µM, which demonstrates for ivabradine on target level being 7.7- to 149-fold less potent than the compounds in accordance with the invention (i.e. the tested compounds A, B, C, D, E and F). In contrast, the pyrazole compounds X and Y with certain structural similarities to the tested compounds of the invention were not identified as inhibitors of the HCN4 current.

### Example 3 - Compound A: Effect on Naᵥ1.5, Caᵥ1.2, Kᵥ1.5, and HCN4 currents recorded from stably transfected CHO cells

### Compound A

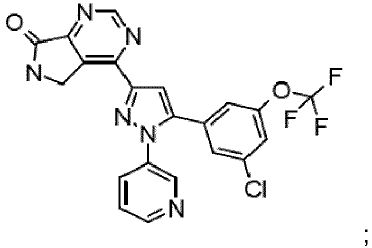

i.e. 4-[5-[3-Chloro-5-(trifluoromethoxy) phenyl]-1-(pyridin-3-yl)-1H-pyrazol-3-yl]-5,6-dihydro-7H-pyrrolo[3,4-d]pyrimidin-7-one,
was tested for its effects on human Naᵥ1.5, Caᵥ1.2, Kᵥ1.5, and HCN4 currents recorded from stably transfected CHO cells. The effect of four concentrations of this compound on these currents was investigated.

### Method

The whole-cell patch-clamp technique was used to investigate the effect of Compound A on the currents of Naᵥ1.5, Kᵥ1.5, Caᵥ1.2 and HCN4 channels, expressed in CHO cells (ATCC, CCL-61). Compound A was tested at concentrations of 200 nM, 600 nM, 2000 nM and 6000 nM. For all ion channels 0.1% DMSO was used as vehicle and ZD7288 as suitable blockers were applied to assure accuracy of the method. The selection antibiotics can be dervied from the Table below:

| **Ion channel** | **Host cell** | **Selection antibiotics** |
|---|---|---|
| Naᵥ1.5; | CHO | Geneticin, Puromycin |
| Caᵥ1.2 | CHO | Hygromycin, Geneticin, and Zeocin |
| Kᵥ1.5 | CHO | Hygromycin |
| HCN4 | CHO | Hygromycin, Geneticin |

For all test systems the following conditions were applied:
Equipment: Amplifier EPC-9/10, HEKA Electronics; Headstage: Preamplifier EPC-9/10, HEKA Electronics; Software: PatchMaster, HEKA Electronics.

### Cell culture

In general, CHO cells were passaged at a confluence of about 50 - 80 %. For the electrophysiological measurements, cells were seeded onto 35 mm sterile culture dishes containing 2 mL culture complete medium without antibiotics. Cells were cultivated at a density that enables the recording of single cells (without visible connections to other cells) to avoid electrical coupling.

Conditions: Standard Laboratory Conditions. Cells were incubated at 37°C in a humidified atmosphere with 5 % CO₂ (rel. humidity about 95 %). Culture media: The cells were continuously maintained in and passaged sterile culture flasks containing cell culture medium supplemented with 10% fetal bovine serum and 1.0 % Penicillin/Streptomycin solution. Medium for CHO: F12 (HAM) with L-Glutamine Selection antibiotics: The complete medium as indicated above is supplemented with antibiotics as given in the Table above.

### Tested compound A

The above indicated compound A was tested. Identity: Empirical Formula C21H12ClF3N6O2; Appearance: light brown solid; Solubility: soluble in water; Molecular weight: 472.81 g/mol; Purity (HPLC): 98.37%; Purity (NMR): 92%; Total correction factor A factor of 1.087 was applied (100/92 = 1.087); Storage conditions: thermostatic cabinet (18°C to 24°C), tightly closed container.

### Vehicle

Identity DMSO; Physical form: liquid at room temperature; Source: supplied by Sigma; Purity: 100.0%; Composition: C2H6OS; Molecular weight: 78.13 g/mol; Storage conditions: room temperature (19°C - 30°C).

### Stock solution

Dose levels were as indicated below. Compound A was weighed into a container on a calibrated precision balance and vehicle added to achieve a stock concentration of 6.0 mM (storage at 19°C to 30°C).

The compound stock solutions were stored at 19°C to 30°C (room temperature). Test solutions were prepared freshly every day. Test solutions were prepared shortly before experimentation. During this period, there were no observations indicating any instability of test items dissolved in bath solution.

### Compound A concentrations

Concentrations of 200 nM, 600 nM, 2000 nM and 6000 nM of Compound A were tested. They were diluted from the stock solution using bath solution shortly prior to the electrophysiological experiments and kept at room temperature (19°C to 30°C) when in use. The vehicle concentration was adjusted in all dose formulations to 0.1 % DMSO. Frequency of preparation: daily. Storage of dose formulation: On the day of experimentation dose formulations were maintained at room temperature (19 °C - 30 °C) when in use.

### Bath solution

The 1x bath solution was prepared by diluting 10x bath solution without glucose and 100x glucose solution with water at least every 7 days. Both stock solutions have been prepared prior to the experimental start of the present study and were stored at 1°C to 9°C (10x bath solution) or -10°C to -30°C (100x Glucose solution). When in use, the 1x bath solution was kept at room temperature (19°C - 30°C). When not in use, the 1 x bath solutions were stored at 1°C to 9°C. To analyze the currents of ion channels Naᵥ1.5, Kᵥ1.5, and HCN4, the final bath solutions included the components: NaCl 137 mM; KCI 4 mM; CaCl₂ 1.8 mM; MgCl₂ 1 mM; HEPES 10 mM; D-Glucose 10 mM; pH (NaOH) 7.4.

The following 1x bath solution was kept at room temperature (19°C - 30°C) when in use. When not in use, the 1 x bath solution was stored at 1°C to 9°C. Bay K8644 was added on the day of experimentation. To analyze the currents of the Caᵥ1.2 channel, the final bath solution included the components: NaCl 100 mM; KCI 4.0 mM; NMDG 40 mM; CaCl₂ 5.0 mM; MgCl₂ 1.0 mM; HEPES 10 mM; D-Glucose 5 mM; Sorbitol 2.50 mM; Bay K8644 0.4 µM; pH (HCl) 7.4.

### Pipette solution

The 1x pipette solution was thawed every day out of a frozen 1x pipette solution, which had been prepared prior to the experimental start of the present study, aliquoted and stored at -10°C to -30°C. When in use, the 1x pipette solution was filled into the patch-pipettes and kept at room temperature (19-30°C). The 1x pipette solution will include the components outlined below:
Naᵥ1.5: CsF 135 mM; NaCl 10 mM; HEPES 10 mM; EGTA 5 mM; pH (KOH) 7.3. Kᵥ1.5: KCI 130 mM; MgCl₂ 1 mM; Mg-ATP 5 mM; HEPES 10 mM; EGTA 5 mM; pH (KOH) 7.2.

Caᵥ1.2: CsF 135 mM; NaCl 10 mM; CaCl₂ 0.2 mM; HEPES 10 mM; EGTA 5 mM; pH (KOH) 7.3.

HCN4: KCI 135 mM; MgCl₂ 2.5 mM; CaCl₂ 2.4 mM; Na₂ATP 3.0 mM; EGTA 5.0 mM; HEPES 5.0 mM; pH (KOH) 7.3.

### Treatment with Compound A

To achieve statistical significance, the tested Compound A was analyzed in sufficient numbers (n=3).

Controls: Three additional experiments (n=3) were conducted with bath solution containing 0.1 % DMSO (DMSO vehicle control). The same cells were treated with a suitable blocker to assure accuracy of the method.

### Reference compounds:

Naᵥ1.5 (State-Dependence): Lidocaine hydrochloride monohydrate (C₁₄H₂₂N₂O · HCl · H₂O; Molecular weight: 288.81 g/mol) supplied from Sigma, Germany; description: solid white powder; purity: 100%; storage conditions: room temperature (19°C - 30°C); stock solution 10 mM (DMSO); test solution: 10 µM (Bath solution).
Naᵥ1.5 (Use-Dependence): Amitriptyline hydrochloride (C₂₀H₂₃N·HCl; molecular weight 313.86 g/mol) supplied from Sigma, Germany; description: solid white powder; purity ≥ 98%; storage conditions: refrigerator (1°C - 9°C); stock solution 3 mM (H₂O); test solution 3 µM.
Kᵥ1.5: Terfenadine (C₃₂H₄₁NO₂; Molecular weight; 471.67 g/mol) supplied from Sigma, Germany; description: white solid; purity 99.7%; storage conditions: refrigerator (1°C - 9°C); stock solution: 10 mM (DMSO); test solution: 10 µM.
Caᵥ1.2: Nifedipine (C₁₇H₁₈N2O6; Molecular weight: 346.33 g/mol) supplied from Sigma, Germany; description: yellow powder; purity > 98%; molecular weight 346.33 g/mol; storage conditions: room temperature (19°C - 30°C); stock solution 10 mM (DMSO); test solution 10 µM.
HCN4: ZD7288 (C₁₅H₂₀N₄; Molecular weight 471.67 g/mol) supplied from Sigma, Germany; purity ≥ 98%; storage conditions: refrigerator (1°C - 9°C); stock solution 100 mM (DMSO); test solution 100 µM.

### Experimental protocol

Four test compound concentrations were tested to investigate the effects of test Compound A on Naᵥ1.5, Caᵥ1.2, Kᵥ1.5, and HCN4 channel activity.

The 35 mm culture dishes upon which cells were seeded at a density allowing single cells to be recorded were placed on the dish holder of the microscope and continuously perfused (at approximately 1 mL/min) with the bath solution described in section 4.7. All solutions applied to cells including the pipette solutions were maintained at room temperature (19°C - 30°C). After formation of a Gigaohm seal between the patch electrodes and individual cells (pipette resistance range: 2.0 MΩ - 7.0 MΩ; seal resistance range: > 1 GΩ) the cell membrane across the pipette tip was ruptured to assure electrical access to the cell interior (whole-cell patch-configuration).

As soon as a stable seal could be established outward tail currents were measured upon pulses described in the Table below:

| **Ion channel** | **Holding potential** | **Pulse / Duration** | **Frequency** |
|---|---|---|---|
| Naᵥ1.5 State dependence | -120 / -80mV / 0 mV (resting, fast / slow inactivated state) | 0 mV / 10 ms (resting, fast / slow inactivated state) | 0.03 Hz (between sweeps at one concentration) 0.0083 Hz (between concentrations) |
| Naᵥ1.5 use dependence | -100 mV | -10 mV / 50 ms | 1 Hz |
| Kᵥ1.5 | -80 mV | +40 mV / 5000 ms | 0.05 Hz |
| Caᵥ1.2 | -80 mV / -50 mV repulse (100 ms) | 0 mV / 200 ms | 0.2 Hz |
| HCN4 | -40 mV | -120 mV / 2000 ms | 0.05 Hz |

Once control recordings have been accomplished, cells were continuously perfused with a bath solution containing the Compound A as detailed above. During wash-in of the Compound A the voltage protocol indicated above run continuously again until the steady-state level of block was reached.

Controls: Three additional experiments per channel (n=3) were conducted with bath solution containing 0.1% DMSO (DMSO vehicle control). The same cells were treated with suitable blockers to assure accuracy of the method.

### Data compilation

Values in Ampere (A) of the peak amplitudes of outward tail currents were generated for each voltage step. These data were printed for compilation and analysis. The recorded current amplitudes at the steady state level of current inhibition were compared to those from control conditions measured in the pre-treatment phase of the same cell. The amount of current block was calculated as percentage of control. To determine whether the observed current inhibition was due to Compound A-interaction with the respective ion channel or due to current rundown, these residual currents were compared to those measured in vehicle treated cells. Data from at least two individual experiments were collected and the corresponding mean values and standard deviations were calculated.

### Experimental results - Example 3

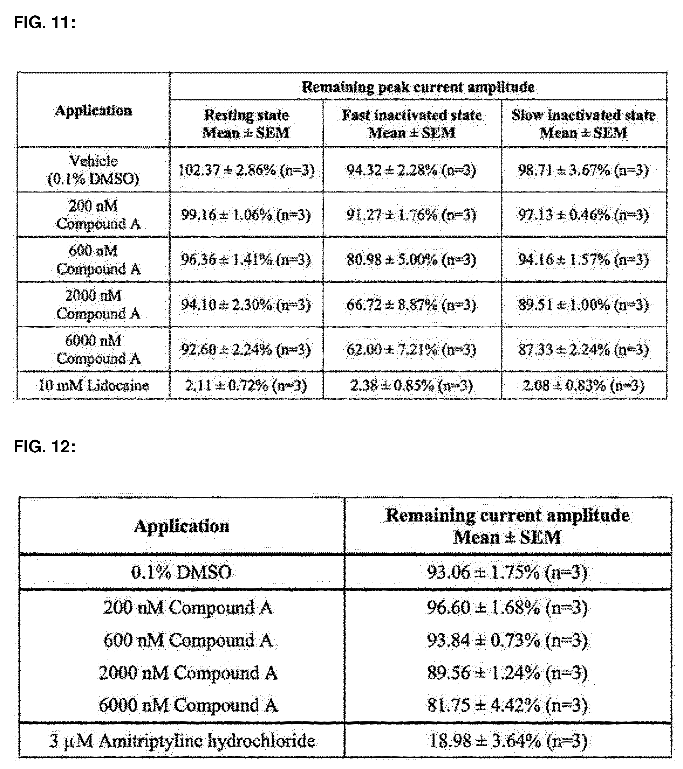

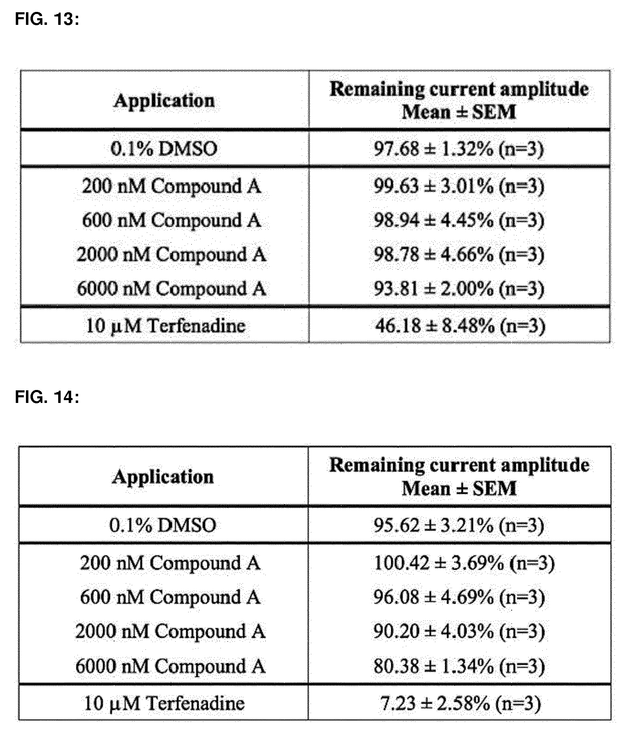

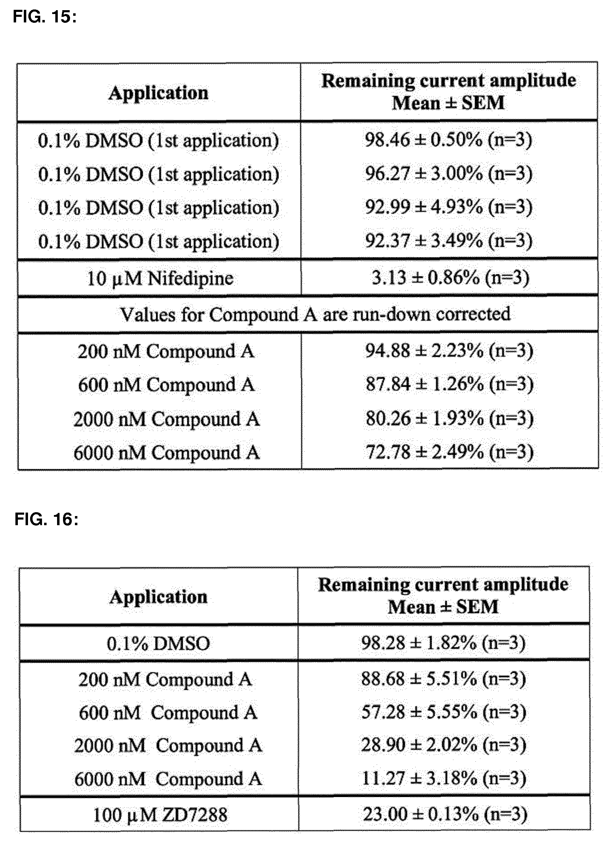

A total of 30 experiments were used for compilation of data and analysis. SigmaPlot 11 was used to calculate the means ± SEM of relative peak current, (Naᵥ1.5, Kᵥ1.5, Caᵥ1.2, HCN4) levels after compound A and the respective blocker application. The representative current traces recorded from compound A and reference blocker treated cells are given in FIG. 11 (Naᵥ1.5, state-dependence), FIG. 12 (Naᵥ1.5, use-dependence - 1 Hz), FIG. 13 (Kᵥ1.5, peak currents), FIG. 14 (Kᵥ1.5, late currents), FIG. 15 (Caᵥ1.2), and FIG.11 16 (HCN4), respectively.

### IC₅₀ determination

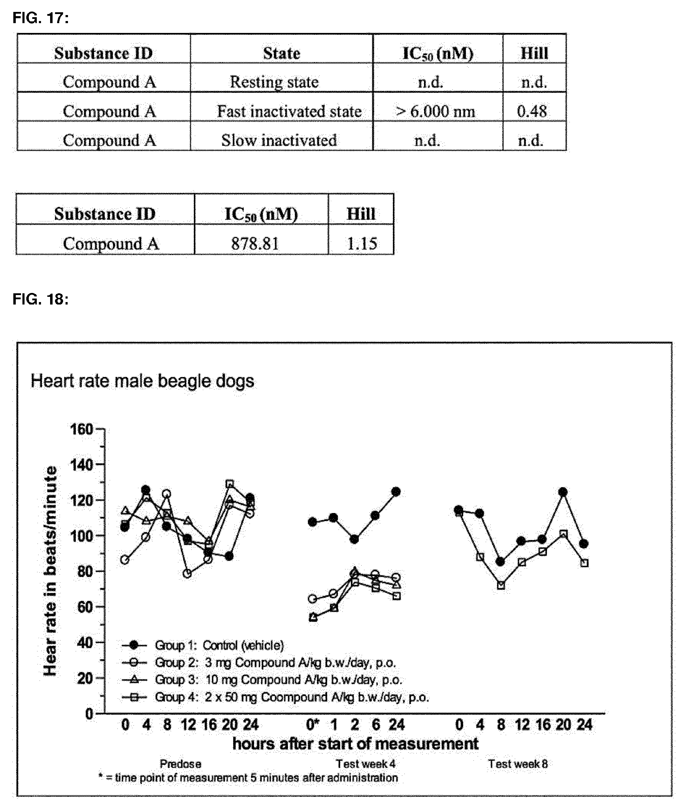

IC₅₀ values were calculated, if the current amplitude was reduced by more than 50 % at ≤ 6 µM of Compound A. As shown in FIG. 17, only for HCN4 and Naᵥ1.5 (fast inactivated state) a reduction of the current amplitude of more than 30 % was observed. For NaV1.5 there was a moderate reduction of the currents determined for the fast inactivated state observed, however, the IC₅₀ was above the highest of 6,000 nM. For HCN4 an IC₅₀ of 878.81 nM (Hill coefficient 1.15) was determined. Hereby, the person skilled in the art is aware that IC₅₀-values may vary in dependency of the specific measurement conditions applied, specifically in case of a complex patch-clamp setup.

### Stability of Compound A

The whole-cell patch-clamp technique was used to demonstrate the effects of Compound A on the currents of Naᵥ1.5, Kᵥ1.5, Caᵥ1.2 and HCN4 channels expressed in CHO cells. For all ion channels 0.1% DMSO was used as vehicle and suitable blockers were applied to assure accuracy of the method. Compound A was tested at concentrations of 200 nM, 600 nM, 2000 nM and 6000 nM.

The Compound A stock solutions were prepared freshly every day of experimentation. Application of Compound A to the CHO cells was completed within one day. During this period, there was no observation indicating any instability of Compound A solved in vehicle. The effects observed on Naᵥ1.5, Kᵥ1.5, Caᵥ1.2 and HCN4 channels of the Compound A dose formulations analyzed remained stable over several hours at room temperature. During this period, there were no observations indicating any instability of tested Compound A solved in bath solution.

### Example 4 - In-vivo example

### Beagle dog experiment - Heart rate reduction by Compound A (4-[5-[3-Chloro-5-(trifluoromethoxy)phenyl]-1-(pyridin-3-yl)-1H-pyrazol-3-yl]-5,6-dihydro-7H-pyrrolo[3,4-d]pyrimidin-7-one application)

### The Compound A

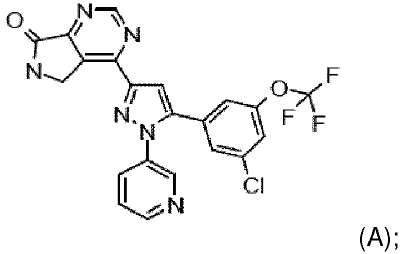

i.e. 4-[5-[3-Chloro-5-(trifluoromethoxy)phenyl]-1-(pyridin-3-yl)-1H-pyrazol-3-yl]-5,6-dihydro-7H-pyrrolo[3,4-d]pyrimidin-7-one,
was administered in a 4-week beagle dog experiment with integrated non-invasive telemetric electrocardiography (ECG) measurements for determining its cardiovascular effects; specifically its effects on the heart rate.

### Conduct of beagle dog experiment

32 Beagle dogs (16 males and 16 females) with parameters in the normal range were tested for cardiovascular effects of Compound A administration and divided into the study groups indicated below.

| Age of the dogs at first dosing | Males: | 7.2 - 7.5 months |
|---|---|---|
| | Females: | 7.1 - 7.4 months |
| | | |
| Body weight of the dogs at first dosing | Males: | 7.0 - 10.5 kg |
| | Females: | 5.5 - 8.2 kg |

The compound A represents the free form of the active drug substance. The dose levels indicated below are expressed in terms of the free base. A correction factor of 1.087 was used when calculating quantities of the tested compound required for dose preparation.

Main study animals: 24 animals (12 males and 12 females); 3 animals/sex/group.

Recovery animals: 8 animals (4 males and 4 females); 2 animals/sex for groups 1 and 4.

Dose levels:
- Group 1: Control (vehicle Labrafil M1944CS);
- Group 2: 3 mg Compound A/kg b.w./day;
- Group 3: 10 mg Compound A /kg b.w./day;
- Group 4: 2 x 50 mg Compound A/kg b.w./day

Duration of study:
- 4 acclimatization weeks
- 4 test weeks
- 4 additional test weeks for the animals scheduled for the recovery period.

Student's t-test was applied for statistics on the telemetry data (p ≤ 0.01).

### Housing and feeding

### Diet

Commercial ssniff^{®} Hd-H V3234 (ssniff Spezialdiäten GmbH, 59494 Soest, Germany) served as food. 40 g/kg b.w. of this food were offered to each dog daily. Food was left for two hours. In case of animals with poor appetite, the food was served for a longer period (up to 8 hours). The residue was removed and weighed.

The food was prepared by mixing the weighed pellets with a constant volume of water.

### Housing

The dogs were kept singly or by twos in kennels maintained at a temperature, which ranged between 17°C and 25 °C. Each kennel had an inside yard and outside yard with a total floor space of 9 m². The cages were specially equipped to keep the animals singly during the daily examinations. The outside yard was accessible at all times (except at the time of feeding and examinations).

### Drinking water

Tap water of drinking water quality was offered *ad libitum.*

### Preparation of the test Compound A - vehicle formulations

The test compound A-vehicle formulations were freshly prepared before each administration.

The vehicle (pure Labrafil M1944CS) was stirred well to ensure homogeneity before use.
a) The required amount of compound A was weighed in a glass vial.
b) The pre-determined amount of Labrafil M1944CS was added to the glass vial containing the test compound A.
c) The content of the vial was subjected to mechanical agitation using a magnetic bar until a uniform suspension was obtained.

The suspensions were kept under agitation until administration to the dogs.

### Administration

The test compound A-formulation of the appropriate concentration was administered by oral administration at a constant administration volume/kg b.w.

### Frequency of administration

Groups 1 and 4: Twice daily at an 8-hour interval for 28 consecutive days.
Groups 2 to 3: Once daily for 28 consecutive days.

### Administration volumes

Groups 1 and 4: 1.25 mL/kg b.w./administration
Groups 2 and 3: 2.5 mL/kg b.w./administration

The first administration of the day was carried out approximately 2 hours before start of feeding. While still in position, the stomach tube was rinsed with 10 mL vehicle immediately after the administration. The control animals received the vehicle at the same administration volume twice daily in the same way as the animals of group 4.

### Dose selection

The dose levels for the 4-week dog study were set at 3, 10, and 2 x 50 mg/kg. The dose levels for this study have been selected based on the results of the 10-day dose-range-finding study in dogs (LPT Study No. 31586) and available (kinetic) data from single dose studies.

For the dog study, single dose data are available at 3, 30 and 100 mg/kg and repeated dose data (10 days of treatment) are available at 30, 100 and 2 x 50 mg/kg. At a single dose of 30 mg/kg, no further increase in exposure or Cmax is apparent. In the 10-day DRF study (LPT Study No. 31586) it was investigated whether after multiple dosing any accumulation would be apparent and therefore dose levels of 30 and 100 mg/kg were selected in this study. In addition, the highest daily dose (i.e. 100 mg/kg) was also administered as 50 mg/kg twice daily (8-h interval) to investigate possible beneficial effects of BID dosing on overall exposure.

The results from the 10-day DRF study showed that Labrafil administered at dose volumes of 5 mL/kg led to vomiting. This clinical sign was not apparent anymore when the dosing volume was lowered to 2.5 mL/kg. No clear and consistent test item related findings were apparent in the 10-day DRF study.

The dose increase from 30 to 100 mg/kg did not reveal a further increase in exposure. Dosing 100 mg/kg over 2 separated 50 mg/kg doses 8 hours apart, did lead to a small increase in exposure.

Based on the above, the dose levels for the 4-week dog study are set at 3 (human equivalent dose), 10 and 2 x 50 mg/kg.

### Group size / Dose levels

The animals were allocated to the 4 test groups employing a pseudo-random body weight stratification procedure that yields groups with approximately equal mean body weight.

| **Group** | **Compound A dose [mg/kg b.w.]^{#}** | **Dose volume [mL/kg b.w.]** | **Frequency of administration** | **Number and sex of animals MS + RP** | **Animal number** | |
|---|---|---|---|---|---|---|
| | | | | | **MS** | **RP** |
| 1 | 0 | 2 x 1.25 | Twice daily in an 8-h interval | 3 + 2 m | 1 - 3 | 4, 5 |
| | Control (Vehicle) | | | 3 + 2 f | 6 - 8 | 9, 10 |
| 2 | 3 | 2.5 | Once daily | 3 m | 11 - 13 | none |
| | Low dose | | | 3 f | 14 - 16 | |
| 3 | 10 | 2.5 | Once daily | 3 m | 17 - 19 | none |
| | Mid dose | | | 3 f | 20 - 22 | |
| 4 | 2x50 | 2 x 1.25 | Twice daily in an 8-h interval | 3 + 2 m | 23 - 25 | 26, 27 |
| | High dose, b.i.d. | | | 3 + 2 f | 28 - 30 | 31, 32 |

| | | | | | | |
|---|---|---|---|---|---|---|
| m: male f: female MS: main study RP: recovery period b.i.d. 'bis in die' (lat.; i.e. twice daily) #: The test compound A represents the free form of the active drug substance. The dose levels are expressed in terms of the free base. Dosing was based on a nominal compound A purity of 100%. A correction factor of 1.087 was applied. | | | | | | |

### Observations

The following observations were made during the course of the Beagle dog study:

### Clinical signs

The animals were observed individually before and after dosing at each time of dosing for any signs of behavioural changes, reaction to treatment or illness.

Kennel side observations included skin/fur, eyes, mucous membranes, respiratory and circulatory systems, somatomotor activity and behaviour patterns. The onset, intensity, and duration of any signs observed were recorded.

### Non-invasive telemetry

Telemetry parameters were monitored at the following times:
- Predose and in test week 4: All 32 animals included in this study.
- At the end of the recovery period
in test week 8: All 8 recovery animals.

Parameters were monitored telemetrically with the EMKA Technologies System continuously starting approximately 24 hours prior to the first treatment (baseline; pre-dose values) and for approximately 24 hours in test week 4 and at the end of the recovery period in test week 8. The dogs were habituated and trained for wearing the jackets equipped with the telemetry transmitter and air-filled cuff placed on their tail.

ECG, skin temperature, respiratory and physical activity data were recorded continuously. Blood pressure data were transmitted in four consecutive 2-minute intervals followed by a 12-minute pause.

The following telemetry parameters were determined:
- Peripheral arterial blood pressure in the tail (blood flow occlusion using an air filled cuff)
- Heart rate
- Electrocardiography (QRS, QT, PQ, RR)
- Respiration (respiratory inductive plethysmography; belts were placed around the thorax and abdomen)
- Skin temperature
- Physical activity.

The report includes the data recorded at the following intervals:
- Pre-dose: Every 4 hours after start of recording (7 (Baseline; approx. 24 hours) tracings per animal)
- Test week 4: Approx. 0, 1, 2, 6, and 24 hours after (Start of treatment to 24 hours post the 1 st administration of the day treatment relating to first dosing of
   the day)
- At the end of the recovery period in Every 4 hours after start of recording (7 test week 8: tracings per animal)

The ECG was evaluated at a time sufficiently before or after the scheduled blood sampling time as blood sampling can cause a disturbance in the ECG recordings.

The electrocardiogram was visually assessed for any abnormalities of the electrical complexes (i.e. arrhythmias, interval prolongations). In addition, the following parameters were evaluated:

| | |
|---|---|
| Heart rate | (beats/min) |
| PQ-interval | (ms) (equivalent to PR interval) |
| QRS-interval | (ms) |
| QT-interval | (ms) |
| RR-interval | (ms) |

Furthermore, the QTc-values were calculated according to the following Bazett-formula: $QTc = \frac{QT \left[sec\right]}{\sqrt{\left(R-R distance \left[sec\right]\right)}}$

### Experimental results - Example 4

### Mortality

### Treatment and recovery period

None of the male and female animals treated with 3, 10, or 2 x 50 mg compound A b.w./day for 4 weeks died prematurely during the course of the study.

### Behaviour, external appearance and faeces

### Treatment and recovery period

No compound A-related signs of systemic intolerance were observed in the male and female animals after oral treatment with 3, 10, or 2 x 50 mg Compound A/kg b.w./day for 4 weeks, neither during the treatment period nor during the 4-week recovery period.

### Non-invasive telemetry

The non-invasive telemetry evaluation was only performed predose and after the 4-week treatment period, and at the end of the recovery period (week 8). Effects are described based on a comparison with the placebo treated animals.

### Treatment period

No compound A-related influence was noted for body weight and body weight gain; food and drinking water consumption.

### Non-invasive telemetry (only week 4 and week 8)

The evaluation of the telemetric parameters of male and female dogs after oral treatment with 3, 10 or 2 x 50 mg Compound A/kg b.w./day revealed the following changes at the end of the treatment period in test week 4:
- a decreased heart rate (up to approximately 50%), and as a consequence,
- also a prolonged RR interval (up to approximately 140%),
- and a prolonged QT interval (up to approximately 50%) were noted for male and female dogs of all three dose level groups, starting within 5 minutes after last administration (1st evaluated telemetric time-point) and lasting until 24 hours after administration (end of telemetric measurement)
- simultaneously, no effect on QTc could be detected as further outlined below.

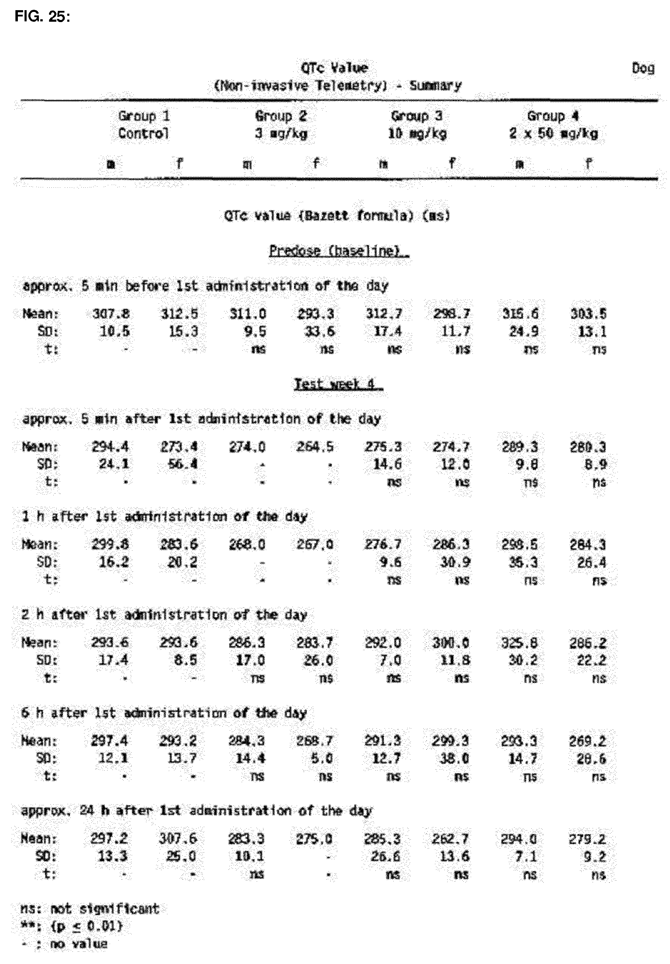

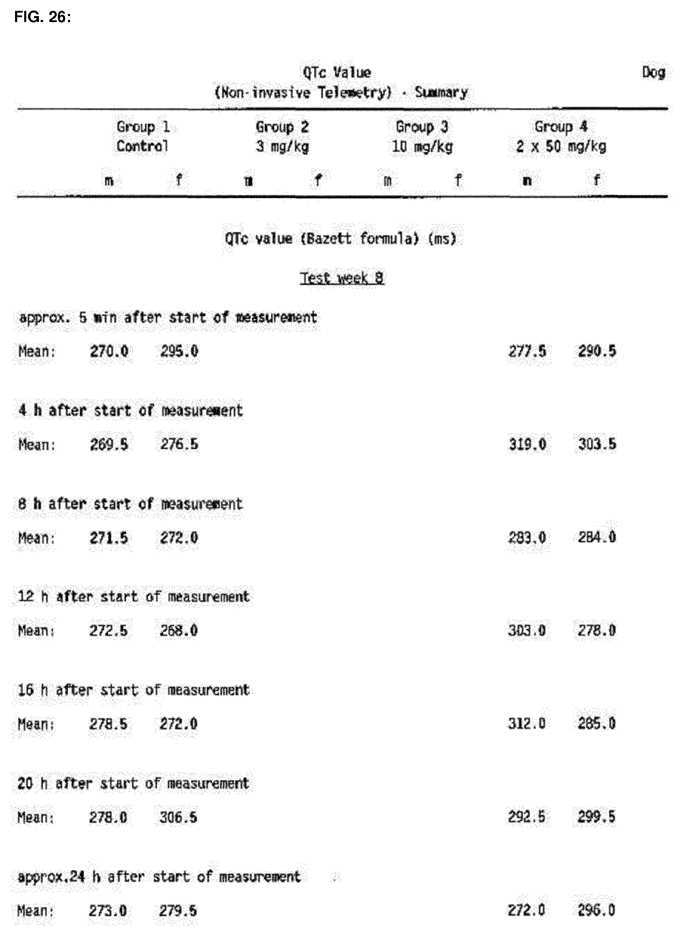

Hereto, the above-mentioned results are depicted by the Figures 18 - 26, respectively.

Statistical significance (at *p* ≤ 0.01, compared to the control group) was reached at various time-points during the 24-h measurement in all three dose level groups in male and female animals (see Figures. 18 to 23, respectively).

A clear dose response was not observed during the 24-h telemetric measurement at the end of the 4 week dosing period. A full recovery of the effects was noted after the 4 week recovery period (i.e. in week 8; see the Figures 18 to 23, respectively).

### Recovery period (restricted to the high dose group)

All changes noted during telemetric recordings for the male and female animals previously treated with 2 x 50 mg compound A/kg b.w./day at the end of the treatment period had completely subsided at the end of the 4-week treatment-free recovery period.

In particular, the heart rate, the RR-intervals, and the QT-intervals of the previously high dosed dogs were again within the range of the control group values (see Figures 18 to 23).

No influence was noted on the body weight, the body weight gain, the food consumption or on any of the laboratory parameters of the previous high dosed animals after 8 weeks.

The macroscopic inspection at necropsy did not reveal any Compound A-related organ changes after 8 weeks.

### Compound A formulation analysis

No compound A-related influences were observed in terms of haematology, clinical biochemistry, urinalysis, ophthamological and auditory examination, organ weights, bone marrow, and histopathology.

Thus, the results of the analysis showed that the compound A-formulations were correctly prepared. The actual concentrations of Compound A within the formulations ranged from 104 % to 110 % of the nominal Compound A concentrations. These values were within the expected range of 80 % to 120 % of the theoretical concentrations.

### Compound A summary

The heart rates, RR intervals, QT, and QTc intervals are shown graphically in the following Figures: Figure 18 (Heart rate - males), Figure 19 (Heart rate - females), Figure 20 (RR interval - males), Figure 21 (RR interval - females), Figure 22 (QT interval - males), Figure 23 (QT interval - females). QTc intervals are depicted in the Figures 24 to 26.

These two plots for males and females appear as being exactly the same. Especially the week 4 and week 8 data.

Thus, a significant lowering of the heart rate and a corresponding extension of the RR- and QT-interval at the end of the 4-week test period was found in all beagle dogs where the Compound A (Molecular weight 472.81 g/mol) was administered orally. 4 weeks after the last dose of compound A (i.e. after 8 weeks), the heart rate, the RR- and QT-interval normalized towards the range of the untreated control animals.

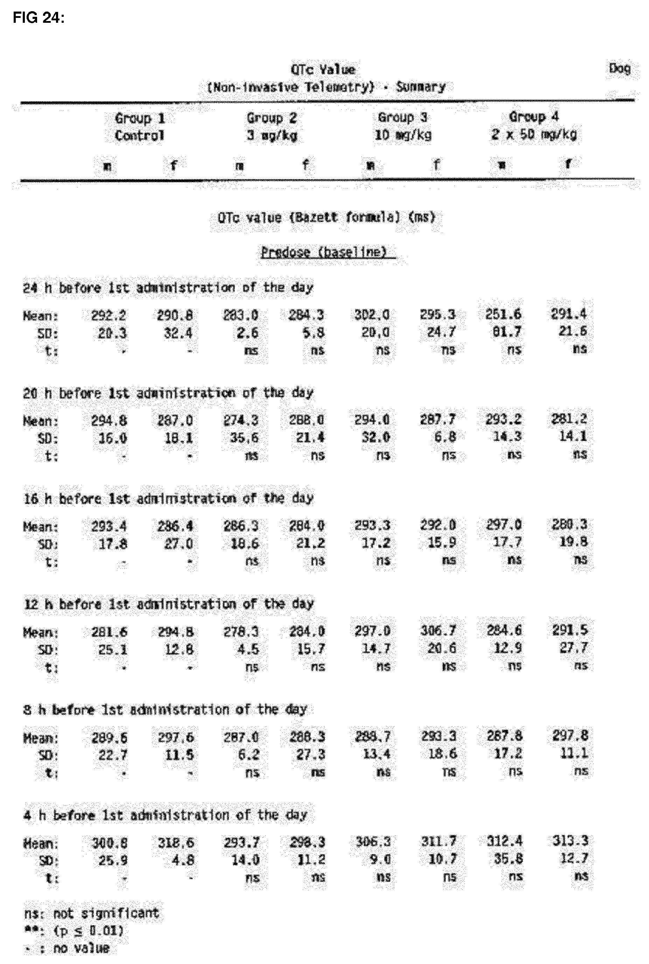

No influence on QTc-interval (see FIGS. 24 to 26), systolic and diastolic blood pressures could be detected for the tested Compound A. Placebo-treated dogs showed no lowering of the heart rate.

Thus, the beagle dog experiments, demonstrate the specific cardiovascular effect of compound A, namely the sole reduction of heart rate, when administered daily by oral administration to beagle dogs for 4 weeks. A complete reversibility of these changes was noted at the end of the 4-week treatment-free recovery period.

Compound A-related changes were noted from the low dose of 3 mg compound A/kg b.w./day onwards during the non-invasive telemetry in form of a decreased heart rate (up to approximately 50%), and as a consequence, also a prolonged RR-interval and a prolonged QT-interval in male and female dogs at the end of the 4-week treatment period.

No compound A-related changes were observed for the behaviour or external appearance of the animals, the body weight and food consumption, or for any of the haematological, biochemical or urinary parameters of the male and female dogs at the end of the 4 week dosing period.

No changes or adverse effects were noted for the eyes and the auditory acuity. Of importance, when directly compared with known side effects of ivabradine treatment.

In view of this, for the person skilled in the art it is readily apparent that the use of the compounds in accordance with the invention in methods of treating and/or preventing the aforementioned diseases may be also exploited in specific patient groups, such as intolerants or non-responders or patients refractory to current therapies with e.g. beta blocker or ivabradine.

The macroscopic inspection at necropsy did not reveal any morphological changes, which are considered being related to the administration of Compound A. No Compound A-related influence was noted on the relative or absolute organ weights of the animals.

The histopathological examination did not reveal any Compound A-related morphological changes. There were no morphological differences between the control dogs and the Compound A treated dogs, neither at the end of the main study nor at the end of the 4-week recovery period.

### Example 5 - Pharmacology - Effects on the cardiovascular systems

Cardiovascular parameters are monitored by telemetry in a suitable *in vivo* model. Control and three escalating dose levels for each animal were tested.
Each animal received all doses (control and escalating doses (low, intermediate, high dose) separated by a washout phase (each animal serves as its own control).

### Example 6 - Pharmaceutical formulations

### Mesoporous silica particles formulation of compound A

Exemplarily, Formac's silica-based drug delivery technology for the solubility-enhancing formulation of the otherwiese poorly water-soluble compound A as active pharmaceutical ingredient (API) has been tested.

Solubility properties, *in vitro* performance and stability of said drug delivery system based on compound A adsorbed onto Formac's silica material OMS-7 was monitored.

Solubility determination: First solubility of compound A was determined in different solvents in order to impregnate the drug onto the silica material.

Impregnation: Compound A was loaded onto OMS-7 via solvent-based impregnation. Briefly, this process involves the addition of a solution of the compound A to the silica material, followed by homogenisation of the wet powder mass and evaporation of the solvent by vacuum drying (50 mbar, 40 °C, 20 hours).

Physical characterization of the silica particles: DSC and XRD method was used to confirm the conversion of crystalline form of compound A into the corresponding amorphous form.

In vitro dissolution: Simulated media such as FassIF and FassGF were used as *in vitro* dissolution medium in order to prove that silica particles increase the dissolution rate of compound A. The corresponding parameters are summarized in below Table 1: Experimental conditions used for in vitro dissolution testing of compound A.

**Table 1 Experimental conditions used for in vitro dissolution testing of AIC195277.**

| | SGF | Medium shift |
|---|---|---|
| Medium | simulated gastric fluid (pH 1.5) | 0-30 min: simulated gastric fluid (pH 1.5) 30-90 min: FaSSIF^{a} |
| Temperature (°C) | 37 | 37 |
| Agitation | Paddle (USP II)-75 RPM | Paddle (USP II) -75 RPM |
| Dose (mg) | 11.5 | 11.5 |
| Volume (ml) | 23 | 23 - made up to 100 ml with FaSSIF after 30 min^{a} |
| Time points (min) | 5-10-30^{b} | 5-10-30-35-45-60-90 |
| Sample volume (ml)^{c} | 1 | 1 |
| Separation undissolved material | Filtration (0.45 µm) | Filtration (0.45 µm) |

Stability studies: The silica particles of compound A obtained at three loadings (20 %, 30 % and 40 %) were subjected to stability studies (at 40 °C/75 % RH) for 1 month.

### Results

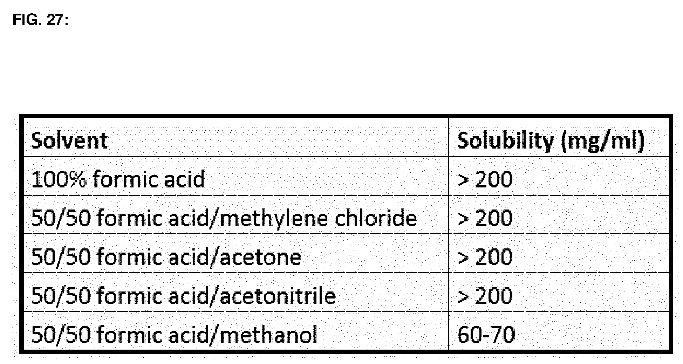

Solubility studies: The table of FIG. 27 shows the solubility of the drug in various solvents suitable for impregnation of the compound A as API onto the silica particles.

Loadability of compound A on OMS-7: Three solvent systems were evaluated in terms of impregnation; i.e. 100 % formic acid, 50/50 % formic acid/methylene chloride and 50/50 % formic acid/acetone. Compound A was impregnated onto OMS-7 to obtain API loadings of 20 %, 30 % and 40 %, after which the samples were assessed for amorphicity by DSC.

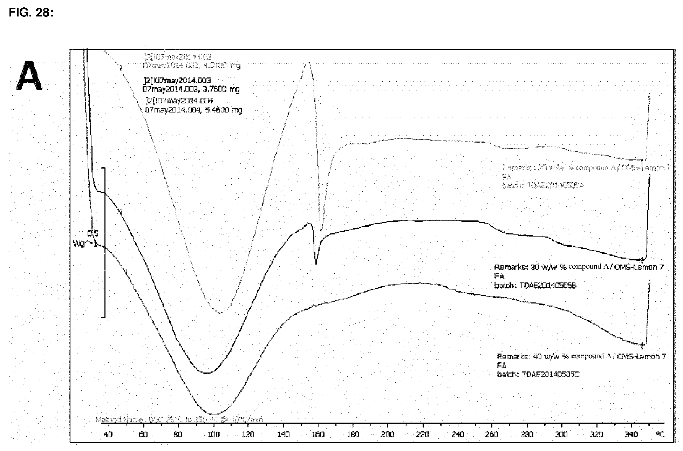

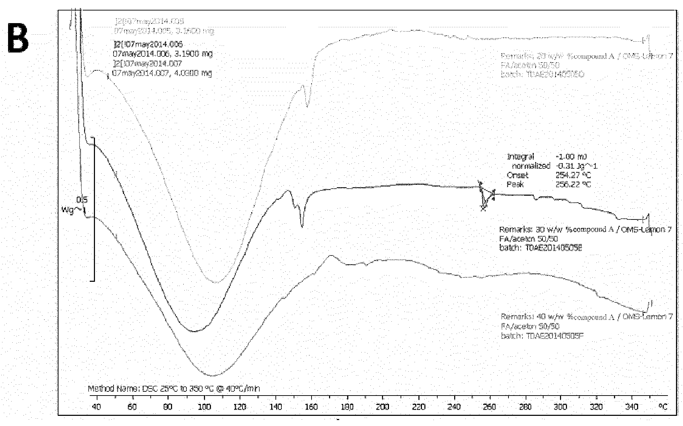

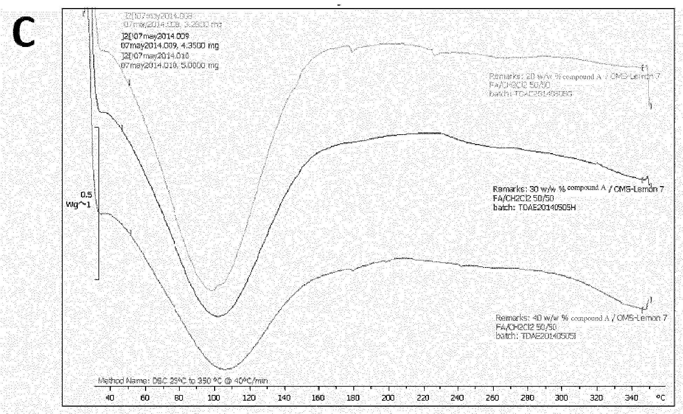

The results are presented in Figures 28 to 32, and show that nearly all samples were devoid of crystalline API (no endothermic signal at the melting point of 278 °C). A small endothermic signal reminiscent of a melting event was apparent in the thermogram compound A-OMS-30 % sample impregnated with 50/50 % formic acid/acetone, which is tentatively ascribed to the melting of a metastable polymorph. Such transitions were not apparent in any of the other samples. The broad endotherm at approx. 100 °C is related to the evaporation of physically adsorbed water and/or formic acid (the boiling point of formic acid is 100.8 °C).

The samples impregnated with 50/50 % formic acid/methylene chloride did not show a thermal event at 160 °C, and therefore this solvent system is preferred for impregnation in accordance with the invention.

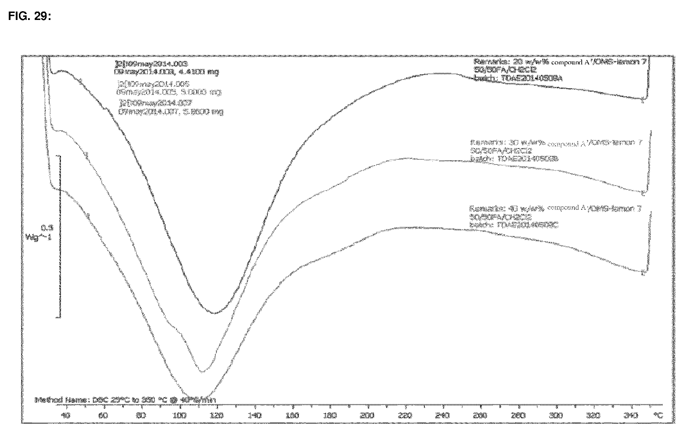

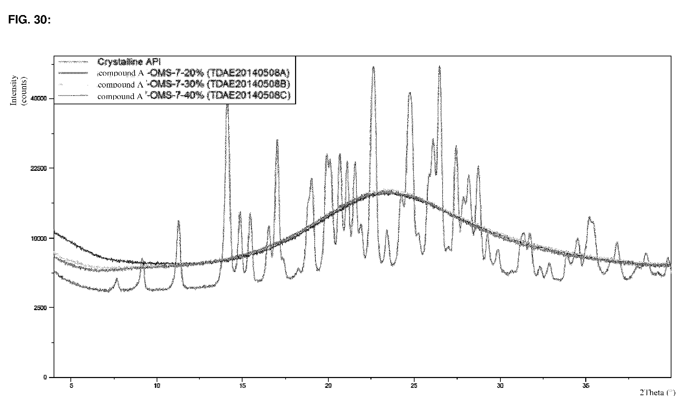

Amorphicity: Both Figures 29 and 30 with its DSC and XRD data demonstrate that all powders are completely amorphous.

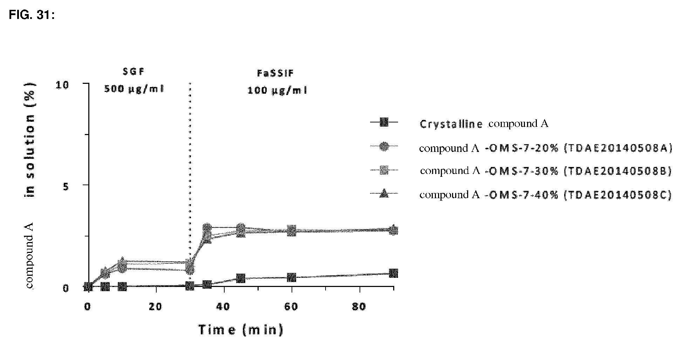

*In vitro* dissolution: The *in vitro* dissolution profiles of crystalline compound A and the compound A-OMS-7 powders are depicted in FIG. 31.

A noticeable improvement of the tested silica-based powders over the crystalline API could be demonstrated. Formulation of drug using mesoporous silica particles resulted in the increase in the dissolution rate of the amorphous drug compared to crystalline drug.
It is noteworthy that, in spite of a broad range of loadings tested (20 % through 40 %), the performance of all tested compound A-OMS-7 powders was consistent.

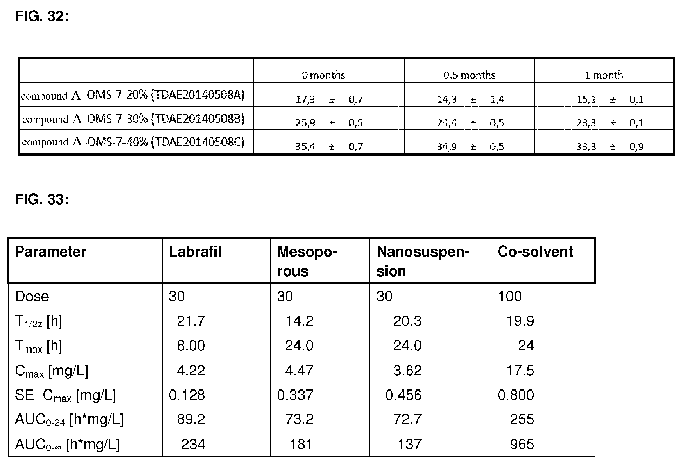

Stability studies: All the tested formulations were stable with respect to the drug content, purity and amorphous nature of API; see FIG. 32.

The above-described data demonstrate that compound A can be converted into its amorphous form via adsorption onto OMS-7 material. Amorphous powders were obtained at 20 %, 30 % and 40 % loading, and porosimetry data confirmed successful deposition of compound A into the OMS-7 pores.

All powders were found to be physically stable for at least 1 month when stored in open containers at 40 °C/75 % RH. A compound A-OMS-7 formulation at 30 % drug loading could be demonstrated as being completely amorphous, and exhibiting an advantageous *in vitro* release performance. Further experiments to verify the effect of pre-dispersion of the compound A-OMS-7 powder suggested that such a pre-dispersion will not significantly affect the performance of the silica system.

### Suspension formulation with Capmul^{®} and Labrafil

An exemplary suspension formulation with Capmul^{®} and Labrafil in accordance with the invention comprises:
- drug loading of 50 mg/ml of a compound of general formula (I)
- a vehicle of 50% of Labrafil^{®} M1944CS, further comprising 7.15 % of acetic acid and 42.85 % of Capmul^{®} MCM EP C8.

### Example 7 - Pharmacokinetic parameters of compound A administered in various formulations to mice and dogs

Compound A was orally administered as non-micronized material, micronized material and ethane sulfonate of compound A suspended in Labrafil^{®} M1944CS and as non-micronized material in three further formulations to mice and dogs in order to demonstrate the resultant systemic exposure; i.e. pharmacokinetic data.

Exemplarily, compound A was formulated as suspension in Labrafil^{®} M1944CS, mesoporous silica particles, aqueous nanosuspension and co-solvent based solution. Single doses of 30 or 500 mg/kg were exemplarily administered to mice, except for the co-solvent based suspension, which was administered at 100 or 500 mg/kg, respectively. A single oral dose of 30 or 100 mg/kg was applied to dogs. Pharmacokinetic parameters were assessed and compared.

### Species tested

Female CD-1 mice. Male and female Beagle dogs.

### Formulations applied

### Labrafil^{®} M1944CS suspension

Compound A was suspended in Labrafil^{®} M1944CS at 3 and 50 mg/mL for the application of 30 and 500 mg/kg to mice. Compound A was suspended in Labrafil^{®} M1944CS at 6 and 50 mg/mL for the application of 30 or 100 mg/kg to dogs.

Micronized compound A was suspended in Labrafil^{®} M1944CS at 6 mg/mL for the application of 30 mg/kg to dogs. Compound A ethane sulfonate was suspended in Labrafil^{®} M1944CS at 15 mg/mL for the application of 30 mg/kg to dogs.

### Mesoporous silica particles

Silica powder was weighed and suspended in a vehicle solution composed of 0.5% Poloxamer 188 and 0.5% sodium lauryl sulfate. The suspension was gently stirred (magnetic stir bar) for approx. 2 min until all powder was wetted and a homogeneous, brown, opaque suspension was obtained. Visible lumps were destroyed by sonication. The suspension was gently stirred until administration to avoid sedimentation. A suspension with a final concentration of 10.7 mg silica powder/mL suspension and 3 mg compound A/mL were prepared to apply 30 mg/kg to mice. Suspensions with final concentration of 179.2 mg silica powder/mL suspension and 50 mg compound A/mL were prepared to apply 500 mg/kg to mice. Suspensions with final concentration of 17.7 mg silica powder/mL suspension and 20 mg compound A/mL were prepared to apply 100 mg/kg to dogs.

### Aqueous nanosuspension

The supplied sterile nanosuspension contained 81.76 mg compound A/g in a vehicle solution composed of 0.034% Poloxamer 188 and 0.5% PVP K17.

The suspension was vortex-mixed for 5 min and subsequently diluted in sterile vehicle to obtain a nanosuspension of 3 mg compound A/g for the low dose group (30 mg/kg) and 50 mg compound A/g for the high dose group (500 mg/kg) in mice.

The suspension was vortex-mixed for 5 min and subsequently diluted in sterile vehicle to obtain a nanosuspension of 30 mg compound A/g for the administration of 100 mg/kg to dogs. The nanosuspension was subjected to sonication for 30 min with intermittent shaking to avoid particle agglomeration. The syringe was rinsed with the nanosuspension 2-3 times prior to injection. The exact amount of administered nanosuspension was determined by weighting the syringe before and after dosage.

### Co-solvent based solution

A ready-to-use vehicle consisting of acetic acid and Capmul MCM C8 EP in a ratio of 14.3 % + 85.7 % (w+w) was supplied. The pH of the final formulation was around 2.5.

**Table 2: Preparation of co-solvent based solutions**

| **Species** | **Dose [mg/kg]** | **Formulation preparation** |
|---|---|---|
| Mouse | 500 | The vehicle was prepared by addition of 4 mL acetic acid: Capmul^{®} MCM C8 EP: Labrafil in a ratio of 14.3 % + 85.7 % (w+w) to 4 mL Labrafil M1944 CS. 400 mg compound A were added to 8 ml vehicle. The suspension was stirred at 55°C for at least 1.5 hours until a dark brown homogeneous suspension was formed. |

### Administration of tested formulations containing compound A

All formulations were orally administered via gavage to mice and dogs:

**Table 3: Dose, vehicle, dose volume and compound A concentration for mice**

| **Dose [mg/kg]** | **Vehicle** | **Dose volume [mL/kg]** | **Concentration [mg/mL]** |
|---|---|---|---|
| 30 | Labrafil^{®} M1944CS | 10 | 3 |
| 500 | Labrafil^{®} M1944CS | 10 | 50 |
| 30 | Mesoporous silica particles | 10 | 3 |
| 500 | Mesoporous silica particles | 10 | 50 |
| 30 | Aqueous nanosuspension | 10 | 3 |
| 500 | Aqueous nanosuspension | 10 | 50 |
| 500 | acetic acid/ Capmul^{®} MCM C8 EP/ Labrafil^{®} | 10 | 50 |

**Table 4: Dose, vehicle, dose volume and test item concentration for dogs**

| **Dose [mg/kg]** | **Vehicle** | **Test item** | **Dose volume [mL/kg]** | **Concentration [mg/mL]** |
|---|---|---|---|---|
| 30 | Labrafil^{®} M1944CS | Compound A | 2 | 15 |
| 100 | Labrafil^{®} M1944CS | Compound A | 2 | 50 |
| 30 | Labrafil^{®} M1944CS | Micronized Compound A | 5 | 6 |
| 30 | Labrafil^{®} M1944CS | Compound A ethane sulfonate | 2 | 15 |
| 100 | Mesoporous silica particles | Compound A | 5 | 20 |
| 100 | Aqueous nanosuspension | Compound A | .33 | 30 |

### Blood Sampling

Blood sampling time points were kept constant for all formulations at 1, 2, 4, 8, 24, 48, 72 and 120 hours after administration of 30 mg/kg or 100 mg/kg to mice.

For the 500 mg/kg dose groups blood was sampled 1, 4, 8, 24, 48, 96, 144 and 192 hours after administration to mice. Three mice were sacrificed per time point.

Blood sampling time points of the dogs were predose 0.5, 1, 2, 4, 8, 12, 24, 48, 72, 96, 120, 144, 168, 192, 216, 264 and 288 hours after administration of a Labrafil^{®} M1944CS suspension and predose 0.5, 1, 2, 4, 8, 12, 24, 48, 72, 96, 144, 192, 240 and 288 hours after administration of all other formulations.

### Data Evaluation

Plasma concentrations were obtained by LC-MS/MS measurements. Pharmacokinetic parameters were calculated by use of the Phoenix™ WinNonLin^{®} software (Build 6.3.0.395, Certara L.P.) using non-compartmental pharmacokinetic models 200-202 (extravascular input) and linear trapezoidal fit (linear Interpolation). All further calculations were carried out with Microsoft Excel 2010.

### Results / Pharmacokinetic data of compound A

### Mouse

The objective was to demonstrate the systemic exposure of compound A after administration in the pharmaceutical formulations of the invention by comparison of compound A formulated as Labrafil^{®} M1944CS suspension, mesoporous silica particles, aqueous nanosuspension or co-solvent based suspension after administration to female CD-1 mice.

Similar plasma concentration vs. time profiles were obtained after oral administration of compound A at 30 mg/kg formulated as either mesoporous silica particles or Labrafil^{®} M1944CS suspension. Accordingly, similar exposure (AUC_{0-∞}) was obtained.

The Labrafil^{®} M1944CS suspension showed slightly higher exposure with 234 h*mg/L when compared to mesoporous silica particles with 181 h*mg/L. Maximal plasma concentrations (Cₘₐₓ) were observed 8 hours after administration of the Labrafil^{®} M1944CS suspension at 4.22 mg/kg and 24 hours after administration of the mesoporous silica particles at 4.47 mg/L.

The aqueous nanosuspension showed clearly lower values for AUC0-∞ and Cmax with 137 h*mg/kg and 3.62 mg/L. The aqueous nanosuspension was very foamy and the correct dose was determined by weighing the application syringe before and after administration. The applied doses were found to be within a range of 25.3 to 31.7 mg/kg and were in good agreement with the nominal dose of 30 mg/kg

At high dose with 500 mg/kg, the mesoporous silica particles showed slightly higher exposure (AUC_{0-∞}) and plasma concentrations when compared to the Labrafil^{®} M1944CS suspension but also higher inter-individual variability. Exposure was found to be 567 and 644 h*mg/kg for the Labrafil^{®} M1944CS suspension and the mesoporous silica particles, respectively. Cₘₐₓ accounted for 7.43 mg/L and 7.57 mg/L for compound A administered as Labrafil^{®} M1944CS suspension and mesoporous silica particles.

The oral administration of compound A formulated as aqueous nanosuspension at dose levels of 500 mg/kg to mice showed lower exposure in terms of AUC0-∞ when compared to the exposure obtained from the other formulations. AUC0-∞ was 346 h*mg/L and Cmax was 7.56 mg/L.

No test item-related signs of systemic intolerance were noted for any of the mice treated once orally with 30 mg or 500 mg compound A/kg body weight formulated as Labrafil^{®} M1944CS suspension, mesoporous silica particles or as aqueous nanosuspension.

After oral dosage of compound A at 500 mg/kg formulated in Capmul^{®}/acetic acid and Labrafil^{®} M1944CS, the mean maximal plasma concentration (Cₘₐₓ) of 4.79 mg/L was shifted to later time points and was found 48 hours after administration. AUC_{0-∞} could not be calculated as the terminal elimination phase was described by less than three blood sampling time points. AUC₀₋₂₄ was 64.4 h*mg/L.

No test item-related signs of systemic intolerance were noted for any of the mice treated once orally with 500 mg/kg body weight. None of the animals died and no test item-related changes were noted.

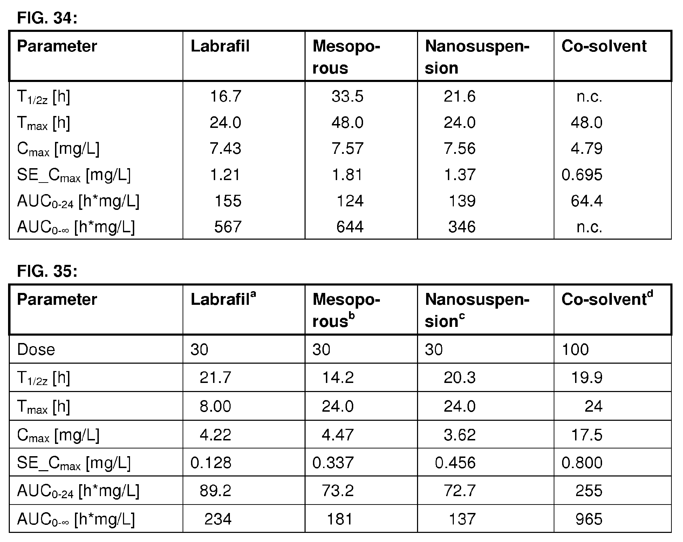

Selected pharmacokinetic parameters are summarized in the Tables of Figures 33 and 34.

### Dog

The objective was to demonstrate the highest exposure by comparison of the pharmaceutical formulations of the inventions with compound A; being it micronized compound A, compound A mono ethane sulfonate and non-micronized compound A after administration at 30 mg compound A/kg to dogs.

Further comparisons were performed with non-micronized compound A formulated as Labrafil^{®}M1944CS suspension, mesoporous silica particles, or aqueous nanosuspension. All formulations were administered at 100 mg/kg to dogs.

Maximal plasma concentrations were observed 4 to 12 hours after administration of compound A at 30 mg/kg to dogs at 2.02, 2.46 and 3.03 mg/L for micronized compound A, ethane sulfonate and non-micronized compound A, respectively.

The corresponding exposure in terms of AUC_{0-∞} was 190, 218 and 297 h*mg/L for micronized compound A, ethane sulfonate and non-micronized compound A. With two treatments (micronized compound A and compound A mono ethane sulfonate) vomiting occurred in two to three out of four animals which does not exclude, that the exposure would have been even higher if no vomiting had occurred.

Selected pharmacokinetic parameters after administration of non-micronized compound A, micronized compound A and the ethane sulfonate of compound A are summarized in the Table of FIG. 35.

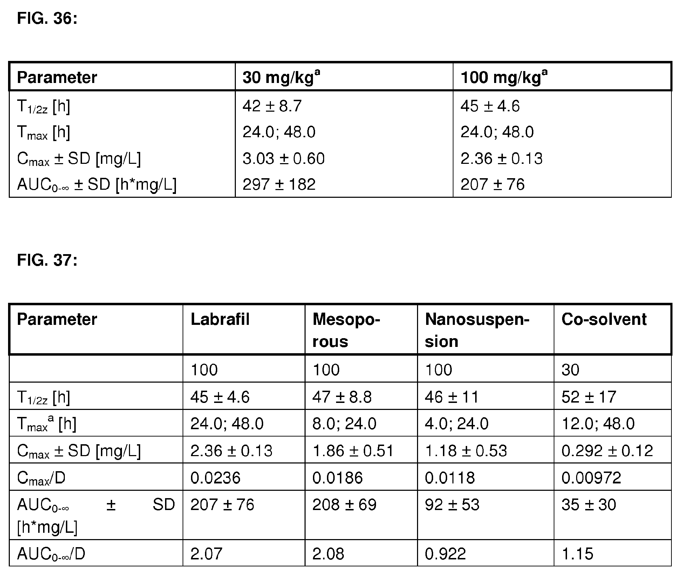

The administration of compound A at 30 and 100 mg/kg to dogs led to similar exposures and plasma concentrations. In an attempt to detect the maximal possible effect of formulations further investigations were performed at 100 mg/kg. A comparison of selected pharmacokinetic parameters is given in the Table of FIG. 36.

Comparable plasma concentration vs. time profiles were obtained after oral administration of 100 mg compound A/kg body weight to dogs formulated as either Labrafil^{®} M1944CS suspension or mesoporous silica particles. Accordingly, similar exposure was obtained. Maximal plasma concentrations were observed 24 to 48 hours after administration of 100 mg compound A/kg as Labrafil^{®} M1944CS suspension at 2.36 mg/mL and a corresponding exposure in terms of AUC_{0-∞} of 207 h*mg/L. With compound A formulated as mesoporous silica particles maximal plasma concentrations were found 8 to 24 hours after administration at 1.86 mg/L and an AUC_{0-∞} of 208 h*mg/L.

Significantly lower exposure was obtained after administration of 100 mg compound A/kg formulated as aqueous nanosuspension. The correct dose was determined by weighing the application syringe before and after administration due to the foamy consistence of the aqueous nanosuspension. The applied doses ranged from 92.2 to 101 mg/kg and were found to be in good agreement with the nominal dose of 100 mg/kg. The maximal plasma concentration was 1.18 mg/mL 4 to 24 hours after administration. The corresponding exposure (AUC0-∞) was 92 h*mg/L.

After single oral administration of compound A formulated as Labrafil^{®} M1944CS, mesoporous silica particles or aqueous nanosuspension at a dose level of 100 mg/kg to dogs, no test item-related signs of systemic intolerance were noted for any of the individuals.

Selected pharmacokinetic parameters are summarized in the Table of FIG. 37.

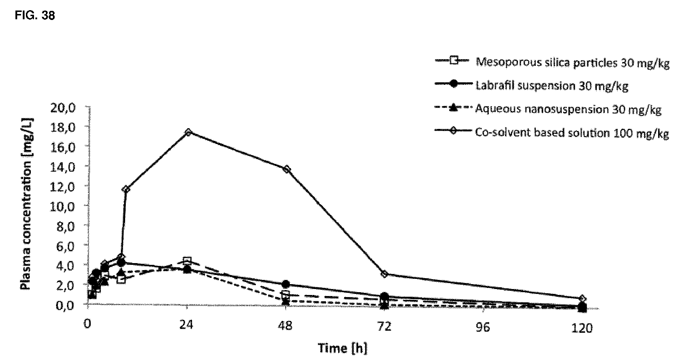

Charts on mean plasma concentration versus time profiles of the above-described examples can be derived from the Figures 38 to 45.

### Definitions

With the context of the present invention, the term "excipient(s)" or similar terms herein means any substance, not itself a therapeutic agent, used as a diluent, adjuvant, or vehicle for delivery of a therapeutic agent to a subject or added to a pharmaceutical composition to improve its handling or storage properties or to permit or facilitate formation of a solid dosage form such as a tablet, capsule, or a solution or suspension suitable for oral, parenteral, intradermal, subcutaneous, or topical application. Excipients can include, by way of illustration and not limitation, solubizers, diluents, disintegrants, binding agents, adhesives, wetting agents, polymers, lubricants, glidants, stabilizers, and substances added to mask or counteract a disagreeable taste or odor, flavors, dyes, fragrances, and substances added to improve appearance of the composition. Acceptable excipients include (but are not limited to) stearic acid, citric acid, maleic acid, magnesium stearate, magnesium oxide, sodium and calcium salts of phosphoric and sulfuric acids, magnesium carbonate, talc, gelatin, acacia gum, sodium alginate, pectin, dextrin, mannitol, sorbitol, lactose, sucrose, starches, gelatin, cellulosic materials, such as cellulose esters of alkanoic acids and cellulose alkyl esters, low melting wax, cocoa butter or powder, polymers such as polyvinyl-pyrrolidone, polyvinyl alcohol, and polyethylene glycols, inert carriers such as silica particles and other pharmaceutically acceptable materials. The choice of excipient will to a large extent depend on factors such as the particular mode of administration, the effect of the excipient on solubility and stability, and the nature of the dosage form.

According to the present invention, the term "treatment" or "treating" and inhibiting, delaying, halting, ameliorating, attenuating, restrict, reduce, suppress, recoiling or cure a disease, condition, an illness, an injury or a health disorder, the development, the course or progression of such conditions and / or symptoms of such conditions. The term "treatment" is understood here as synonymous with the term "treatment". The terms "prevention", "prophylaxis" or "prevention" are used in the present invention interchangeably and refer to avoiding or reducing the risk of a disease, ailment, injury or health disorder, an unfolding or progression of such conditions and / or the symptoms of such conditions, to suffer or to have. The treatment or prevention of a disease, condition, an illness, an injury or a health disorder can be partial or complete.

References herein to "treat", "treating", "treatment" and the like include curative, palliative and prophylactic treatment.

By "pharmaceutically acceptable" is meant the carrier, vehicle, or diluent and/or salt must be compatible with the other ingredients of the formulation, and not deleterious to the recipient thereof.

The term "pharmaceutically effective amount", as used herein, refers to an amount of the compound of Formula I (or a combination agent or a Formula I compound in combination with a combination agent) sufficient to treat, prevent onset of or delay or diminish the symptoms and physiological manifestations of the indications described herein.

The term "coronary artery disease", as used herein, is selected, but not limited to, the group consisting of atherosclerotic plaque (e.g., prevention, regression, stablilization), vulnerable plaque (e.g., prevention, regression, stabilization), vulnerable plaque area (reduction), arterial calcification (e.g., calcific aortic stenosis), increased coronary artery calcium score, dysfunctional vascular reactivity, vasodilation disorders, coronary artery spasm, first myocardial infarction, myocardia re-infarction, ischemic cardiomyopathy, stent restenosis, PTCA restenosis, arterial restenosis, coronary bypass graft restenosis, vascular bypass restenosis, decreased exercise treadmill time, angina pectoris/chest pain, unstable angina pectoris, exertional dyspnea, decreased exercise capacity, ischemia (reduce time to), silent ischemia (reduce time to), increased severity and frequency of ischemic symptoms, reperfusion after thrombolytic therapy for acute myocardial infarction.

The term "hypertension", as used herein, is selected, but not limited to, the group consisting of lipid disorders with hypertension, systolic hypertension and diastolic hypertension.

The term "diabetes", as used herein, refers to any of a number of diabetogenic states including type I diabetes, type II diabetes, Syndrome X, Metabolic syndrome, lipid disorders associated with insulin resistance, impaired glucose tolerance, non-insulin dependent diabetes, microvascular diabetic complications, reduced nerve conduction velocity, reduced or loss of vision, diabetic retinopathy, increased risk of amputation, decreased kidney function, kidney failure, insulin resistance syndrome, pluri-metabolic syndrome, central adiposity (visceral)(upper body), diabetic dyslipidemia, decreased insulin sensitization, diabetic retinopathy/neuropathy, diabetic nephropathy/micro and macro angiopathy and micro/macro albuminuria, diabetic cardiomyopathy, diabetic gastroparesis, obesity, increased hemoglobin glycoslation (including HbA1C), improved glucose control, impaired renal function (dialysis, endstage) and hepatic function (mild, moderate, severe).

"Metabolic syndrome," also known as "Syndrome X," refers to a common clinical disorder that is defined as the presence of increased insulin concentrations in association with other disorders including viceral obesity, hyperlipidemia, dyslipidemia, hyperglycemia, hypertension, and potentially hyperuricemis and renal dysfunction.

According to the present invention, the term "heart failure", both acute and chronic manifestations of heart failure, as well as specific or related disease forms such as acute congestive heart failure, right heart failure, left ventricular failure, global failure, ischemic cardiomyopathy, dilated cardiomyopathy, hypertrophic cardiomyopathy, idiopathic cardiomyopathy, congenital heart disease, heart failure in valvular heart, mitral valve stenosis, mitral regurgitation, aortic stenosis, aortic regurgitation, tricuspid stenosis, tricuspid insufficiency, pulmonary valve stenosis, pulmonary valve insufficiency, combined valvular, heart muscle inflammation (myocarditis), chronic myocarditis, acute myocarditis, viral myocarditis, diabetic heart failure, alcoholic cardiomyopathy, cardiac storage diseases, diastolic congestive heart failure and systolic heart failure and acute phases of exacerbation of chronic heart failure (worsening heart failure).

The expression "slow rate heating" or similar expressions denote within the context of the present invention that a chemical reaction or an apparatus and/or device, wherein a chemical reaction takes place, is gradually heated by adequate heating steps to allow for a dosed thermal energy supply depending on the chemical reaction or apparatus and/or device used. For instance, and only exemplarily without being limited thereto, a slow heating rate could mean to increase the temperature of a chemical reaction or an apparatus and/or device by 10 +/- 5 °C per hour. The person skilled in the art is well aware how to apply a slow rate heating for a chemical reaction or an apparatus and/or device used, which may differ from reaction to reaction or apparatus/device to apparatus/device in terms of technical adjustments that would have to be performed, so to realize an adequate slow rate heating (see e.g. Lehrbuch der Technischen Chemie: Chemische Prozesskunde, Band 3, Ulfert Onken and Arno Behr, John Wiley & Sons, 1st edition (29. May 1996), ISBN-10: 3527308644, ISBN-13: 978-3527308644; Wilhelm Keim, Arno Behr and Günter Schmitt: Grundlagen der industriellen Chemie. Technische Produkte und Prozesse, ISBN 3-7935-5490-2 (Salle), ISBN 3-7941-2553-3 (Sauerländer)).

### Figure Description

FIG. 1: HPLC analysis of an exemplary batch of synthesis of compound 5 according to synthesis phase I.
FIG. 2: 1H-NMR spectrum of an exemplary final batch of synthesis of compound 5 according to synthesis phase I.
FIG. 3: HPLC chromatogram of the combined batches of crude compound A (top chromatogram) and of the final product compound A (middle chromatogram): Column: Luna Phenomex (C18, 150 x 2.0 mm, 3.0 µm). Flow: 0.25 ml/min. Column temp: 25 ºC. Eluent A: 0.1 % formic acid in water. Eluent B: 0.1 % formic acid in MeCN. Gradient: t=0 min 40% B, t=1 min 40% B, t=16 min 70% B, t=16.1 min 98% B, t=17 min 98% B. Detection: DAD (252 nm (4 nm)). HPLC chromatogram of the final product compound A (bottom chromatogram): Column: Agilent Eclipse XDB C18 5 µ 4.6 x 150 mm. Column temp: 35°C. Flow: 1.5 mL/min. Eluent C: 0.1% phosphoric acid in water). Eluent D: 0.1% phosphoric acid in acetonitrile. Gradient: t = 0 min 10% D, t = 1 min 10% D, t = 18 min 95% D, t = 23 min 95% D. Detection: DAD (220 - 320 nm).
FIG. 4: ¹H-NMR of the final product compound A.
FIG. 5: Table - Analytical results for the batch of compound A obtained by Example 1 (final release testing).
FIGS. 6-9: SigmaPlot 11 was used to calculate the means ± SEM of relative steady state current blockade for each concentration of test item. Analysis of Variance (ANOVA), multisample comparison (Dunnett) was conducted to test statistical significance of the test concentrations and the reference compounds versus vehicle (0.1% DMSO in Bath solution) using GraphPad Prism 5. Representative current traces recorded from test item treated and blocker treated cells are shown.
FIG. 10: IC₅₀ determination - The dose-response curves for the ion channels treated with the test items were constructed with a sigmoidal two parameter equation: ${current}_{peak , relaive} = 100 / 1 + {\left(X/{IC}_{50}\right)}^{H} ,$ where X is the actual drug concentration, IC₅₀ is the concentration of drug at half maximal inhibition and H is the Hill coefficient. SigmaPlot 11.0 was used for construction of the curves.
FIG. 11: Na_{V}1.5 - State-Dependence
FIG. 12: Na_{V}1.5 - Use-Dependency (1 Hz)
FIG. 13: K_{V}1.5, peak currents
FIG. 14: K_{V}1.5, late currents
FIG. 15: Caᵥ1.2 - State-Dependence
FIG. 16: HCN4 - State-Dependence
FIG. 17: IC₅₀ values of Na_{V}1.5 and HCN4 channels, state dependence; n.d.: not determined
FIG. 18: Heart rate of male beagle dogs - mean values per group

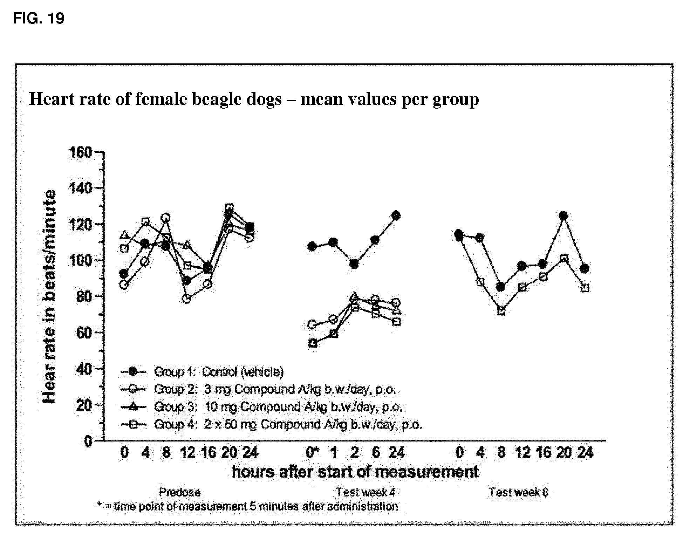

FIG. 19: Heart rate of female beagle dogs - mean values per group

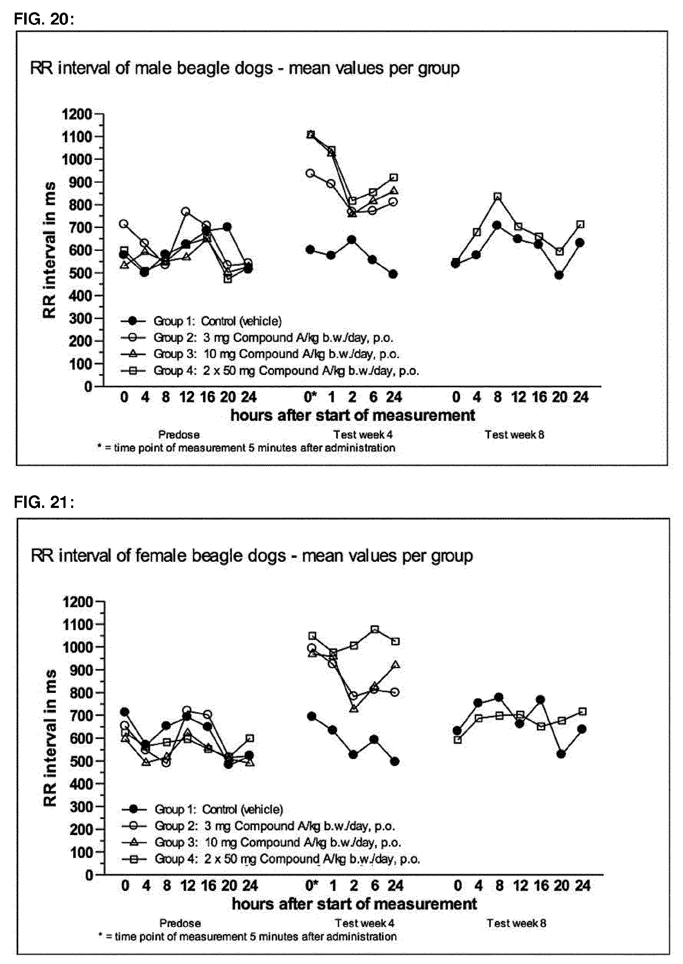

FIG. 20: RR interval of male beagle dogs - mean values per group
FIG. 21: RR interval of female beagle dogs - mean values per group

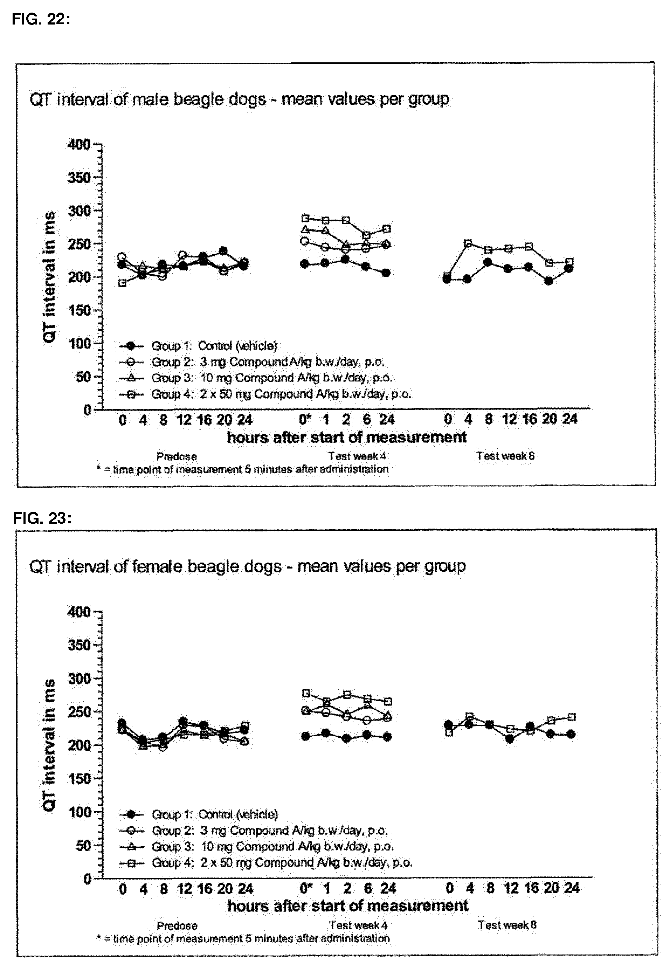

FIG. 22: QT interval of male beagle dogs - mean values per group
FIG. 23: QT interval of female beagle dogs - mean values per group
FIGS. 24-26: QTc values of female and male beagle dogs
FIG. 27: Table - Solubility data of mesoporous silica-based formulation for compound A according to Example 6.
FIG. 28: DSC thermograms of compound A-OMS-7 powders at 20 %, 30 % and 40 % drug loading. (A): Samples impregnated with pure formic acid. (B): Samples impregnated with 50/50 % formic acid/acetone. (C): Samples impregnated with 50/50 % formic acid/methylene chloride.
FIG. 29: Amorphicity - DSC data demonstrate that all tested powders are completely amorphous.
FIG. 30: Amorphicity - XRD data demonstrate that all powders are completely amorphous.
FIG. 31: In vitro dissolution profiles of crystalline compound A and the corresponding compound A-OMS-7-powders.
FIG. 32: API content in compound A-OMS-7 powders before and after storage in open containers at 40 °C / 75 % RH.
FIG. 33: Table - Selected pharmacokinetic parameters (means) after oral administration of 30 or 100 mg compound A/kg to female mice
FIG. 34: Table - Selected pharmacokinetic parameters (means) after oral administration of 500 mg compound A/kg to female mice
FIG. 35: Table - Selected pharmacokinetic parameters (means) after oral administration of 30 mg compound A/kg to dogs
FIG. 36: Table - Selected pharmacokinetic parameters (means) of compound A after oral administration of a Labrafil^{®} M1944CS suspension at 30 or 100 mg/kg to dogs
FIG. 37: Table - Selected pharmacokinetic parameters (means) after oral administration of 30 or 100 mg compound A/kg to dogs
FIG. 38: Mean plasma concentration vs. time profiles after oral administration of 30 or 100 mg compound A/kg to female mice (top) and semi-logarithmic plot (bottom)

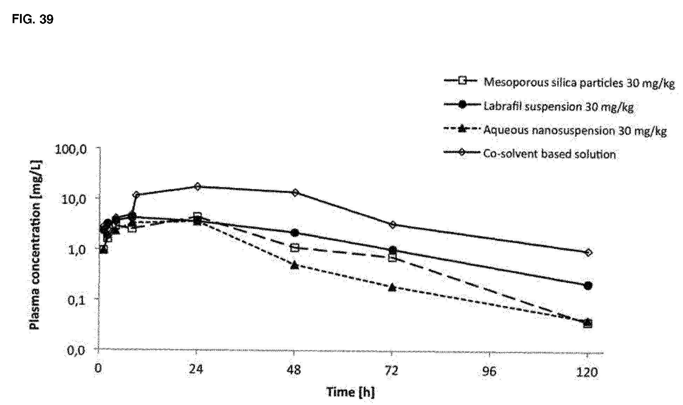

FIG. 39: Mean plasma concentration vs. time profiles after oral administration of 30 or 100 mg compound A/kg to female mice with semi-logarithmic plot

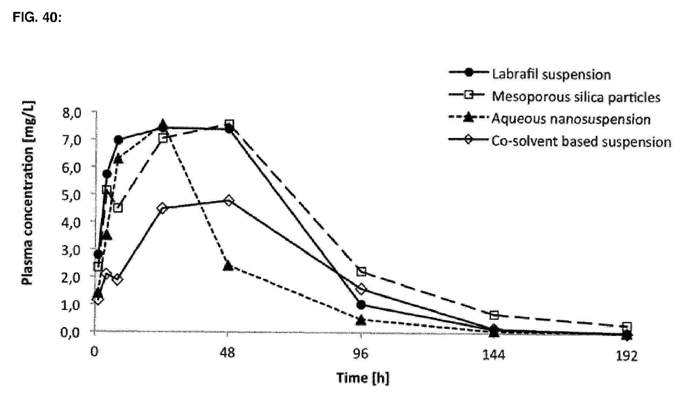

FIG. 40: Mean plasma concentration vs. time profiles after oral administration of 500 mg compound A/kg to female mice

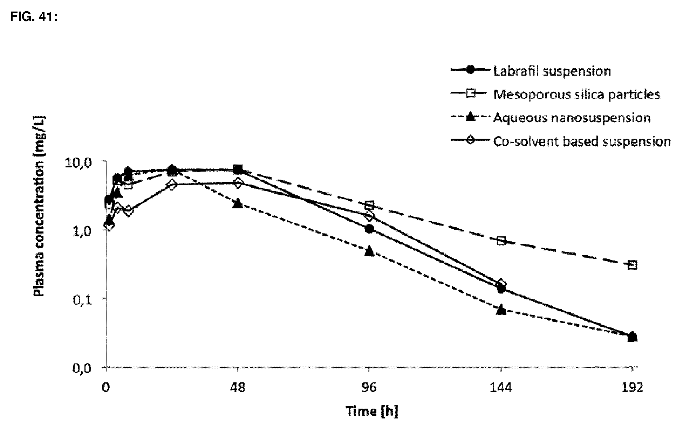

FIG. 41: Mean plasma concentration vs. time profiles after oral administration of 500 mg compound A/kg to female mice with semi-logarithmic plot

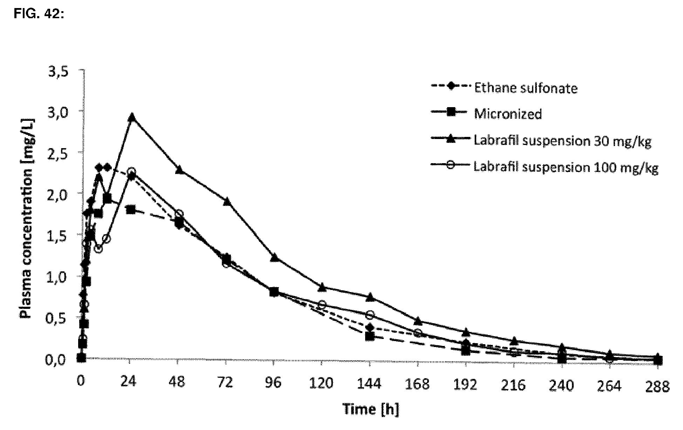

FIG. 42: Mean plasma concentration vs. time profiles after oral administration of 30 or 100 mg compound A/kg to dogs

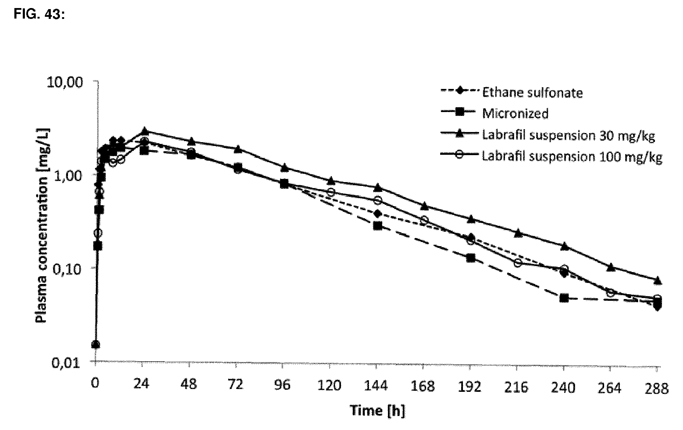

FIG. 43: Mean plasma concentration vs. time profiles after oral administration of 30 or 100 mg compound A/kg to dogs with semi-logarithmic plot

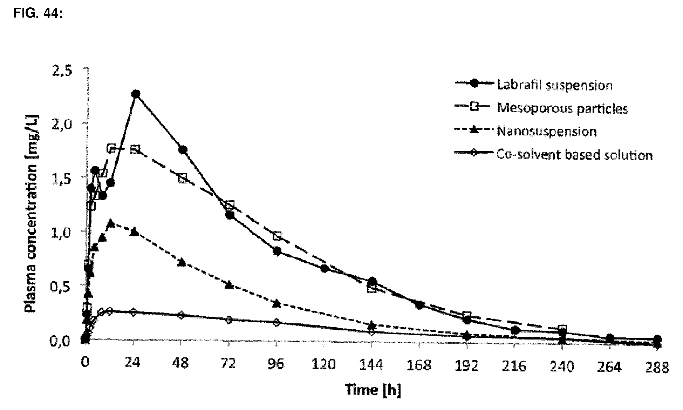

FIG. 44: Mean plasma concentration vs. time profiles after oral administration of 100 mg compound A/kg to dogs

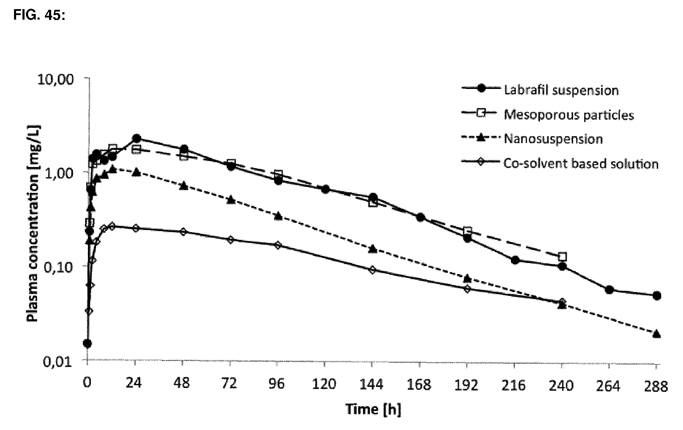

FIG. 45: Mean plasma concentration vs. time profiles after oral administration of 100 mg compound A/kg to dogs with semi-logarithmic plot

## Claims

1. A compound of the general formula (I) 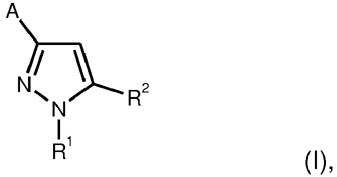 wherein
A stands for a group of formula (i) or (ii) 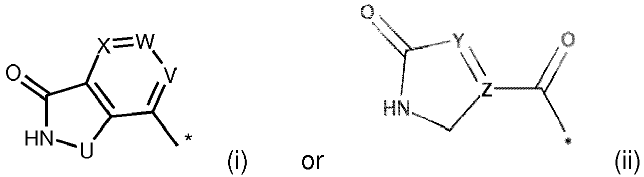
wherein in formula (i)
U stands for carbon,
whereby carbon can be substituted with 1 to 2 alkyl substituents, which are selected independently of one another, or with an oxo substituent,
V stands for nitrogen,
W stands for carbon,
whereby carbon can be substituted with a substituent selected from the group consisting of halogen, amino, hydroxy, methoxy, and methyl,
X stands for nitrogen or carbon,
whereby carbon can be substituted with a substituent selected from the group consisting of halogen, amino, hydroxy, methoxy, and methyl, and
* is the point of attachment to the carbon atom;
wherein in formula (ii)
Y stands for carbon,
whereby carbon can be substituted with halogen, amino, hydroxy, methoxy, and methyl,
Z stands for carbon or nitrogen, and
* is the point of attachment to the carbon atom,
R¹ stands for phenyl or pyridyl,
whereby phenyl is substituted with 1 to 2 substituents, the substituents being selected independently of one another from the group consisting of halogen, hydroxy, amino, cyano, and nitro
R² stands for phenyl,
whereby phenyl is substituted with 1 to 2 substituents, the substituents being selected independently of one another from the group consisting of halogen, hydroxy, amino, nitro, (C1-C4)-alkyl, (C1-C4)-alkylamino and (C1-C4)-alkoxy,
wherein
alkyl, alkylamino and alkoxy in turn may be substituted one to three times identically or differently with radicals selected from the group consisting of halogen, hydroxy, amino,
and their co-crystals or the polymorphs of their co-crystals, and their salts, their solvates or the solvates of their salts
for use in methods of treating and/or preventing cardiovascular diseases and / or comorbidities thereof.

2. The compound of formula (I) for the use in a method of treating and/or preventing cardiovascular diseases and / or comorbidities thereof according to claim 1,
in which A stands for a group of formula (i) or (ii), 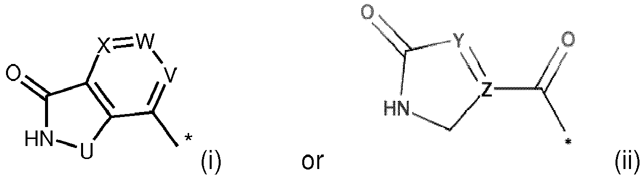 wherein in formula (i)
U stands for carbon,
W stands for carbon,
whereby carbon can be substituted with a methyl,
X stands for nitrogen or carbon,
whereby carbon can be substituted with a methyl,
X preferably stands for nitrogen or carbon, and
* is the point of attachment to the carbon atom,
wherein
R¹ stands for phenyl or pyridyl,
whereby phenyl is substituted with 1 to 2 substituents, the substituents being selected independently of one another from the group consisting of halogen, cyano, and nitro,
whereby said 1 to 2 substituents are more preferably selected independently of one another from the group consisting of halogen and cyano, and said halogen is more preferably selected independently of one another from Cl and F,
whereby
R¹ preferably stands for pyridyl,
wherein
R¹ more preferred stands for 3-pyridyl or 4-pyridyl,
wherein
R¹ most preferred stands for 3-pyridyl,
and
wherein
R² stands for phenyl,
whereby phenyl is substituted with 1 to 2 substituents, the substituents being selected independently of one another from the group consisting of halogen, and (C1-C4)-alkoxy,
wherein
alkoxy in turn may be substituted one to three times identically or differently with radicals selected from the group consisting of halogen,
whereby said 1 to 2 substituents are more preferably selected independently of one another from the group consisting of halogen and (C1-C4)-alkoxy, and said halogen is more preferably selected independently of one another from Cl and F, and the alkoxy on their part can be substituted one to three times with fluorine atoms, more preferred with two fluorine atoms, most preferred with three fluorine atoms,
R² preferably stands for phenyl,
whereby phenyl is substituted with two substituents, said two substituents being more preferably selected independently of one another from the group consisting of halogen and (C1-C3)-alkoxy, and the halogen more preferably being selected independently of one another from Cl and F, and the alkoxy on their part can be substituted with three fluorine atoms,
R² most preferred stands for 3-Cl-5-CF₃O-phenyl;
wherein in formula (ii)
Y stands for carbon, whereby carbon can be substituted with amino,
Z stands for carbon or nitrogen, and
* is the point of attachment to the carbon atom,
R¹ stands for phenyl or pyridyl,
whereby phenyl is substituted with two substituents, the substituents being selected independently of one another from the group consisting of halogen and cyano,
R¹ stands preferably for phenyl or 3-pyridyl,
whereby phenyl is substituted with two substituents, the substituents being selected independently of one another from the group consisting of halogen and cyano,
whereby said halogen is more preferably selected independently of one another from Cl and F,
most preferred said halogen is fluorine,
and wherein
R² stands for phenyl,
whereby phenyl is substituted with 1 to 2 substituents, the substituents being selected independently of one another from the group consisting of halogen, and (C1-C4)-alkoxy,
wherein
alkoxy in turn may be substituted one to three times identically or differently with radicals selected from the group consisting of halogen,
whereby said 1 to 2 substituents are more preferably selected independently of one another from the group consisting of halogen and (C1-C4)-alkoxy, and said halogen is more preferably selected independently of one another from Cl and F, and the alkoxy on their part can be substituted one to three times with fluorine atoms, more preferred with two fluorine atoms, most preferred with three fluorine atoms,
R² preferably stands for phenyl,
whereby phenyl is substituted with two substituents, said two substituents being more preferably selected independently of one another from the group consisting of halogen and (C1-C3)-alkoxy, and the halogen more preferably being selected independently of one another from Cl and F, and the alkoxy on their part can be substituted with three fluorine atoms,
R² most preferred stands for 3-Cl-5-CF₃O-phenyl,
and their co-crystals or the polymorphs of their co-crystals, and their salts, their solvates or the solvates of their salts.

3. The compound of formula (I) for the use in a method of treating and/or preventing cardiovascular diseases and / or comorbidities thereof according to any of the claims 1 or 2,
in which A stands for a group of formula (i) or (ii), 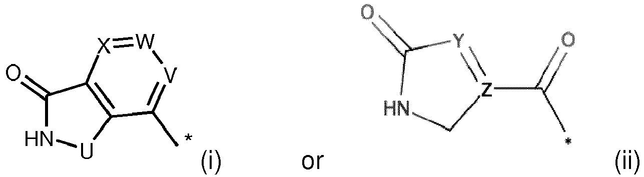 wherein in formula (i)
U stands for CH2,
V stands for N,
W stands for CH or CMe, wherein CMe stands for C-CH3,
X stands for N or CH, and
* is the point of attachment to the carbon atom,
and wherein
R¹ stands for phenyl or 3-pyridyl or 4-pyridyl,
R¹ preferably stands for 3-pyridyl, and
whereby phenyl is substituted with two substituents, where the substituents are selected independently of one another from the group consisting of halogen and cyano,
R² stands for phenyl,
whereby phenyl is substituted with two substituents, the substituents being selected independently of one another from the group consisting of halogen and (C1-C3)-alkoxy,
wherein
halogen is preferably chlorine, and
alkoxy on their part can be substituted with three fluorine atoms;
wherein in formula (ii)
Y stands for CH2 or C-NH2,
Z stands for CH or N, and
* is the point of attachment to the carbon atom,
and wherein
R¹ stands for phenyl or 3-pyridyl or 4-pyridyl,
R¹ preferably stands for 3-pyridyl, and
whereby phenyl is substituted with two substituents, where the substituents are selected independently of one another from the group consisting of halogen and cyano,
R² stands for phenyl,
whereby phenyl is substituted with two substituents, the substituents being selected independently of one another from the group consisting of halogen and (C1-C3)-alkoxy,
wherein
halogen is preferably chlorine, and
alkoxy on their part can be substituted with three fluorine atoms,
and their co-crystals or the polymorphs of their co-crystals, and their salts, their solvates or the solvates of their salts.

4. The compound of formula (I) for the use in a method of treating and/or preventing cardiovascular diseases and / or comorbidities thereof according to any of the claims 1 to 3,
in which A stands for a group of formula (i) or (ii), 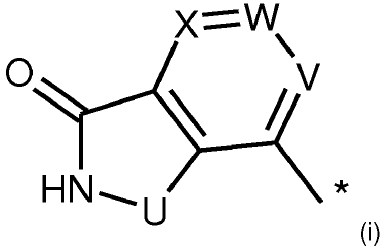 or 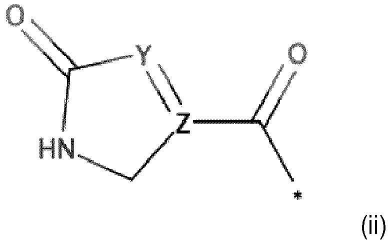 wherein in formula (i)
U stands for CH2,
V stands for N,
W stands for CH or CMe, wherein CMe stands for C-CH3,
X stands for N or CH, and
* is the point of attachment to the carbon atom,
and wherein
R¹ stands for 3-pyridyl,
R² stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part can be substituted with three fluorine atoms;
wherein in formula (ii)
Y stands for CH2 or C-NH2,
Z stands for CH or N, and
* is the point of attachment to the carbon atom,
and wherein
R¹ stands for 3-pyridyl,
R² stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part can be substituted with three fluorine atoms,
and their co-crystals or the polymorphs of their co-crystals, and their salts, their solvates or the solvates of their salts.

5. The compound of formula (I) for the use in a method of treating and/or preventing cardiovascular diseases and / or comorbidities thereof according to any of the preceding claims,
in which A stands for a group of formula (i) or (ii), 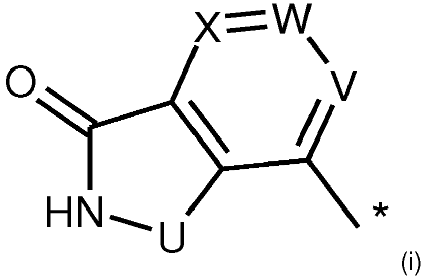 or 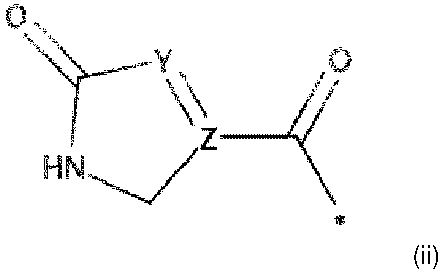 wherein in formula (i)
U stands for CH2,
V stands for N,
W stands for CH,
X stands for N, and
* is the point of attachment to the carbon atom,
and wherein
R¹ stands for 3-pyridyl, and
R² stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part is substituted with three fluorine atoms;
wherein in formula (ii)
Y stands for CH2 or C-NH2,
Z stands for CH or N, and
* is the point of attachment to the carbon atom,
and wherein
R¹ stands for 3-pyridyl,
R² stands for phenyl,
whereby phenyl is substituted with two substituents, the substituents being selected independently of one another from the group consisting of halogen and (C1-C2)-alkoxy,
wherein
halogen is preferably chlorine, and
alkoxy on their part can be substituted with three fluorine atoms,
and their co-crystals or the polymorphs of their co-crystals, and their salts, their solvates or the solvates of their salts.

6. The compound of formula (I) for the use in a method of treating and/or preventing cardiovascular diseases and / or comorbidities thereof according to any of the preceding claims,
in which A stands for a group of formula (i) or (ii),
wherein formula (i) is selected from a group consisting of 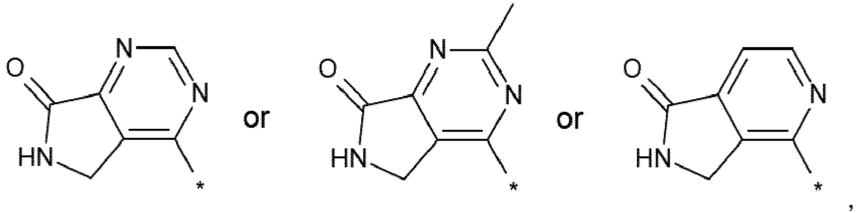 and wherein
R¹ is selected from a group consisting of 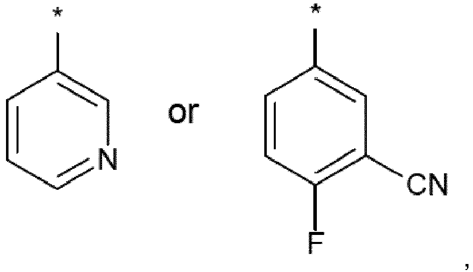
and wherein
R² is 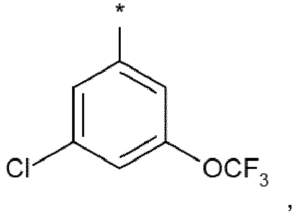
and wherein
* is the point of attachment to the carbon atom;
wherein formula (ii) is selected from a group consisting of 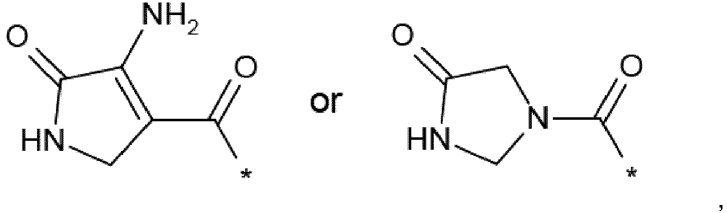 and wherein
R¹ is selected from a group consisting of 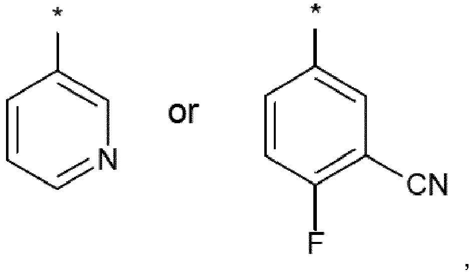
and wherein
R² is 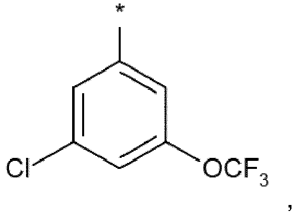
and wherein
* is the point of attachment to the carbon atom
and their co-crystals or the polymorphs of their co-crystals, and their salts, their solvates or the solvates of their salts.

7. The compound of formula (I) for the use in a method of treating and/or preventing cardiovascular diseases and / or comorbidities thereof according to any of the preceding claims, wherein said cardiovascular diseases are selected from a group comprising CA, HHD, tachycardia, IST, AP and SAP, MI, CAD, HF, and stroke, and wherein said comorbidities are selected from a group comprising diabetes, metabolic syndrome, obesity, and CKD.

8. A pharmaceutical composition comprising at least one compound in accordance with any of the claims 1 to 6 or a co-crystal thereof or the polymorphs of said co-crystal, or a salt, a solvate or the solvate of a salt thereof in combination with at least one inert, nontoxic, pharmaceutically suitable adjuvant for the use in a method of treatment and/or prevention of cardiovascular diseases and / or comorbidities thereof.

9. The pharmaceutical composition of claim 8, wherein said cardiovascular diseases are selected from a group comprising CA, HHD, tachycardia, IST, AP and SAP, MI, CAD, HF, and stroke, and wherein said comorbidities are selected from a group comprising diabetes, metabolic syndrome, obesity, and CKD.

10. A pharmaceutical formulation, wherein said formulation is a nanosuspension comprising:
- a compound of general formula (I) according to any of the claims 1 to 6 or a co-crystal thereof or the polymorphs of said co-crystal, or a salt, a solvate or the solvate of a salt thereof with 1 wt % to 40 wt %, and further comprising
- a surfactant(s) selected from the group comprising:
- Tween 80: 0.1 wt % to 10 wt %,
- Sodium deoxycholate: 0.1 wt % to 10 wt %,
- Sodium taurocholate: 0.1 wt % to 10 wt %,
- Sodium glycocholate: 0.1 wt % to 10 wt %,
- Solutol HS 15: 0.1 wt % to 10 wt %,
- Spans: 0.1 wt % to 10 wt %,
- Soluplus: 0.1 wt % to 10 wt %,
- TPGS:0.1 wt%to 10 wt %,
- SLS:0.1 wt % to 10 wt %,
- polyoleate: 0.1 wt % to 10 wt %,
added either alone or in combination, and/or further comprising
- a polymer(s) selected from the group comprising:
- Polyvinylpyrridone (povidone): 0.5 wt % to 5 wt %,
- Poloxamer 188: 0.5 wt % to 5 wt %,
- Cellolosics: 0.5 wt % to 5 wt %,
added either alone or in combination.

11. A pharmaceutical formulation, wherein said formulation is a solid dispersion comprising:
- HPMC-AS with either citric or maleic acid of 20 wt %, and
- containing up to 30 wt % of a compound of the general formula (I) in accordance with any of the claims 1 to 6 or a co-crystal thereof or the polymorphs of said co-crystal, or a salt, a solvate or the solvate of a salt thereof.

12. A pharmaceutical formulation, wherein said formulation is a mesoporous silica based formulation, **characterized by** impregnation of approx. 5 wt % to 40 wt % of a compound of general formula (I) according to any of the claims 1 to 6 or a co-crystal thereof or the polymorphs of said co-crystal, or a salt, a solvate or the solvate of a salt thereof as API, onto silica material, and wherein said mesoporous silica particles can range from 10 wt % to 95 wt %, and wherein polymers and surfactants can be added to said formulation, whereby the concentration of said polymers and surfactants may range from 0.01 wt % to 5 wt %.

13. A pharmaceutical formulation, wherein said formulation is a Capmul^{®} based co-solvent formulation, comprising:
- a drug loading of a compound of general formula (I) according to any of the claims 1 to 6 or a co-crystal thereof or the polymorphs of said co-crystal, or a salt, a solvate or the solvate of a salt thereof from 0.1 to 10 mg/ml,
- the vehicle Capmul^{®} MCM EP C8 between 5 wt % to 70 wt %, and
- acetic acid, which can range from 1 wt % to 15 wt %.

14. A pharmaceutical formulation according to any of the claims 10 to 13, wherein said pharmaceutical formulation is for the use in a method of treating and/or preventing cardiovascular diseases and / or comorbidities thereof.

15. A process for the synthesis of the compound 4-[5-[3-Chloro-5-(trifluoromethoxy)phenyl]-1-(pyridin-3-yl)-1H-pyrazol-3-yl]-5,6-dihydro-7H-pyrrolo[3,4-d]pyrimidin-7-one in accordance with claim 1, said process comprises the steps of:
Step 1)
- reacting the starting material 3-chloro-5-(trifluoromethoxy)acetophenone (i.e. compound 1 a) towards compound 2a in the presence of KOEt and MeOH: 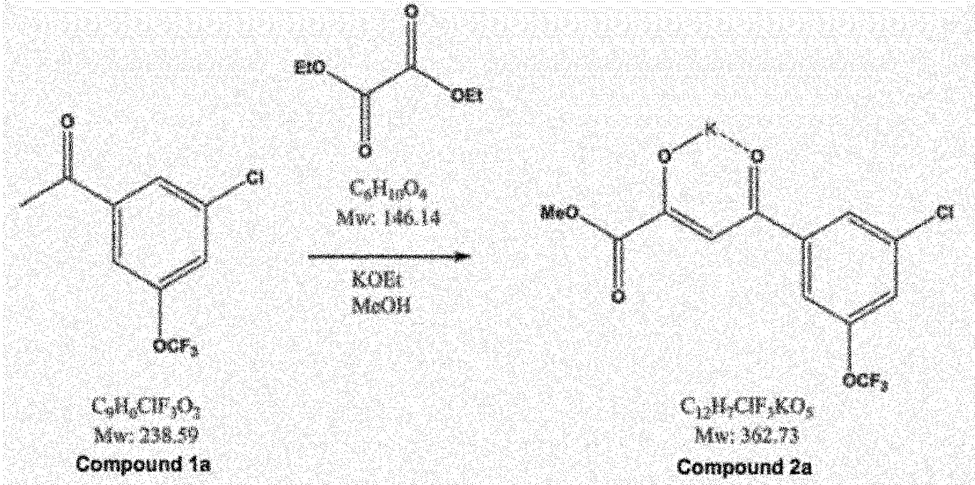
- followed by isolation of compound 2a in solid form as its potassium salt
- using said potassium salt without further purification in the next process step 2),
Step 2)
- reacting compound 2a obtained in step 1) in the presence of EtOH by a condensation reaction with compound 3a towards a compound 5a as depicted below, whereby said reaction mixture must be heated by slow rate heating,
- followed by the reaction of intermediate compound 5a in the presence of NaOH and the addition of hydrochloric acid solution (HCl) with a pH ≥ 2, towards intermediate compound 6a,
- after adjusting the pH, the product compound 6a precipitates out of solution and can be isolated in solid form,
- the obtained dry compound 6a can be used without further purification in the next process step 3): 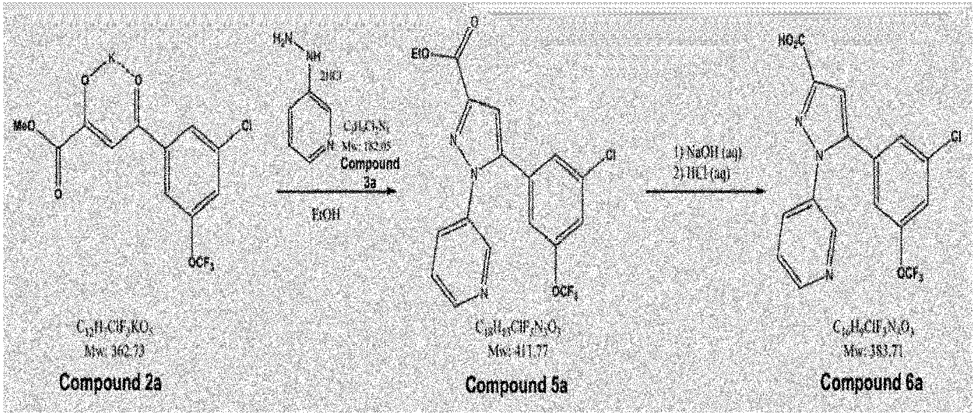
Step 3)
- reacting the starting material compound 6a via the intermediate acid chloride compound 7a in the presence of C2O2Cl2 and DMF as well as CH2Cl2 towards compound 8a in the presence of DiPEA as depicted below, and whereby this reaction is conducted with oxalyl chloride at ambient temperature at approx. 25 ± 5°C and the N,O-dimethylhydroxyl amine is added as its HCl salt,
- the reaction product 8a is isolated in solid form, and used without further purification in the next process step 4): 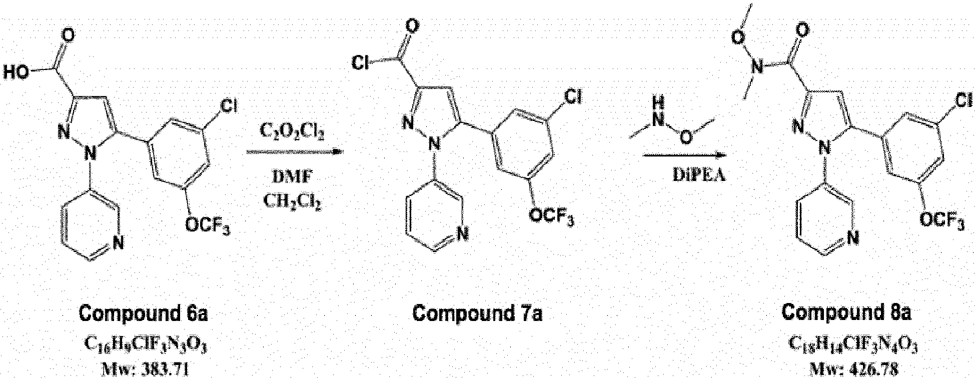
Step 4)
- the synthesis of compound 9a starting from the Weinreb amide compound 8a follows in the presence of MeMgCl and THF as depicted below,
- compound 9a is isolated as a solution in THF,
- this solution can be used in the next step of the synthesis without further purification: 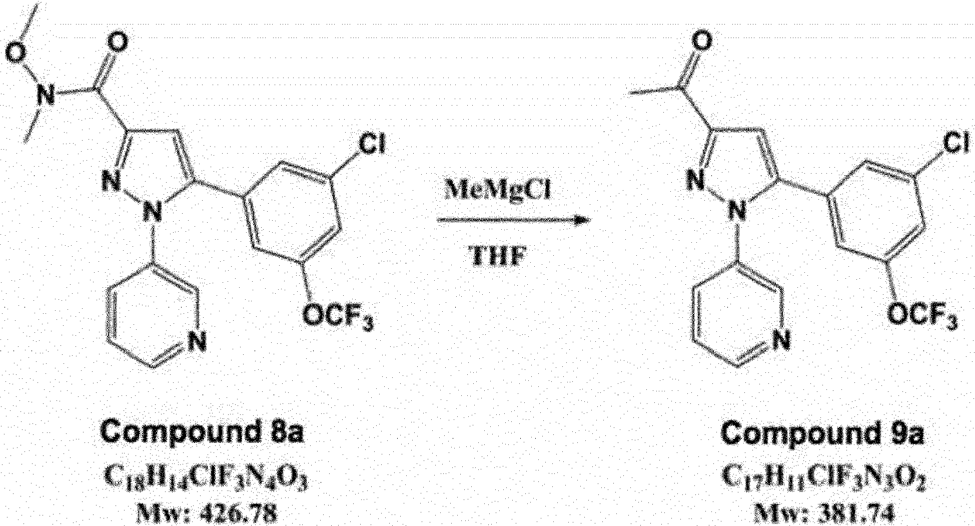
Step 5)
- as depicted below, the synthesis of compound 10a follows in the presence of a sodium ethoxide solution in ethanol, starting from the ketone derivative compound 9a, which is harvested as a tetrahydrofuran solution in step 4), 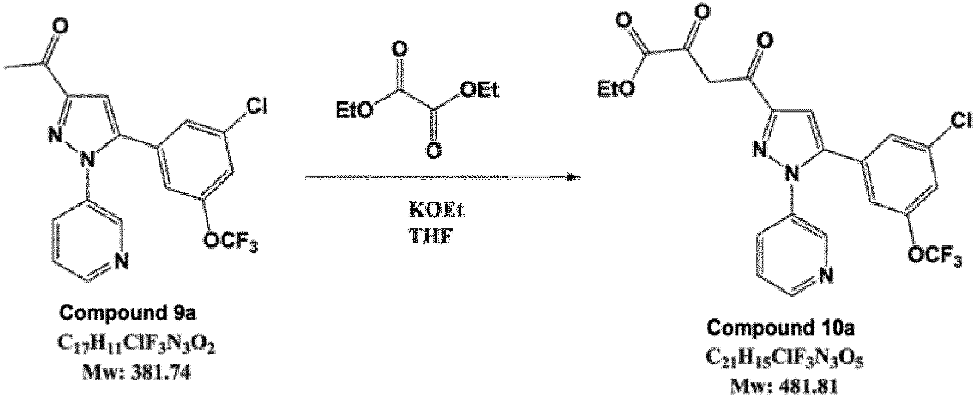
Step 6)
- the synthesis of compound 12a follows in a two-step process in which first a keto-imine (i.e. compound 11 a) is formed, which is not isolated, followed by a Mannich reaction yielding the compound 12a, as depicted below, whereby in both steps the solvent acetic acid is used, and whereby the ammonia donor is in both steps ammonium acetate,
and whereby the first step is performed at approx. 45 ± 5°C, while the second step is performed at approx. 35 ± 5°C,
- the product compound 12a precipitates out of solution by addition of ethanol to the reaction mixture, 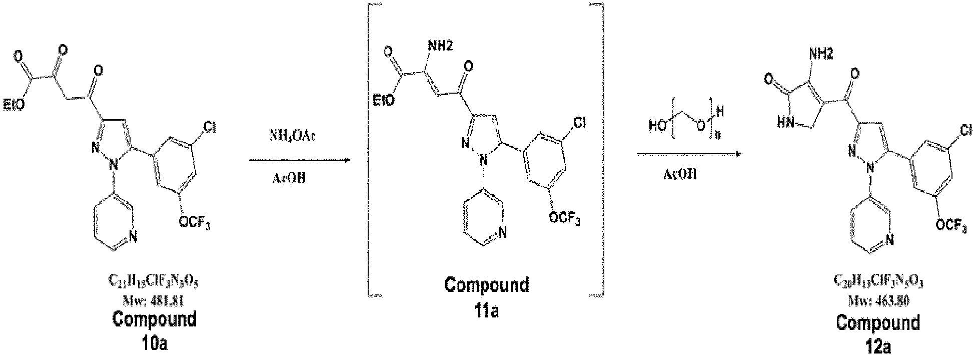
Step 7)
- the synthesis of compound 4-[5-[3-Chloro-5-(trifluoromethoxy)phenyl]-1-(pyridin-3-yl)-1 H-pyrazol-3-yl]-5,6- dihydro-7H-pyrrolo[3,4-d] pyrimidin-7-one as crude product follows as depicted below, starting from the intermediate compound 12a, thereby in the condenser the reaction mixture is heated and kept under pressure, 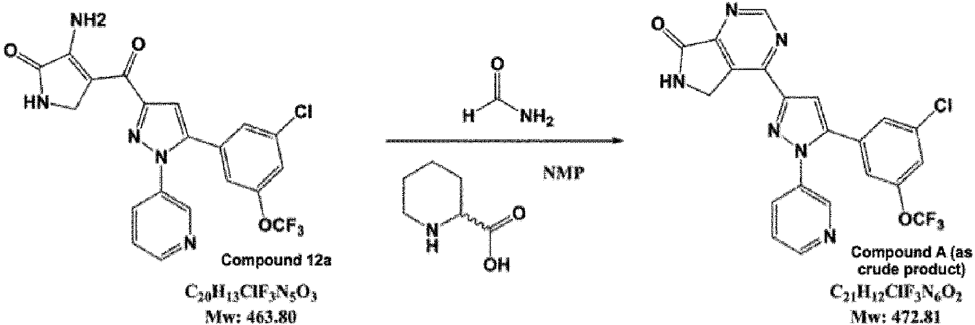
Step 7a)
- the finalization of the above synthesis process is **characterized by** two distinct purification steps, whereby
- the first step comprises the crystallization of the crude product compound 4-[5-[3-Chloro-5-(trifluoromethoxy)phenyl]-1-(pyridin-3-yl)-1H-pyrazol-3-yl]-5,6-dihydro-7H-pyrrolo[3,4-d] pyrimidin-7-one out of an acetic acid solution, using water as anti-solvent, and
- the second step consists of dissolution of the purified compound 4-[5-[3-Chloro-5-(trifluoromethoxy)phenyl]-1-(pyridin-3-yl)-1H-pyrazol-3-yl]-5,6-dihydro-7H-pyrrolo[3,4-d] pyrimidin-7-one in a mixture of dichloromethane and methanol in order to perform a screening filtration,
- the purified product 4-[5-[3-Chloro-5-(trifluoromethoxy)phenyl]-1-(pyridin-3-yl)-1 H-pyrazol-3-yl]-5,6- dihydro-7H-pyrrolo[3,4-d] pyrimidin-7-one can be isolated by filtration after a solvent switch to methanol.
